# FASCICULE DE BREVET EUROPEEN

(11) **EP 2 794 923 B1**
(45) Date de publication et mention de la délivrance du brevet: **20.09.2017**
(21) Numéro de dépôt: 12819084.0
(22) Date de dépôt: 18.12.2012
(51) Int. Cl.: C12Q 1/68

(54) **PROCEDE POUR LE DIAGNOSTIC IN VITRO DU CANCER DU POUMON**
VERFAHREN ZUR IN-VITRO-DIAGNOSE VON LUNGENKREBS
METHOD FOR THE DIAGNOSIS IN VITRO OF LUNG CANCER

(30) Priorité: 20.12.2011 FR 1162027
(43) Date de publication de la demande: 29.10.2014
(73) Titulaire: Biomérieux, 69280 Marcy l'Étoile (FR); Hospices Civils de Lyon, 69002 Lyon (FR)
(72) Inventeur: PEROT, Philippe, 16710 Saint-Yrieix (FR); MALLET, François, 69100 Villeurbanne (FR); MUGNIER, Nathalie, 69003 Lyon (FR)
(74) Mandataire: Agasse, Stéphane
(86) Numéro de dépôt international: PCT/FR2012/052970
(87) Numéro de publication internationale: WO 2013/093324

(56) Documents cités:
- WO-A2-02/46477
- US-A1- 2007 037 147
- DATABASE EMBL [Online] 23 novembre 2001 (2001-11-23), "Homo sapiens chromosome 8, clone RP11-1082L8, complete sequence.", XP002681866, extrait de EBI accession no. EM_HUM:AC100858 Database accession no. AC100858
- DATABASE EMBL [Online] 15 octobre 1999 (1999-10-15), "Human DNA sequence from clone RP4-603I14 on chromosome 1p31.3-33", XP002695969, extrait de EBI accession no. EM_STD:AL122001 Database accession no. AL122001
- DATABASE EMBL [Online] 16 août 2000 (2000-08-16), "Homo sapiens chromosome 3 clone RP11-296N12, WORKING DRAFT SEQUENCE, 30 unordered pieces.", XP002695970, extrait de EBI accession no. EM_HTG:AC078979 Database accession no. AC078979
- SEIFARTH WOLFGANG ET AL: "Comprehensive analysis of human endogenous retrovirus transcriptional activity in human tissues with a retrovirus-specific microarray", JOURNAL OF VIROLOGY, vol. 79, no. 1, 1 janvier 2005 (2005-01-01), pages 341-352, XP002694975, THE AMERICAN SOCIETY FOR MICROBIOLOGY, US ISSN: 0022-538X
- J. PACES: "HERVd: the Human Endogenous RetroViruses Database: update", NUCLEIC ACIDS RESEARCH, vol. 32, no. 90001, 1 janvier 2004 (2004-01-01), page D50, XP055056305, ISSN: 0305-1048, DOI: 10.1093/nar/gkh075
- STAUFFER YVES ET AL: "Digital expression profiles of human endogenous retroviral families in normal and cancerous tissues.", CANCER IMMUNITY, vol. 4, 2, 11 février 2004 (2004-02-11), page 18PP, XP002695976, ISSN: 1424-9634
- Philippe Pérot ET AL: "Microarray-Based Sketches of the HERV Transcriptome Landscape", PLoS ONE, vol. 7, no. 6, 1 January 2012 (2012-01-01) , page e40194, XP055034857, ISSN: 1932-6203, DOI: 10.1371/journal.pone.0040194
- Philippe Pérot ET AL: "Microarray-based sketches of the HERV transcriptome landscape: Supplementary Table S3", PloS one, vol. 7, no. 6, 28 June 2008 (2008-06-28), XP055356742,

## Description

Les rétrovirus endogènes constituent la descendance de rétrovirus infectieux s'étant intégrés sous leur forme provirale dans des cellules de la lignée germinale et ayant été transmis par ce biais dans le génome de la descendance de l'hôte.

Le séquençage du génome humain a permis de révéler l'extrême abondance des éléments transposables ou de leurs dérivés. De fait, les séquences répétées représentent près de la moitié du génome humain et les rétrovirus endogènes et les rétrotransposons en composent 8% avec un nombre s'élevant, à ce jour, à plus de 400 000 éléments.

L'abondance des éléments rétroviraux endogènes (ERVs) présents actuellement dans le génome humain est le résultat d'une centaine d'endogénisations réussies au cours de l'évolution de la lignée humaine. Les différentes vagues d'endogénisation s'étalent sur une période allant de 2 à 90 millions d'années avant notre ère et ont été suivies de l'expansion du nombre de copies par des phénomènes de type « copier/coller » avec possibilité d'apparition d'erreurs, conduisant à partir d'un provirus ancestral à la formation d'une famille de HERV, c'est à dire un ensemble d'éléments présentant des homologies de séquences. Les plus anciens éléments, ceux de la famille HERV-L, se seraient intégrés avant l'émergence des mammifères. Deux familles HERV-F et HERV-H sont apparues dans la période où les premiers primates faisaient leur apparition. Les familles HERV-FRD et HERV-K(HML-5) intégrées il y a 40 à 55 millions d'années, sont spécifiques des primates supérieurs. En revanche les familles HERV-W et HERV-E, par exemple, se sont intégrées 5 à 10 millions d'années plus tard après la séparation avec les singes du nouveau monde, et sont spécifiques des Catarhini (Hominoides et Cercopithèques).

Les séquences ERVs sont représentées sur l'ensemble des chromosomes, avec une densité variant selon les familles et il n'existe pas de corrélation entre la proximité physique des ERVs et leur proximité phylogénétique.

Les ERVs ont longtemps été considérés comme des parasites ou comme de simples déchets de l'ADN. Néanmoins, l'impact des ERVs sur l'organisme n'est pas uniquement limité à leur participation passée dans le modelage du génome ou à des recombinaisons délétères pouvant encore subvenir.

L'abondance et la complexité structurelle des ERVs rendent les analyses de leur expression très compliquées et souvent difficilement interprétables. La détection de l'expression de HERV peut refléter l'activation transcriptionnelle de un ou de plusieurs loci au sein d'une même famille. Le ou les loci activés peuvent de plus varier en fonction du tissu et/ou du contexte.

Les présents inventeurs ont maintenant découvert et démontré, que des séquences d'acides nucléiques correspondant à des loci précisément identifiés d'éléments rétroviraux endogènes, sont associés au cancer de la prostate et que ces séquences sont des marqueurs moléculaires de la pathologie. Les séquences identifiées sont soit des provirus, c'est à dire des séquences contenant tout ou partie des gènes *gag, pol* et env flanqués en 5' et 3' de longues terminaisons répétées (LTR ou « Long Terminal Repeat » selon la terminologie anglo-saxonne), soit tout ou partie des LTR ou des gènes isolés. Les séquences ADN identifiées sont respectivement référencées en SEQ ID NO : 1 à 75 dans le listage de séquences, leur localisation chromosomique est identifiée dans le tableau ci-dessous (NCBI 36/hg18), ainsi que leur expression, surexpression ou sous-expression représentées par le « ratio d'expression » entre échantillon cancéreux et échantillon normal. Quand l'expression de l'acide nucléique ou le changement d'expression de l'acide nucléique est spécifique du tissu prostate on indique cette information par le symbole « x » dans la colonne tissu cible. Ceci signifie que si une expression ou un changement d'expression de l'acide nucléique concerné est déterminé dans un compartiment biologique autre que le tissu prostate, ceci représente, à distance, une signature d'un cancer de la prostate. Les séquences ADN identifiées comme étant spécifiques du tissu prostate sont respectivement référencées en SEQ ID NOs : 1, 3, 4, 8, 10, 11, 15, 16, 21 et 32. Les séquences ADN identifiées comme étant non spécifiques du tissu prostate sont respectivement référencées en SEQ ID NOs : 2, 5, 6, 7, 9, 12, 13, 14, 17, 18, 19, 20, 22, 23, 24, 25, 26, 27, 28, 29, 30, 31, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74 et 75.

**Tableau**

| SEQ ID NO: | Localisation chromosomique | Tissu cible | Ratio d'expression cancer/normal |
|---|---|---|---|
| 1 | (-) chr 8: 125981185-125988649 | x | 6,5 |
| 2 | (-) chr 11: 60237235-60238528 | | 5,1 |
| 3 | (-) chr 19: 20721466-20730278 | x | 3,5 |
| 4 | (-) chr 1: 46569499-46569788 | x | 2,7 |
| 5 | (-) chr 2: 165222667-165224367 | | 2,4 |
| 6 | (-) chr 5: 146727162-146727562 | | 2,4 |
| 7 | (-) chr 7: 79651365-79652053 | | 2,3 |
| 8 | (-) chr 3: 75004674-75009922 | x | 2,2 |
| 9 | (+) chr 19: 60146916-60147844 | | 2,1 |
| 10 | (+) chr 3: 74957891-74960634 | x | 2,1 |
| 11 | (-) chr 19: 58096473-58098768 | x | 2,1 |
| 12 | (+) chr 1: 46568022-46568774 | | 2,1 |
| 13 | (+) chr 6: 142192789-142193227 | | 2,1 |
| 14 | (+) chr 8: 8063655-8067207 | | -2,0 |
| 15 | (+) chr 19: 15807768-15807978 | x | 2,0 |
| 16 | (-) chr 13: 94759022-94759378 | x | 2,0 |
| 17 | (+) chr 12: 31851416-31851846 | | -2,0 |
| 18 | (-) chr 4: 92495874-92498563 | | 2,0 |
| 19 | (+) chr 4: 69952306-69955060 | | 1,9 |
| 20 | (-) chr 2: 157905798-157908183 | | -1,9 |
| 21 | (+) chr 1: 46558555-46559522 | x | 1,9 |
| 22 | (-) chr 10: 20449793-20453869 | | -1,9 |
| 23 | (-) chr X: 135840667-135841473 | | 1,9 |
| 24 | (+) chr 20: 24856581-24861663 | | 1,9 |
| 25 | (+) chr 4: 153982431-153982932 | | 1,9 |
| 26 | (-) chr 1: 144779633-144780605 | | 1,9 |
| 27 | (-) chr X: 153489882-153497212 | | -1,9 |
| 28 | (+) chr 11: 117186039-117190257 | | -1,8 |
| 29 | (-) chr 3: 117306894-117312765 | | 1,8 |
| 30 | (+) chr 8: 8094180-8100651 | | -1,8 |
| 31 | (+) chr 2: 188084458-188084785 | | 1,8 |
| 32 | (-) chr 10: 93051085-93057066 | x | 1,7 |
| 33 | (-) chr 2: 54587807-54590183 | | 1,7 |
| 34 | (-) chr 2: 188741658-188747663 | | 1,7 |
| 35 | (+) chr X: 92571323-92580146 | | 1,7 |
| 36 | (-) chr 4: 92408723-92409131 | | 1,6 |
| 37 | (+) chr 8: 90837193-90837630 | | 1,6 |
| 38 | (+) chr 2: 201711970-201712935 | | -1,6 |
| 39 | (-) chr 1: 154420719-154426128 | | 1,6 |
| 40 | (+) chr 6: 152853219-152859441 | | -1,6 |
| 41 | (-) chr 7: 139899253-139900211 | | -1,6 |
| 42 | (+) chr 1: 146832410-146833382 | | 1,6 |
| 43 | (-) chr 1: 144779633-144780605 | | 1,6 |
| 44 | (+) chr 1: 148879269-148880889 | | -1,6 |
| 45 | (-) chr 5: 34514678-34514916 | | 1,6 |
| 46 | (-) chr 3: 176879333-176879730 | | 1,6 |
| 47 | (+) chr 8: 74896654-74897392 | | 1,5 |
| 48 | (-) chr 20: 15911118-15913833 | | 1,5 |
| 49 | (-) chr 6: 14405150-14411033 | | 1,5 |
| 50 | (-) chr 5: 92818136-92819135 | | -1,5 |
| 51 | (-) chr 8: 54598330-54600779 | | 1,5 |
| 52 | (-) chr X: 78969339-78970117 | | 1,5 |
| 53 | (+) chr 3: 147554294-147559942 | | 1,5 |
| 54 | (-) chr 1: 15334421-15335379 | | 1,5 |
| 55 | (-) chr 8: 12395268-12398823 | | -1,5 |
| 56 | (-) chr 3: 171872658-171878745 | | -1,5 |
| 57 | (-) chr 2: 207379807-207385596 | | 1,5 |
| 58 | (+) chr 6: 131686129-131689771 | | -1,4 |
| 59 | (-) chr 4: 47707230-47708025 | | 1,4 |
| 60 | (-) chr 2: 142963716-142969364 | | 1,4 |
| 61 | (+) chr 5: 130936343-130941430 | | -1,4 |
| 62 | (-) chr 3: 186574589-186580188 | | 1,4 |
| 63 | (+) chr 18: 70111304-70117249 | | -1,4 |
| 64 | (-) chr 8: 56851123-56851350 | | 1,4 |
| 65 | (+) chr 19: 63013510-63014746 | | 1,3 |
| 66 | (+) chr 3: 75269085-75276706 | | 1,3 |
| 67 | (-) chr 19: 58067390-58068685 | | 1,3 |
| 68 | (+) chr 8: 91057690-91058157 | | 1,3 |
| 69 | (+) chr 7: 35702274-35703153 | | 1,3 |
| 70 | (-) chr 13: 90298826-90304533 | | -1,2 |
| 71 | (-) chr 13: 40347160-40352498 | | 1,2 |
| 72 | (-) chr 7: 130691523-130692332 | | 1,2 |
| 73 | (+) chr X: 9597773-9597824 | | -1,2 |
| 74 | (+) chr 10: 92557026-92562997 | | 1,2 |
| 75 | (+) chr 5: 43015565-43018176 | | 1,2 |

La présente invention a donc pour objet un procédé pour le diagnostic, *in vitro,* du cancer de la prostate, *in vitro,* du cancer de la prostate dans un échantillon biologique prélevé chez un patient, qui comprend une étape de détection d'au moins un produit d'expression d'au moins une séquence d'acide nucléique, ladite séquence d'acide nucléique étant choisie parmi les séquences identifiées en SEQ ID NOs : 1, 3, 4, 8, 10, 11, 15, 16, 21 et 32 ou parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 1, 3, 4, 8, 10, 11, 15, 16, 21 et 32.

Le diagnostic permet d'établir si un individu est malade ou non malade. Le pronostic permet d'établir un degré de gravité de la maladie (grades et/ou stades) qui a une incidence sur la survie et/ou qualité de vie de l'individu. Dans le cadre de la présente invention, le diagnostic peut être très précoce.

Le pourcentage d'identité décrit ci-dessus a été déterminé en prenant en considération la diversité nucléotidique dans le génome. Il est connu que la diversité nucléotidique est plus élevée dans les régions du génome riches en séquences répétées que dans les régions ne contenant pas de séquences répétées. A titre d'exemple, Nickerson D. A. et al. (1) ont montré une diversité d'environ 0,3% (0,32%) dans des régions contenant des séquences répétées.

La capacité de discrimination d'un état cancéreux de chacune des séquences identifiées ci-dessus a été mise en évidence à l'aide d'une analyse statistique utilisant la procédure SAM (5) suivie d'une correction par le taux de faux positif (6) et d'une suppression des valeurs inférieures à 2⁶. Par conséquent chacune des séquences identifiées ci-dessus présente une différence d'expression significative entre un état tumoral et un état normal. Il en résulte qu'une différence d'expression observée pour une des séquences précitées constitue une signature de la pathologie. Bien entendu, il est possible de combiner les différences d'expression relevées pour plusieurs des séquences référencées ci-dessus par exemple par une ou des associations de 2, 3, 4, 5, 6, 7, 8, 9, 10 et plus voire jusqu'à 75 des séquences listées, de préférence par une ou des associations de 2, 3, 4, 5, 6, 7, 8, 9, 10 des séquences respectivement identifiées en SEQ ID NOs : 1, 3, 4, 8, 10, 11, 15, 16, 21 et 32. En particulier les séquences identifiées en SEQ ID NOs : 1, 4 et 10, prises seules ou en combinaison (2 à 2 ou 3) constituent une ou des signatures privilégiées.

Dans un mode de réalisation du procédé selon l'invention, on détecte le produit d'expression d'au moins deux séquences d'acide nucléique, lesdites au moins deux séquences d'acide nucléique étant choisies parmi les séquences identifiées comme étant spécifiques du tissu prostate, c'est à dire choisies dans le groupe de séquences identifiées en SEQ ID NOs : 1, 3, 4, 8, 10, 11, 15, 16, 21 et 32 ou parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 1, 3, 4, 8, 10, 11, 15, 16, 21 et 32.

Dans un autre mode de réalisation du procédé de l'invention, on détecte le produit d'expression d'au moins une séquence choisie parmi les séquences identifiées comme étant spécifiques du tissu prostate, c'est à dire choisies dans le groupe de séquences identifiées en SEQ ID NOs : 1, 3, 4, 8, 10, 11, 15, 16, 21 et 32 et le produit d'expression d'au moins une séquence choisie parmi les séquences identifiées comme étant non spécifiques du tissu prostate, c'est à dire choisies dans le groupe de séquences identifiées en SEQ ID NOs : 2, 5, 6, 7, 9, 12, 13, 14, 17, 18, 19, 20, 22, 23, 24, 25, 26, 27, 28, 29, 30, 31, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74 et 75 ou le produit d'expression d'au moins une séquence choisie parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 1, 3, 4, 8, 10, 11, 15, 16, 21 et 32 et le produit d'expression d'au moins une séquence choisie parmi les séquences qui présentent au moins 99% d'identité, de préférence au moins 99,5% d'identité et avantageusement au moins 99,6% ou au moins 99,7% d'identité avec une des séquences identifiées en SEQ ID NOs : 2, 5, 6, 7, 9, 12, 13, 14, 17, 18, 19, 20, 22, 23, 24, 25, 26, 27, 28, 29, 30, 31, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74 et 75.

De préférence dans le procédé de l'invention on détecte le produit d'expression d'au moins une séquence d'acide nucléique, de préférence d'au moins deux séquences d'acide nucléique ou de trois séquences d'acide nucléique, lesdites séquences d'acide nucléique étant choisies dans le groupe de séquences identifiées en SEQ ID NOs : 1, 4 et 10, ou parmi les séquences qui présentent au moins 99% d'identité, de préférence au moins 99,5% d'identité et avantageusement au moins 99,6% ou au moins 99,7% d'identité avec les séquences identifiées en SEQ ID NOs : 1, 4 et 10.

Le produit d'expression détecté est au moins un transcrit ARN, en particulier au moins un ARNm ou au moins un polypeptide.

Lorsque le produit d'expression est un transcrit ARNm, il est détecté par toute méthode appropriée, telle que l'hybridation, le séquençage ou l'amplification. L'ARNm peut être détecté directement par mise en contact avec au moins une sonde et/ou au moins une amorce qui sont conçues pour s'hybrider dans des conditions expérimentales prédéterminées aux transcrits ARNm, la mise en évidence de la présence ou de l'absence d'hybridation à l'ARNm et éventuellement la quantification de l'ARNm. Parmi les méthodes préférées on peut citer l'amplification (par exemple la RT-PCR, la NASBA, etc.), l'hybridation sur puce ou encore le séquençage. L'ARNm peut également être détecté indirectement à partir d'acides nucléiques dérivés desdits transcrits, comme les copies ADNc, etc.

Généralement le procédé de l'invention comprend une étape initiale d'extraction de l'ARNm de l'échantillon à analyser.

Ainsi, le procédé peut comprendre :
(i) une étape d'extraction de l'ARNm de l'échantillon à analyser,
(ii) une étape de détection et de quantification de l'ARNm de l'échantillon à analyser,
(iii) une étape d'extraction de l'ARNm dans un échantillon de référence, qui peut être un échantillon sain et provenant du même individu ou,
(iv) une étape de détection et de quantification de l'ARNm de l'échantillon sain,
(iii) une étape de comparaison de la quantité d'ARNm exprimé dans l'échantillon à analyser et dans l'échantillon de référence ; la détermination d'une quantité d'ARNm exprimé dans l'échantillon à analyser différente de la quantité d'ARNm exprimé dans l'échantillon de référence sain pouvant être corrélée avec le diagnostic d'un cancer de la prostate (la différence de la quantité d'ARNm dans le tissu prostate cancéreux par rapport à la quantité d'ARNm exprimé dans le tissu prostate sain étant
   indifféremment une expression, une surexpression ou une sous-expression);
et en particulier :
(i) une extraction de l'ARNm à analyser de l'échantillon,
(ii) une détermination, dans l'ARN à analyser, d'un niveau d'expression d'au moins une séquence ARN dans l'échantillon, de préférence d'au moins deux séquences ARN dans l'échantillon, la séquence ARN et les séquences ARN étant respectivement le produit de transcription d'au moins une séquence d'acide nucléique choisie parmi les séquences identifiéesci-dessus, et
(iii) une comparaison du niveau d'expression de la ou des séquence(s) ARN définie(s) en (ii) avec un niveau d'expression de référence ; la détermination d'un niveau d'expression de l'ARN à analyser présentant une différence par rapport au niveau d'expression de référence pouvant être corrélé avec le diagnostic d'un cancer de la prostate (comme déterminé ci-dessus) ; ou
   (i) une étape d'extraction de l'ARNm de l'échantillon à analyser,
   (ii) une étape de détection et de quantification de l'ARNm de l'échantillon à analyser,
   (iii) une étape de comparaison de la quantité d'ARNm exprimé dans l'échantillon à analyser par rapport à une quantité d'ARNm de référence, la détermination d'une quantité d'ARNm exprimé dans l'échantillon à analyser différente de la quantité d'ARNm de référence pouvant être corrélée avec le diagnostic d'un cancer de la prostate (la différence de la quantité d'ARNm dans l'échantillon à analyser par rapport à la quantité d'ARNm de référence étant indifféremment une expression, une surexpression ou une sous-expression).

Dans un mode de réalisation du procédé de l'invention on prépare des copies ADN de l'ARNm, on met en contact les copies ADN avec au moins une sonde et/ou au moins une amorce dans des conditions prédéterminées permettant l'hybridation et en ce qu'on détecte la présence ou l'absence d'hybridation aux dites copies ADN.

Le produit d'expression qui est détecté peut aussi être un polypeptide qui est le produit de la traduction d'au moins un des transcrits décrits ci-dessus. Auquel cas, on détecte le polypeptide exprimé par mise en contact avec au moins un partenaire de liaison spécifique dudit polypeptide, en particulier un anticorps ou un analogue d'anticorps ou un aptamère. Le partenaire de liaison est de préférence un anticorps, par exemple un anticorps monoclonal ou un anticorps polyclonal hautement purifié ou un analogue d'un anticorps, par exemple une protéine d'affinité aux propriétés compétitives (nanofitine^{™}).

Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Les nanofitines™ sont de petites protéines qui comme les anticorps sont capables de se lier à une cible biologique permettant ainsi de la détecter, de la capturer ou tout simplement de la cibler au sein d'un organisme. On les présente, entre autres, comme des analogues d'anticorps.

Les aptamères sont des oligonucléotides synthétiques capables de fixer un ligand spécifique.

L'invention concerne également l'utilisation d'au moins une séquence d'acide nucléique, isolée, comme marqueur moléculaire pour le diagnostic *in vitro* du cancer de la prostate, caractérisée en ce que ladite séquence d'acide nucléique consiste en :
(i) au moins une séquence ADN choisie parmi les séquences identifiées en SEQ ID NOs : 1, 3, 4, 8, 10, 11, 15, 16, 21 et 32 et en particulier les séquences SEQ ID Nos : 1, 4 et 10, ou
(ii) au moins une séquence ADN choisie parmi les séquences identifiées en SEQ ID NOs : 1, 3, 4, 8, 10, 11, 15, 16, 21 et 32 et en particulier choisies parmi les séquences SEQ ID Nos : 1, 4 et 10, ou
(iii) au moins une séquence ADN qui présente respectivement au moins 99% d'identité, de préférence au moins 99,5% d'identité et avantageusement au moins 99,6% ou au moins 99,7% d'identité avec deux séquences telles que définies en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité, de préférence au moins 99,5% d'identité et avantageusement au moins 99,6% ou au moins 99,7% d'identité avec les séquences telles que définies en (i).

L'invention concerne encore un procédé pour évaluer l'efficacité d'un traitement du cancer de la prostate qui comprend un étape d'obtention d'une série d'échantillons biologiques, une étape de détection d'au moins un produit d'expression d'au moins une séquence d'acide nucléique dans ladite série d'échantillons biologiques, ladite séquence d'acide nucléique étant choisie parmi les séquences identifiées en SEQ ID NOs : 1, 3, 4, 8, 10, 11, 15, 16, 21 et 32 ou des séquences qui présentent au moins 99% d'identité, de préférence au moins 99,5% d'identité et avantageusement au moins 99,6% ou au moins 99,7% d'identité avec les séquences identifiées en SEQ ID NOs 1, 3, 4, 8, 10, 11, 15, 16, 21 et 32.

Dans un autre mode de réalisation du procédé, on détecte le produit d'expression d'au moins une séquence d'acide nucléique, de préférence d'au moins deux séquences d'acides nucléiques ou de trois séquences d'acide nucléique, lesdites séquences d'acide nucléiques étant choisies dans le groupe de séquences identifiées en SEQ ID NOs 1, 4 et 10 ou parmi les séquences qui présentent au moins 99% d'identité respectivement, de préférence au moins 99,5% d'identité et avantageusement au moins 99,6% ou au moins 99,7% d'identité avec les séquences identifiées en SEQ ID NOs : 1, 4 et 10.

Par échantillon biologique on entend un tissu, un fluide, des composants desdits tissu et fluide, tels que des cellules ou des corps apoptotiques, des vésicules excrétées, comprenant notamment des exosomes et des microvésicules. A titre d'exemple, l'échantillon biologique peut être issu d'une biopsie de prostate réalisée au préalable chez un patient suspecté d'être atteint d'un cancer de la prostate ou être issu d'une biopsie pratiquée sur un organe autre que la prostate chez un patient présentant des métastases. Dans ce deuxième cas, quand le changement d'expression de l'acide nucléique (marqueur moléculaire) est spécifique de l'organe prostate, il est possible de remonter au cancer primaire, c'est à dire au cancer du prostate. L'échantillon biologique peut être également un fluide biologique, tel que du sang ou une fraction sanguine (sérum, plasma), de l'urine, de la salive, du liquide céphalo-rachidien, de la lymphe, du lait maternel, du sperme, de même que des composants desdits fluides, en particulier des vésicules excrétées telles que définies ci-dessus. Par exemple, la détection d'un transcrit spécifique du tissu prostate dans un exosome ou une microvésicule, originaire d'une cellule épithéliale, signe soit la présence d'un cancer primaire, soit des métastases, sans qu'il soit nécessaire de faire un prélèvement au niveau de l'organe.

### Figures :

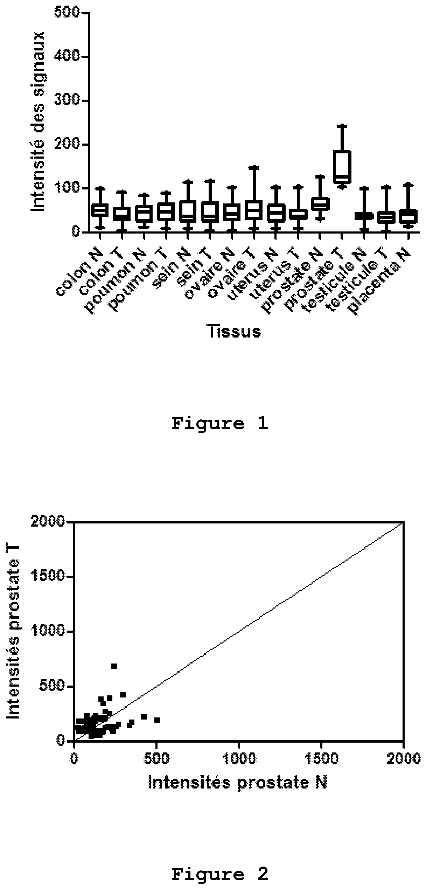

Les figures 1 et 2 représentent le différentiel d'expression observé dans le cancer de la prostate pour un ensemble de séquences HERV. Plus précisément, la figure 1 (clustering) regroupe de manière exploratoire les éléments HERV qui ont un tropisme d'expression associé au cancer de la prostate par rapport à l'ensemble des tissus contrôles, et la figure 2 montre les différences statistiques d'expression d'éléments HERV entre prostate normale et prostate tumorale.
Les figures 3 et 4 montrent la détection de séquences HERV dans deux fluides biologiques : les urines et les sérums.
Les figures 5 à 7 montrent trois exemples de séquences HERV présentant un différentiel d'expression dans les urines en association avec le statut clinique cancéreux des patients.

### Exemples :

### Exemple 1 : Identification de séquences HERV présentant un différentiel d'expression dans le cancer de la prostate

### Méthode :

L'identification de séquences HERV présentant un différentiel d'expression dans le cancer de la prostate repose sur la conception et l'utilisation d'une puce à ADN haute densité au format GeneChip, dénommée HERV-V2, conçue par les inventeurs et dont la fabrication a été sous-traitée à la société Affymetrix. Cette puce contient des sondes qui correspondent à des séquences HERV distinctes au sein du génome humain. Ces séquences ont été identifiées à partir d'un ensemble de références prototypiques découpées en régions fonctionnelles (LTR, *gag, pol* et *env*) puis, par une recherche de similarité à l'échelle du génome humain entier (NCBI 36/hg18), 10 035 loci HERV distincts ont été identifiés, annotés, et finalement regroupés dans une banque de données nommée HERVgDB3.

Les sondes entrant dans la composition de la puce ont été définies à partir de HERVgDB3 et sélectionnées en appliquant un critère de spécificité d'hybridation, dont le but est d'exclure du procédé de création les sondes présentant un risque d'hybridation élevé avec une cible non recherchée. Pour cela, les séquences de HERVgDB3 ont d'abord été segmentées par ensemble de 25 nucléotides (25-mers) chevauchants, aboutissant à un ensemble de sondes candidates. Le risque d'hybridation aspécifique a ensuite été évalué pour chaque sonde candidate en réalisant des alignements sur l'ensemble du génome humain à l'aide de l'algorithme KASH (2). Un score expérimental sanctionne le résultat de l'hybridation, addition de l'impact du nombre, du type et de la position des erreurs dans l'alignement. La valeur de ce score est corrélée au potentiel d'hybridation cible/sonde. La connaissance de tous les potentiels d'hybridation d'une sonde candidate sur l'ensemble du génome humain permet d'évaluer sa spécificité de capture. Les sondes candidates qui présentent une bonne affinité de capture sont conservées puis regroupées en « probesets » et enfin synthétisées sur la puce HERV-V2.

Les échantillons analysés à l'aide de la puce haute densité HERV-V2 correspondent à des ARN extraits de tumeurs et aux ARN extraits des tissus sains adjacents à ces tumeurs. Les tissus analysés sont la prostate, avec en contrôles le sein, l'ovaire, l'utérus, le côlon, le poumon, le testicule et le placenta. Dans le cas du placenta, seuls des tissus sains ont été utilisés. Pour chaque échantillon, 50ng d'ARN ont servi à la synthèse de cDNA en utilisant le protocole d'amplification connu sous le nom de WTO. Le principe de l'amplification WTO est le suivant : des amorces aléatoires, ainsi que des amorces ciblant l'extrémité 3' du transcrit ARN, sont ajoutées, avant une étape de transcription inverse suivie d'une amplification linéaire et simple brin désignée SPIA. Les cDNA sont ensuite dosés, caractérisés et purifiés, puis 2µg sont fragmentés, marqués à la biotine en extrémité 3' par l'action de l'enzyme *terminal transferase.* Le produit cible ainsi préparé est mélangé à des oligonucléotides de contrôle, puis l'hybridation est réalisée selon le protocole recommandé par la société Affymetrix. Les puces sont alors révélées et lues pour acquérir l'image de leur fluorescence. Un contrôle qualité se basant sur les contrôles standards est réalisé, et un ensemble d'indicateurs (MAD, MAD-Med plots, RLE) servent à exclure les puces non conformes à une analyse statistique.

L'analyse des puces consiste d'abord en un pré-traitement des données par l'application d'une correction du bruit de fond basée sur l'intensité des signaux des sondes tryptophanes, suivie d'une normalisation RMA (3) basée sur la méthode des quantiles. Puis une double correction des effets liés aux lots d'expériences est réalisée en appliquant la méthode COMBAT (4) afin de garantir que les différences d'expression observées sont d'origine biologique et non techniques. A ce stade, une analyse exploratoire des données est conduite à l'aide d'outils de regroupement de données par partitionnement euclidien (*clustering*), et enfin une analyse statistique utilisant la procédure SAM (5) suivie d'une correction par le taux de faux positif (6) et d'une suppression des valeurs inférieures à 2⁶ est appliquée pour la recherche de séquences présentant un différentiel d'expression entre l'état normal et l'état tumoral d'un tissu.

### Résultats :

Le traitement des données générées par l'analyse des puces à ADN HERV-V2 à l'aide de cette méthode a permis d'identifier un ensemble de « probesets » présentant une différence d'expression statistiquement significative entre la prostate normale et la prostate tumorale. Les résultats du *clustering* ainsi que la recherche d'expression différentielle au sein des échantillons contrôles a par ailleurs mis en évidence des éléments HERV dont l'expression différentielle est spécifiquement associée à la prostate tumorale.

Les séquences nucléotidiques des éléments HERV présentant un différentiel d'expression dans la prostate tumorale sont identifiées par les SED ID N°1 à 75, la localisation chromosomique de chaque séquence est donnée dans le référentiel NCBI 36/hg18, et la mention « tissu cible » (une croix) pointe les éléments dont l'expression différentielle n'a été observée que dans la comparaison entre prostate normale et prostate tumorale (par rapport aux comparaisons au sein des tissus contrôles). Une valeur indicative du ratio d'expression entre état normal et état tumoral est également communiquée, et sert à ordonner les séquences dans un soucis de présentation uniquement.

### Exemple 2 : Détection de séquences HERV dans les fluides biologiques

### Principe :

Les inventeurs ont montré que des séquences HERV sont détectées dans les fluides biologiques, ce qui permet entre autres de caractériser un cancer de la prostate par recours à une détection à distance de l'organe primaire. Une étude a été menée sur 20 échantillons d'urine et 38 échantillons de sérum provenant d'individus différents.

Les sérums et les urines ont été centrifugés dans les conditions suivantes :
Sérums : 500 g pendant 10 minutes à 4°C. Le surnageant a été récupéré et centrifugé de nouveau à 16500 g pendant 20 minutes à 4°C. Le surnageant de cette deuxième centrifugation, dépourvu de cellules, mais comprenant encore des exosomes, des microvésicules, des acides nucléiques, des protéines, a été analysé sur des puces. La puce est la puce HERV-V2 utilisée selon les modalités précédemment décrites.
Urines : après recueil, centrifugation à 800 g pendant 4 minutes à 4°C. Le culot a été récupéré avec du RNA protect cell reagent™. Puis, centrifugation à 5000 g pendant 5 minutes avant ajout du tampon de lyse sur le culot. La puce est la puce HERV-V2 utilisée selon les modalités précédemment décrites.

### Résultats :

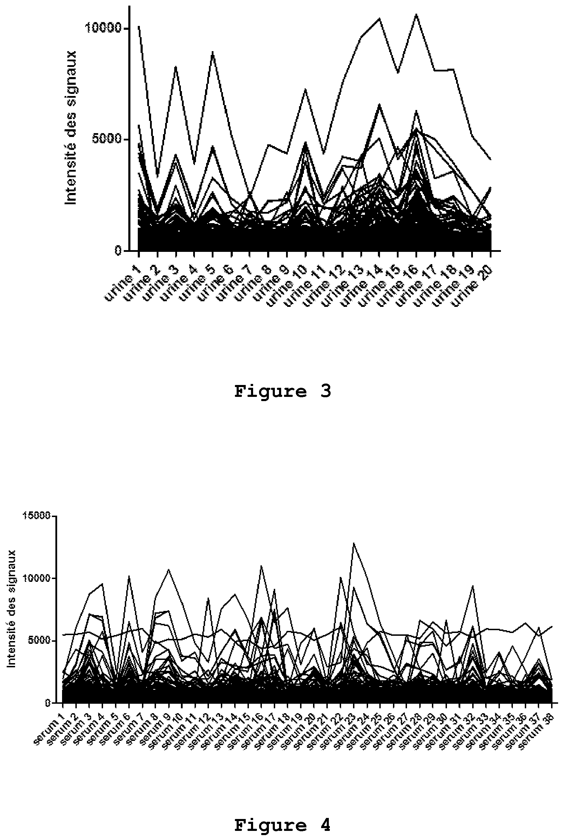

Un nombre important de signaux positifs, incluant les signaux d'expression correspondant aux séquences listées dans le tableau ci-dessus, a été détecté à la fois dans les surnageants de sérums et dans les culots cellulaires provenant d'urines, comme illustré dans les figures 3 et 4. Ceci confirme que les fluides biologiques, en particulier le sérum et l'urine, sont une source utilisable de matériel biologique pour la détection de séquences HERV. Il est communément accepté que le seuil de positivité est de l'ordre de 2⁶ soit 64.

### Exemple 3 : Mise en évidence d'une expression différentielle de séquences HERV dans des fluides biologiques dans le cas du cancer de la prostate

### Principe :

Deux classes cliniques ont été définies : (PBPNeg) absence de cancer de la prostate établie par références biopsiques ; (CAPR) cancer de la prostate établi après analyse anatomopathologique des pièces de prostatectomies du patient. Les urines des patients ont été recueillies et traitées selon le protocole décrit plus haut. La puce HERV-V2 a été utilisée selon les modalités précédemment décrites afin de mettre en évidence les séquences HERV présentant un différentiel d'expression entre les deux classes cliniques dans une étude incluant 20 patients.

### Résultats :

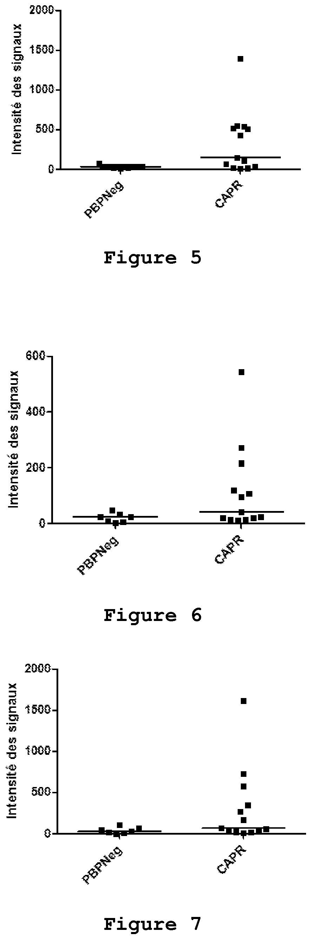

Un ensemble de séquences HERV présentant un différentiel d'expression statistiquement significatif entre les classes cliniques a été identifié. Trois exemples parmi ces séquences HERV sont montrés en figures 5 à 7. Chaque point représente la valeur d'expression de la séquence considérée chez un individu. La barre horizontale indique la médiane des valeurs. Les trois exemples montrent le caractère discriminant du niveau d'expression des séquences considérées dans la mesure où les variances des groupes PBPNeg et CAPR sont significativement différentes (test de Fisher, pvalue inférieure à 0,05).

### Références bibliographiques

1. Nickerson,D.A., Taylor,S.L., Weiss,K.M., Clark,A.G., Hutchinson,R.G., Stengard,J., Salomaa,V., Vartiainen,E., Boerwinkle,E. and Sing,C.F. (1998) DNA sequence diversity in a 9.7-kb region of the human lipoprotein lipase gene. Nat. Genet., 19, 233-240.
2. Navarro,G. and Raffinot,M. (2002) Flexible Pattern Matching in Strings: Practical On-Line Search Algorithms for Texts and Biological Sequences. Cambridge University Press.
3. Irizarry,R.A., Hobbs,B., Collin,F., Beazer-Barclay,Y.D., Antonellis,K.J., Scherf,U. and Speed,T.P. (2003) Exploration, normalization, and summaries of high density oligonucleotide array probe level data. Biostatistics (Oxford, England), 4, 249-264.
4. Johnson,W.E., Li,C. and Rabinovic,A. (2007) Adjusting batch effects in microarray expression data using empirical Bayes methods. Biostatistics (Oxford, England), 8, 118-127.
5. Tusher,V.G., Tibshirani,R. and Chu,G. (2001) Significance analysis of microarrays applied to the ionizing radiation response. Proceedings of the National Academy of Sciences of the United States of America, 98, 5116-5121.
6. Storey,J.D. and Tibshirani,R. (2003) Statistical significance for genomewide studies. Proceedings of the National Academy of Sciences of the United States of America, 100, 9440-9445.

## Revendications

1. Procédé pour le diagnostic, in vitro, spécifique, du cancer de la prostate dans un échantillon biologique prélevé chez un patient qui comprend une étape de détection d'au moins un produit d'expression d'au moins une séquence d'acide nucléique, ladite séquence d'acide nucléique étant choisie parmi les séquences identifiées en SEQ ID NOs 1, 3, 4, 8, 10, 11, 15, 16, 21 et 32 ou parmi les séquences qui présentent au moins 99% d'identité avec une des séquences identifiées en SEQ ID NOs 1, 3, 4, 8, 10, 11, 15, 16, 21 et 32.

2. Procédé selon la revendication 1, **caractérisé en ce qu'**on détecte en outre le produit d'expression d'au moins une séquence d'acide nucléique, ladite séquence d'acide nucléique étant choisie dans le groupe de séquences identifiées en SEQ ID NOs: 2, 5-7, 9, 12-14, 17-20, 22-31 et 33-75 ou parmi les séquences qui présentent au moins 99% d'identité avec les séquences identifiées en SEQ ID NOs : 2, 5-7, 9, 12-14, 17-20, 22-31 et 33-75.

3. Procédé selon la revendication 1, dans lequel est détecté le produit d'expression d'au moins deux séquences d'acide nucléique choisies dans le groupe de séquences identifiées en SEQ ID NOs : 1, 3, 4, 8, 10, 11, 15, 16, 21 et 32 ou parmi les séquences qui présentent au moins 99% d'identité avec les séquences identifiées en SEQ ID Noms : 1, 3, 4, 8,10,11,15,16, 21 et 32.

4. Procédé selon la revendication 3, dans lequel est détecté le produit d'expression d'au moins deux séquences d'acide nucléique, de préférence de trois séquences d'acide nucléique, choisies dans le groupe de séquences identifiées en SEQ ID NOs 1, 4 et 10, ou parmi les séquences qui présentent au moins 99% d'identité avec les séquences identifiées en SEQ ID NOs : 1, 4 et 10.

5. Procédé selon la revendication 1, dans lequel le produit d'expression détecté est au moins un transcrit ARN ou au moins un polypeptide.

6. Procédé selon la revendication 5, **caractérisé en ce que** le transcrit ARN est au moins un ARNm.

7. Procédé selon la revendication 5 ou 6, dans lequel le transcrit ARN, en particulier l'ARNm est détecté par hybridation, par amplification ou par séquençage.

8. Procédé selon la revendication 7, dans lequel l'ARNm est mis en contact avec au moins une sonde et/ou au moins une amorce dans des conditions prédéterminées permettant l'hybridation et en ce qu'on détecte la présence ou l'absence d'hybridation à l'ARNm.

9. Procédé selon la revendication 6 ou 7, **caractérisé en ce qu'**on prépare des copies ADN de l'ARNm, on met en contact les copies ADN avec au moins une sonde et/ou au moins une amorce dans des conditions prédéterminées permettant l'hybridation et **en ce qu'**on détecte la présence ou l'absence d'hybridation aux dites copies ADN.

10. Procédé selon la revendication 5, dans lequel on détecte le polypeptide exprimé par mise en contact avec au moins un partenaire de liaison spécifique dudit polypeptide, en particulier un anticorps ou un analogue d'anticorps, une protéine d'affinité ou un aptamère.

11. Utilisation d'au moins une séquence d'acide nucléique, isolée, comme marqueur moléculaire pour le diagnostic in vitro spécifique du cancer de la prostate, **caractérisée en ce que** la séquence d'acide nucléique consiste en
(i) au moins une séquence ADN choisie parmi les séquences identifiées en SEQ ID NOs : 1, 3, 4, 8, 10, 11, 15, 16, 21 et 32 et en particulier parmi les séquences identifiées en SEQ ID NOs : 1, 4 et 10, ou
(ii) au moins une séquence ADN complémentaire d'au moins une séquence choisie parmi les séquences identifiées en SEQ ID NOs : 1, 3, 4, 8, 10, 11, 15, 16, 21 et 32 et en particulier parmi les séquences identifiées en SEQ ID NOs : 1, 4 et 10, ou
(iii) au moins une séquence ADN qui présente au moins 99% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'au moins une séquence choisie parmi les séquences qui présentent au moins 99% d'identité avec les séquences telles que définies en (i).

12. Procédé pour évaluer l'efficacité d'un traitement du cancer de la prostate qui comprend une étape de détection d'au moins un produit d'expression d'au moins une séquence d'acide nucléique, ladite séquence d'acide nucléique étant choisie parmi les séquences identifiées en SEQ ID NOs : 1, 3, 4, 8, 10, 11, 15, 16, 21 et 32 ou parmi les séquences qui présentent au moins 99% d'identité avec les séquences identifiées en SEQ ID NOs : 1, 3, 4, 8, 10, 11, 15, 16, 21 et 32.

13. Procédé selon la revendication 12, dans lequel est détecté le produit d'expression d'au moins une, voire deux séquences d'acide nucléique, de préférence trois séquences d'acide nucléique, lesdites séquences d'acide nucléiques étant choisies dans le groupe de séquences identifiées en SEQ ID NOs : 1, 4 et 10, ou parmi les séquences qui présentent au moins 99% d'identité avec les séquences identifiées en SEQ ID NOs : 1,4 et 10.

## Patentansprüche

1. Verfahren zur spezifischen *in vitro*-Diagnose von Prostatakrebs in einer biologischen Probe, entnommen von einem Patienten, das einen Schritt des Nachweisens von mindestens einem Expressionsprodukt von mindestens einer Nukleinsäuresequenz umfasst, wobei die Nukleinsäuresequenz ausgewählt ist aus den Sequenzen, identifiziert in SEQ ID Nr. 1, 3, 4, 8, 10, 11, 15, 16, 21 und 32 oder aus den Sequenzen, die mindestens 99% Identität mit einer der Sequenzen aufweist, identifiziert in SEQ ID Nr. 1, 3, 4, 8, 10, 11, 15, 16, 21 und 32.

2. Verfahren nach Anspruch 1, **dadurch gekennzeichnet, dass** außerdem das Expressionsprodukt von mindestens einer Nukleinsäuresequenz nachgewiesen wird, wobei die Nukleinsäuresequenz ausgewählt ist aus der Gruppe von Sequenzen, identifiziert in SEQ ID Nr. 2, 5 - 7, 9, 12 - 14, 17 - 20, 22 - 31 und 33 - 75 oder aus den Sequenzen, die mindestens 99% Identität mit den Sequenzen aufweisen, identifiziert in SEQ ID Nr. 2, 5 - 7, 9, 12 - 14, 17 - 20, 22 - 31 und 33 - 75.

3. Verfahren nach Anspruch 1, wobei das Expressionsprodukt von mindestens zwei Nukleinsäuresequenzen nachgewiesen wird, ausgewählt aus der Gruppe von Sequenzen, identifiziert in SEQ ID Nr. 1, 3, 4, 8, 10, 11, 15, 16, 21 und 32 oder aus den Sequenzen, die mindestens 99% Identität mit den Sequenzen aufweisen, identifiziert in SEQ ID Nr. 1, 3, 4, 8, 10, 11, 15, 16, 21 und 32.

4. Verfahren nach Anspruch 3, wobei das Expressionsprodukt von mindestens zwei Nukleinsäuresequenzen nachgewiesen wird, vorzugsweise drei Nukleinsäuresequenzen, ausgewählt aus der Gruppe von Sequenzen, identifiziert in SEQ ID Nr. 1, 4 und 10, oder aus den Sequenzen, die mindestens 99% Identität mit den Sequenzen aufweisen, identifiziert in SEQ ID Nr. 1, 4 und 10.

5. Verfahren nach Anspruch 1, wobei das nachgewiesene Expressionsprodukt mindestens ein RNA-Transkript oder mindestens ein Polypeptid ist.

6. Verfahren nach Anspruch 5, **dadurch gekennzeichnet, dass** das RNA-Transkript mindestens ein RNAm ist.

7. Verfahren nach Anspruch 5 oder 6, wobei das RNA-Transkript, insbesondere RNAm, durch Hybridisierung, durch Amplifizierung oder durch Sequenzierung nachgewiesen wird.

8. Verfahren nach Anspruch 7, wobei die RNAm mit mindestens einer Sonde und/oder mindestens einem Primer unter vorbestimmten Bedingungen in Kontakt gebracht wird, die die Hybridisierung ermöglichen, und dadurch, dass die Anwesenheit oder die Abwesenheit der Hybridisierung zu RNAm nachgewiesen wird.

9. Verfahren nach Anspruch 6 oder 7, **dadurch gekennzeichnet, dass** DNA-Kopien von RNAm hergestellt werden, die DNA-Kopien mit mindestens einer Sonde und/oder mindestens einem Primer unter vorbestimmten Bedingungen in Kontakt gebracht werden, die die Hybridisierung ermöglichen, und dadurch, dass die Anwesenheit oder die Abwesenheit der Hybridisierung zu den DNA-Kopien nachgewiesen wird.

10. Verfahren nach Anspruch 5, wobei das Polypeptid, exprimiert durch das In-Kontakt-Bringen mit mindestens einem spezifischen Bindungspartner des Polypeptids, insbesondere einem Antikörper oder einem Antikörperanalog, einem Affinitätsprotein oder einem Aptamer nachgewiesen wird.

11. Verwendung von mindestens einer isolierten Nukleinsäuresequenz als Molekülmarker für die spezifische *in vitro*-Diagnose von Prostatakrebs, **dadurch gekennzeichnet, dass** die Nukleinsäuresequenz aus Folgendem besteht:
(i) mindestens einer DNA-Sequenz, ausgewählt aus den Sequenzen, identifiziert in SEQ ID Nr. 1, 3, 4, 8, 10, 11, 15, 16, 21 und 32 und insbesondere aus den Sequenzen, identifiziert in SEQ ID Nr. 1, 4 und 10, oder
(ii) mindestens einer zusätzliche DNA-Sequenz, ausgewählt aus den Sequenzen, identifiziert in SEQ ID Nr. 1, 3, 4, 8, 10, 11, 15, 16, 21 und 32 und insbesondere aus den Sequenzen, identifiziert in SEQ ID Nr. 1, 4 und 10, oder
(iii) mindestens einer DNA-Sequenz, die mindestens 99% Identität mit einer Sequenz, wie z. B. in (i) und (ii) definiert, aufweist, oder
(iv) mindestens einer RNA-Sequenz, die das Transkriptionsprodukt einer Sequenz, ausgewählt aus den Sequenzen, wie z. B. in (i) definiert, ist, oder
(v) mindestens einer RNA-Sequenz, die das Transkriptionsprodukt mindestens einer Sequenz ist, ausgewählt aus den Sequenzen, die mindestens 99% Identität mit den Sequenzen wie z. B. in (i) definiert, aufweisen.

12. Verfahren zur Bewertung der Wirksamkeit einer Behandlung von Prostatakrebs, die einen Schritt des Nachweisens von mindestens einem Expressionsprodukt von mindestens einer Nukleinsäuresequenz umfasst, wobei die Nukleinsäuresequenz ausgewählt ist aus den Sequenzen, identifiziert in SEQ ID Nr. 1, 3, 4, 8, 10, 11, 15, 16, 21 und 32, oder aus den Sequenzen, die mindestens 99% Identität mit den Sequenzen aufweisen, identifiziert in SEQ ID Nr. 1, 3, 4, 8, 10, 11, 15, 16, 21 und 32.

13. Verfahren nach Anspruch 12, wobei das Expressionsprodukt von mindestens einer, sogar zwei Nukleinsäuresequenzen, vorzugsweise drei Nukleinsäuresequenzen nachgewiesen wird, wobei die Nukleinsäuresequenzen ausgewählt sind aus der Gruppe von Sequenzen, identifiziert in SEQ ID Nr. 1, 4 und 10, oder aus den Sequenzen, die mindestens 99% Identität mit den Sequenzen aufweisen, identifiziert in SEQ ID Nr. 1, 4 und 10.

## Claims

1. A method for the *in vitro* specific diagnosis of prostate cancer in a biological sample taken from a patient which comprises a step of detecting at least one expression product of at least one nucleic acid sequence, said nucleic acid sequence being chosen from the sequences identified in SEQ ID NOs 1, 3, 4, 8, 10, 11, 15, 16, 21 and 32 or from the sequences which have at least 99% of identity with one of the sequences identified in SEQ ID NOs 1, 3, 4, 8, 10, 11, 15, 16, 21 and 32.

2. The method according to claim 1, **characterized in that** the expression product of at least one nucleic acid sequence is further detected, said nucleic acid sequence being chosen from the group of sequences identified in SEQ ID NOs: 2, 5-7, 9, 12-14, 17-20, 22-31 and 33-75 or from the sequences which have at least 99% of identity with the sequences identified in SEQ ID NOs: 2, 5-7, 9, 12-14, 17-20, 22-31 and 33-75.

3. The method according to claim 1, wherein there is detected the expression product of at least two nucleic acid sequences chosen from the group of sequences identified in SEQ ID NOs: 1, 3, 4, 8, 10, 11, 15, 16, 21 and 32 or from the sequences which have at least 99% of identity with the sequences identified in SEQ ID NOs: 1, 3, 4, 8, 10, 11, 15, 16, 21 and 32.

4. The method according to claim 3, wherein there is detected the expression product of at least two nucleic acid sequences, preferably three nucleic acid sequences, chosen from the group of sequences identified in SEQ ID NOs 1, 4 and 10, or from the sequences which have at least 99% of identity with the sequences identified in SEQ ID NOs: 1, 4 and 10.

5. The method according to claim 1, wherein the detected expression product is at least one RNA transcript or at least one polypeptide.

6. The method according to claim 5, **characterized in that** the RNA transcript is at least one mRNA.

7. The method according to claim 5 or 6, wherein the RNA transcript, particularly the mRNA is detected by hybridization, amplification or sequencing.

8. The method according to claim 7, wherein the mRNA is brought into contact with at least one probe and/or at least one primer under predetermined conditions allowing the hybridization and the presence or the absence of hybridization with mRNA to be detected.

9. The method according to claim 6 or 7, **characterized in that** DNA copies of the mRNA are prepared, the DNA copies are brought into contact with at least one probe and/or at least one primer under predetermined conditions allowing the hybridization and the presence or the absence of hybridization with said DNA copies to be detected.

10. The method of claim 5, wherein the expressed polypeptide is detected by bringing into contact with at least one specific binding partner of said polypeptide, in particular an antibody or an antibody analog, an affinity protein or an aptamer.

11. A use of at least one isolated nucleic acid sequence as a molecular marker for the specific *in vitro* diagnosis of prostate cancer, **characterized in that** the nucleic acid sequence consists of
(i) at least one DNA sequence chosen from the sequences identified in SEQ ID NOs: 1, 3, 4, 8, 10 , 11, 15, 16, 21 and 32 and in particular from the sequences identified in SEQ ID NOs: 1, 4 and 10, or
(ii) at least one DNA sequence complementary to at least one sequence chosen from the sequences identified in SEQ ID NOs: 1, 3, 4, 8, 10, 11, 15, 16, 21 and 32 and in particular from the sequences identified in SEQ ID NOs: 1, 4 and 10, or
(iii) at least one DNA sequence which has at least 99% of identity with a sequence as defined in (i) and (ii), or
(iv) at least one RNA sequence which is the transcription product of a sequence chosen from the sequences as defined in (i), or
(v) at least one RNA sequence which is the transcription product of at least one sequence chosen from the sequences which have at least 99% of identity with the sequences as defined in (i).

12. The method for evaluating the efficiency of a prostate cancer treatment which comprises a step of detecting at least one expression product of at least one nucleic acid sequence, said nucleic acid sequence being chosen from the sequences identified in SEQ ID NOs: 1, 3, 4, 8, 10, 11, 15, 16, 21 and 32 or from the sequences which have at least 99% of identity with the sequences identified in SEQ ID NOs: 1, 3, 4, 8, 10, 11, 15, 16, 21 and 32.

13. The method according to claim 12, wherein the expression product of at least one or even two nucleic acid sequences, preferably three nucleic acid sequences, is detected, said nucleic acid sequences being chosen from the group of sequences identified in SEQ ID NOs: 1, 4 and 10, or from the sequences which have at least 99% of identity with the sequences identified in SEQ ID NOs: 1, 4 and 10.


### SEQUENCE LISTING

<110> bioMérieux
<120> Procédé pour le diagnostic ou le pronostic in vitro du cancer du sein
<130> ENDOBREAST
<150> FR1162095
   <151> 2011-12-20
<160> 31
<170> PatentIn version 3.5
<210> 1
   <211> 3135
   <212> DNA
   <213> Homo sapiens
<400> 1
   tgactctctt tttggactca gcccacctgc acccaggtga aataaacagc catgttggtc 60
   acacaaagcc tgtgtggtgg tctcttctca tggacgcgca tgaaattcgg tgccgtgact 120
   cggatcgggg gacctccctt gggagatcaa tcccctgtcc tcctgctctt tgctctgtga 180
   gaaagatcca cctaccacct caggtcctta gacagacaag cccaagaaac atctcaccaa 240
   tttcaaatcc ggtaagtggc cactttttac tctcttttcc aatctccctc actatccctc 300
   aacctctttc tcctttcaat cttggcgtca cacttcaatc tctcccttct cttaatttca 360
   attcctttca ttttctggga gagacaaagc agacacgttt tatcggtgga cccaaaactc 420
   cagcgcctgt cacggactgg gaaggcagcc ttcccttggt gtttaatcat tgcagggaca 480
   cctctctgat tattcaccca cgtttcaaag gtgtcagacc acgcagggat gcctgccttg 540
   gtccttcacc cttagcagca agtcccgctt ttctggggga ggggcaagtt ccccaactcc 600
   ttctctccct ctctacccct tctctgcttt tctggggaag gggcaagtac ccctcaaccc 660
   cttctccttc gcccttagcg gcaagtcccg cttttctagg gggcaagaac ccccaatccc 720
   ttatttccgc accccaacct cttatttctg tgccccaatc ccttatttcc atgccccgac 780
   cccccttccc acttttctgg agggtaagaa cccccgaacc ccttccctcc atatctctat 840
   gctctctttt ctctgggttt gcctccttca ctatgggcaa ccttccacca tccattcctc 900
   cttctccctt agcctgactt ctcacgaact taaaacctct tcaactcaca cctgacctaa 960
   aacctaaacg ccttattttc ttctgcaatg ctgcttgacc ccaatacaaa ctcaacagta 1020
   gttccaaata gccaaaaaat ggcactttga atttttccat cctgcaaaat ctaaataatt 1080
   cttgctgtaa aatagacaaa tggtctgagg tgcctgatgt ccaggcattc ttttacacat 1140
   cagtcccttc ctagtctctg tgcccaatgc aactcgtacc aaatcttcct tctttccctc 1200
   ccgcctgtcc cctcagtccc aaccccaagc gtcactgagt ctttctaatc ttccttttct 1260
   acagacccat ctgacctctc ccctcctctc caggccaagc taggtcccaa ttcttcctca 1320
   gcctcccctc ctccacccta taatcctttt atcacctccc ctcctcacac cctgtctggc 1380
   ttatagtttc gttcagtgac tagcccttcc ccacctgtcc agcaatttat tcttaaaaag 1440
   gtggctgaag ctaaaggcat agtcaaggtg aatgctcctt tttctttatc ccaaatcaga 1500
   tagcgtttag gctctttttc atcaaatata aaaatccagc ccagttcatg acttgtttgg 1560
   ctgcaacctt gagacgcttt acagccctag accctaaaag gtcaaaaggc catcttattc 1620
   tcaaaataca ttttattacc caatgtgctc ccgacattaa ataaaattcc aaaaattgga 1680
   atctggccct caaaccccac aacaggattt aattaacctc accttcaagg tgtacagtaa 1740
   cagaaaaaaa gttgcaattc cttgcctcca ctgtgagaca aaccccagcc acatctccag 1800
   cacacaagaa cttccaaatg cctgaaccac agcagccagg tattcctcca gaacctcctt 1860
   ccccaggagc ttgctacacg tgccggaaat ctggccactg ggccaaggaa tgccggcagc 1920
   ccgggattcc tcctaagccg cgtcccatct gtgtgggacc ccactgaaaa tcggactgtt 1980
   caactcacct ggcagccact cccagagccc ctggaactct ggcccaaggc tctctgactg 2040
   actccttctc ggcttagcgg ctgaagactg acgctgcctg atcacctcga aagccccgta 2100
   gaccatcaca gacgccgagc ttcgagtaac tctcacagtg gaaggtaagt ccgtcgcctt 2160
   aatcaatacg gaggctaccc actccacatt accttctttt caagggcctg tttcccttgc 2220
   ctccataact gttgtgagta ttgacggcca ggcttctaaa cctcttagaa ctccccaact 2280
   ctggtgccaa cttagatgat actcttttaa gcactccttt tagttatccc cacctgccca 2340
   gttcccttat taggccgaga cactttaact aaattatctg cttccctgac tattcctgga 2400
   ctacagctac atctcattgc cacccttctt cccaatccaa agcctccttt gcatcctcct 2460
   cttgtattcc cccaccttaa cccacaagta taagatacct ctactccctc cttggcgact 2520
   gatcatgcac cccttaccat ctcattaaaa ccttatcacc cttaccctgt tcaatgccaa 2580
   tatcccatcc cacagcatgc tttgaaagga ttaaagcctg ttatcactcg cctgctacag 2640
   catggccttt taaagcctat aaactctcct tacaattccc ccattttgcc tgtcctaaaa 2700
   ccagacaagc cttacaagtt agttcaggat ctgtgcctta tcaaccaaat tgttttgcct 2760
   atccacccta tggtgtcaaa cccatatact ctcctatcct ccatacctcc ctccacaatc 2820
   cattattatg ttctggatct caagcatgct ttctttacta ttcctttgct cccatcatcc 2880
   cagcctctct tcgctttcac ttggactgac cctgacaccc attaggctca gcaaattacc 2940
   tgggctgtac tgctgcaagg cgtcacagac agcccccatt acttcaatca agcccaaatt 3000
   tcatcctcat ctgttaccta tctcagcata attcttcata aaaacacagg tgctccccct 3060
   gctgctcgtg tccgattaat cccccaaacc tcaatccctt acaaaacaac tccttttctt 3120
   cctaggtatg gttag 3135
<210> 2
   <211> 5682
   <212> DNA
   <213> Homo sapiens
<400> 2
   atatcccctg tgacctgcac gtatacatcc agatggctga ttcctgatga cattccacca 60
   caaaagaagt gaaaatggcc tcttcctgcc ttaactgatg acattatctt gtgaaattcc 120
   ttctctgggc tcatcctggc tcaaaagctc ccctactgag caccttgtga cctctactcc 180
   tgcccgccag agaacaacct tcctttctcc tttacctacc taaatcctat aaaacggccc 240
   caccccatct cctttcgctg actcttttcg gactcagccg gcctgcaccc aggtgaaata 300
   aacagcttta ttgctcacac aaagcctgtt tggtggtctc ttcacatgga cgcgagtgaa 360
   atttagtgcc gtgactccga tcgggggacc tcccttgggg gatcaatccc ctgtcctcct 420
   gctctttgct ccgtgagaaa gatccaccta tgccctcagg tcctcagacc aaccagacca 480
   agaaacatct caccaatttc aaatccgaaa agcggcctct ttttactctc ttctccaacc 540
   tccctcacta tccctcaacc tctttctcgt ttcaatcttg gtgtcacact tcaatctctc 600
   ccttctctta atttcaattc ttttcatttt ctggtagaga caaaggagac acgttttatc 660
   cgtggaccca aaactccggc gccggtcatg gactagggaa ggcagccttc ccttggtgtt 720
   taatcactgc aaggacacct ctctgattat tcacccaggt ttcagacgtg tcagaccacg 780
   cagggacacc tgccttcgtc cttcaccctt agcggcaagt cccacttttc tgggggaggg 840
   acaggaaccc cgacctctta tctctgcgcc ccatccctta tttccgtgcc ccgacctctt 900
   atctctgtgc cacaacccct tatttccatg ccccaacccc ttctctgctt ttctggagga 960
   caagaacccc ccaccccttc tccgtgtctc tactctcttt tctctgggct tgcctccttc 1020
   actataggca agcttccacc ttccattcct ccttcttcct tagcctgtgt tcttaagaac 1080
   ttaaaacctc ttcaactctc acctgaccta aaatctaagc gtcttatttt cttctgcaat 1140
   gccgcttgac cccaatacaa actcgacagt aattccaaat agccggaaaa cggcactttc 1200
   aatttttcca tcctacaaga tctaaataat tcttgttgta aaataggcaa acggtttgag 1260
   gtgcctgacg tccaggcatt cttttacaca ttggtccctc cctagtccct gttcccaatg 1320
   tgactcgtcc caaatcttcc atctttccct cccacctgtc ccctcagtcc caaccccaag 1380
   catcgctgag tctttgtaat cttccttttc tgcagaccca tctaacctgt cccctcctcg 1440
   ccaggccgag ctaggtccca attcttcctc agcctctgct cctccaccct ataatccttt 1500
   tatcacctcc cttcctcaca cccggtccgg cttacagttt cgttccgtga ctagcccttc 1560
   cccacctgcc cagcaattta ctcttaaaaa ggtgactgga gctaaaggca tagtcaaggt 1620
   taatgctcct ttttctttat cccaaatcag atagcgttta ggctcttttt catcaaatat 1680
   aaaaatccag cccagttcat ggcttgtttg gcagcaaccc tgagacgctt tacagcccta 1740
   gatcctaaaa ggtcaaaagg ccgtcttatt ctcaatatac attttattac ccaatctgct 1800
   cccgacatta aatgaaactc caaaaattaa attccagccc tcaaacccca caacaggact 1860
   taattaacct cgccttcaag gtgtacaata atagagtaga ggcagccaag tagcaacata 1920
   tttctgagtt gcaattcctt gcctccactg tgagacaaac cccagccaca tctccagcac 1980
   acaagaactt ccaaacacct aaaccgcagt ggccaggcgt tcctccagaa ctgcctcccc 2040
   caggagcttg ctacaagtgc cagaaatctg gccaccaggc caaggaatgc ccgcagccca 2100
   ggattcctcc taagccgtgt cccatctgtg caggacccca ctggaaatcg gactgttcaa 2160
   ctcacctggc agccactccc agagcccctg gaactctggc ccaaggctct ctgactgact 2220
   ccttcccaga tcttctcggc ttagcggctg aaggctgatg ctgcccgatc gcctcggaag 2280
   ccccgtagac catcacggat gccgagcttt aggtaactct cacagtggaa ggtaaatcca 2340
   tcctcttctt aatcaatatg gaggctatgc actccacatt accttctttt taagggcctg 2400
   tttcccttgc ctccataact gttgtgggta ttgacagcca ggcttctaaa cctcttaaaa 2460
   ctccccaact ctggtgccaa attagacaat actcttttaa gcactccttt ttagttatcc 2520
   ccacctgccc agttccctta ttaggccgag acactttaac taaattatct gcttccctga 2580
   ctattcctgg actacagcta catctcattg ccgcccttct tcccaatcca aagcctcctt 2640
   tgcgtcctcc tcttgtatcc ccccaacctt aacccacaag tataagatac ctctactccc 2700
   tccttggcga ccgatcatgc accccttacc atctcattaa aacctaatca cccttaccct 2760
   gctcaatgcc aatatcccat tccacagcat gctttgaaag gattaaagcc tgttatcact 2820
   cgcctgctac agcatggcct tttaaagcct ataaactctc cttacaattc ccccatttta 2880
   cctgtcctaa aaccagacaa gccttacaag ttagttcagg atttatgcct tatcaaccaa 2940
   attgttttgc ctatccgccc catggtgcca aacccatata ctctcctatc ctcaatacct 3000
   ccctccacaa tccattattg tgttctggat ctcaaacatg ctttccttac tattcctttg 3060
   cacccttcat cccagcctct ctttgctttc acttggactg accctgacac ccatcaggct 3120
   cagcaaatta cctaggctgt actgccacaa acttcagaca gcccccatta cttcagtcaa 3180
   gcccaaattt cttccttatc tgttacccgt ctcggcataa ttctcataaa aacacacgtg 3240
   ctctccctgc tgatcgtgtc tgactaatct ctcaaacccc aacctcttct acaaaacaac 3300
   aactcctttc tttccggggc atggttggat actttcgcct ttggatacct ggttttgcca 3360
   tcctaacaaa accattatat acactcacaa aaggaaacct agctgacccc atagatccta 3420
   aatcctttcc ccactcctct ttccgttcct tgaagacagc tttaaagact gcccccaccc 3480
   tagctttccc tgattcatcc caaccctttt cattacacac agctgaagtg cagggttgtg 3540
   cagtcggaat tcttacacaa ggaccgggat cgcgtcctgt agcctttttg tccaaacaac 3600
   ttgaccttac tgttttaggc tggccatcat gtctccatgc agcggctgct gcctccctaa 3660
   tacttttaga ggcccttaaa atcacaaact atgctcaact cactctctac agctctcata 3720
   atttccaaaa tctattttct tcctcacacc tgttgcatat actttctgct ccccggctcc 3780
   ttcagctgta ctcactcttt gttgagtctc ccgcaattac cattgttcct ggcccgggct 3840
   tcaatctggc ctcccacatt attcctgata ccacacctga ccctcatgac tgcatctctc 3900
   tgatccacct gatgttcacc ccatttcccc acatttcctt cttccctgtt tctcaccctg 3960
   atcacacttg gtttattgat ggcagttcca caggcgtaat cgccaaacac cagcaaaggc 4020
   aggctatgct atagtacaag ccactagccc acctcttaga acctctcatt tcctttccat 4080
   catggaaata tatcctcaag gaaataactt ctcagtgttc catgtgctat tctactactc 4140
   ctcagggatt attcaggccc cctcccttcc ctacacatca agctcaggga tttgccccca 4200
   cccaggactg gcaaattggc ttcactcaac atgccccgag tcaggaaact aaaatacctc 4260
   ttggtctagg tagacacttt cactggatag gtagaggcct tctccacagg gtctaagaag 4320
   gccaccacgg tcatttcttc ccttctgtca gacataattc ctcggtttgg ccttcccacc 4380
   tctatacagt ccaatagcag accggccttt attagtcaaa tcagctaagc attttttcag 4440
   gctcttagta ttcagtgaaa cctttatatc ccttacagtc ctcagttttc aggaaaggta 4500
   gaacagacta atggtctttt aaaaacacac ctcaccaagc tcagccacca acttaaaaag 4560
   gactggacga tacttttacc acttcccctt ctcagaattt aggcctgtcc tcagaaagct 4620
   acagggtaca gcccatttga gctcctgtat agaggctcct ttttattagg ccccagtctc 4680
   attccagaca ccagaccaac ttggactgtg ccccaaaaaa cttgtcatcc ctactatctt 4740
   ctgtctaatc atactcctat caccgttctc aactactcac acatgccctg ctcttgttta 4800
   cactgccagt ttacactgtt tctccaagcc atcacagctg atatctcctg gtgctatccc 4860
   caaaccacca ctcttaactc ttgaagtaaa taaataattt ttgctggcaa ggctatgctg 4920
   aacctcctta ggcactctct aattagatgt cccaggtcct cccaattctt agtcctttaa 4980
   tacctgtttc tctccttctc ttattccgtt tagtttttca attcatacaa aaccgtatcc 5040
   aggccatcac caataattct aaatgacaaa tgtttcttct aacaacccca caatatcacc 5100
   ccttaccaca aaatcttcct tcagcttaat ctctcccact ctaggttccc acgctgcccc 5160
   aatcccgctc gaagcagccc tgagaaacat cgctcattct ctctccatac catccccaaa 5220
   aaattttcac tgtcctaaca ctttaccact atttcgtttt atttttctta ttaatataag 5280
   aagacaggaa tgtcaggcct ctgagcccaa gctaagccat catatcccct gtgacctgca 5340
   cgtacacatc cagatggctg gttcctgcct taactgatga cattccacca caaaagaagt 5400
   gaaaatggcc tgttcctgcc ttaactgatg acaatatctt gtgaaattcc ttctcctggc 5460
   tcaaaagctc ccctactgag caccttgtga cccccactcc tgcctgccag agaacaaccc 5520
   ccctttttcc tttacctacc caaatcctat aaaacagccc cactcctatc tcccttcgct 5580
   gactcttttc ggactcagcc cgcctgcacc caggtgaaat aaacggcttt attgctcaca 5640
   caacgcctgt ttggtggtct cttcacacgg acgcaagtga aa 5682
<210> 3
   <211> 486
   <212> DNA
   <213> Homo sapiens
<400> 3
   agaccactac tactcctgct gccctcctcc ccccaccttg cctagtttac aagacaggag 60
   gaaagagaga aagcaaaaag ttagaaaaca aaacaaaaca gaagtaagat aaatagccag 120
   aagaccttgg cgacaccacc cggccctggt agttaaaaaa aagtaacaat aataataata 180
   tcaacccctg acctaaacta cttgtgttat ctgtaaattc cagacattgt atgaaaaagc 240
   attgcaaaac tttctgctct gttagctgat gcgtgtagcc cccagtcacg ttccccgctt 300
   gcttgagata tcatgaccct ttcacgtgga ccccttagag ttgtaagcct ttaaaaaggc 360
   caagaatttc tttttcaggg atctcagcta ttaagatgca agtctgccaa tgctcctggc 420
   caaataaacc tcttccttct ttaatccggt gcctgaggag ttttgtctgt ggctcgtcct 480
   gctaca 486
<210> 4
   <211> 327
   <212> DNA
   <213> Homo sapiens
<400> 4
   tgggtcaggc acaaagtaag cccaccccac caggaactat gttgaaaaat ttcaagaaag 60
   gatttaaggg agattatggt gttactctga caccaggaaa agttagaact ttatgtgaaa 120
   tagactggcc agcattagag gtggatttcc catcagaagt aagccttgac gggtccgttg 180
   tttcaaaggt atggcacaag gtaacctgta agccagggca cgcaaaccag ttcctgtaca 240
   tagacacttg gttacagctg gttttagatc cgcctcacag tggttgagag aacagcagca 300
   taagcagctg acagaggcaa ggaaaga 327
<210> 5
   <211> 6146
   <212> DNA
   <213> Homo sapiens
<400> 5
   gtcaggcctc tgagcccaag ctaagccatc atatcccctg tgacctgcac gtacacaccc     60
   agatggccag ttcctgcctt aactgatgac attccaccac agaagaagtg aaaatggcct 120
   gttcctgcct taactgatga cactgtcttg tgaaattcct tctcctggct catcctggct 180
   cagaagctcc cctactgagc accttgtgac ccccactctg cctgccagag aacccccatt 240
   tgactgtaat tttcctttat ctacccaaat cctataaaac ggccccaccc ctatctccct 300
   tctctgactc tcttttcaga ctcagccagc ctgcacccag gtgattaaaa gctttattgc 360
   tcacacaaag cctgtttggt gatttcttca cacagactca catgaaattt ggtgccatga 420
   ctcggatcgg gggacctccc ttgggagatc aatcccctgt cctcctgttc tttgctccgt 480
   gaaaaagatc cacctacgac ctcaggtcct cagacccacc agcccaagga acatctcacc 540
   aattttaaat caggtaagtg gcctcttctt actctcttct ccaacctctc tcactatccc 600
   tcaaccactt tctcctttcc actcttcagt ctctcccttc tcttaatttc aattcctttc 660
   attttctggt agagacaaag gagacacgtt ttgtctgtgg acccaaaact ccggcgccgg 720
   tcacggactg ggaaggcagc cttcccttgg tgtttaatca ttgcagggac acctctctga 780
   ttattcaccc acgtttcaga ggtgtcagac cacgcaagga tgcctgcctt ggtccttcac 840
   ccttagcggc aagtcccgct tttctagggg aggggcaagt accccaaccc cttctctcca 900
   tgtctctacc ccttctctgc ctttctgggg ggcaagaaac ccccaatccc ttctccttca 960
   cccttagtgg caagtcccgc ttttctggtg gagaggcaag taccccaacc tcatatctct 1020
   gtgccccgat cccttatttc tgtgccccga cctcttatat ctctgcgccc tgatccctta 1080
   tttctgcggc ccgacctctt atatctctgt gccctgatcc cttatttccg ctccccaccc 1140
   tcttatatct ctgtctcctg atcccttatt tccatgctcc gacctcatat ctctgcgccc     1200
   tgaccccttt cctgcttttc tggagggtaa gaacccccga accccttccc tccatgtctc     1260
   cactctctct tttctctggg cttgcttcct tcactatggg caaccttcca ccttccattc 1320
   ctccttcttc tcccttagcc tgtgttctca aaaacttaaa acctcttcaa ctcacacctg 1380
   acctaaaacc taaatgcctt attttcttct gcaatgccac ttgaccccaa tacaaactcg 1440
   acagtagttc caaatagcca gaaaacagca ctttcaattt ttccatcctg caagatctaa 1500
   ataattcttg tcctaaaatg ggcaaacggt ctgaggtgcc tgatgtccag gcattctttt 1560
   acacatcggt cccttcctag tctctgtgcc cagtgaaact catcccaaat cttccttctt 1620
   tccctctcgc ctgtcccctc agtcccaacc ccaagcgtcg ctgagtcttt ctaatcttcc 1680
   ttttctacag acccatctga cctctcccct cctccccagg ctgctcctcg ccaggccgag 1740
   ctaggtccca attcttcctc agcctccgct cctccaccct ataatctttt tatcacctcc 1800
   cctcctcata cccggtccgg tttacagttt cattccgtga gtagccctcc cccacctgcc 1860
   cagcaatttc ctcttaaaaa ggtggctgaa gctaaaggca tagtcaaggt taatgctcct 1920
   ttttcttcat cagacctctc ccaaatcctg agcatttagg ctctttcatc aaatatgaaa 1980
   aacccagccc agttcatggc ttgttcagca gcaaccctga gacgctttac agccctagac 2040
   cctgaaaggt caaaaggccg tcttattctc aatatacatt ttattaccca atctgctcct 2100
   gacattaaat aaaactccaa aaattaaatt ccggccctga aaccccacaa cagggcttaa 2160
   ttaacctcac cttcaaggtg tacaataata gagacagcca agtagcaaca tatttctgag 2220
   ttgcaattcc ttgcctccac tgtgagacaa accccagcca catctccagc acacaagaac 2280
   ttccaaacac ctaaactgca gtggccaggt gttcctccag aaccgccttc cccaggagct 2340
   tgctataagt gccagaaatc tggacaccag gccaaggaat gcccacagcc cgggattcct 2400
   cctaagccat gtcccatctg tgcgggaccc cactggaaat tggactgttc aactcacctg 2460
   gcagccactc ccagagcccc tggaactctg gcccaaggct ctttgactga ctgcttccca 2520
   gatctcggct tagcagctga agactgccac tgccagatcg cctcgaagcc tacaagacca 2580
   ttacagacac tctgggtaac tctcacagtg gaaagtaagt ccgtcccctt cttaatcaat 2640
   acggaggcta cccaccccac attaccttat tttcaaaggc ctgtttccct tgcttccata 2700
   actgttgtgg gtattgatgg ccaggctttt aaacctgtta aaactcccca actctggtgc 2760
   caacttagac aatattcttt taagcactcc ttttcagtta tccccacctg cccagttccc 2820
   ttattaggcc gagatatttt aaccaaatta tctgcttccc tgactattcc tggactacag 2880
   ccgcatctca ctgctgccct tctccccaac ccaaagcctc cttcgcgtct tcctctcata 2940
   tccccccacc ttaacccaca agtatgggac atctctactc cttccctggc aacagatcac 3000
   atgcccatta ccatcccatt ataacctaat cacccttacc ccgctcaaca ccaatatccc 3060
   atcccatagc acgctttaaa aggattaaag cctgttatca ctcgcctgct acagcatggg 3120
   cttctaaaac ctataaactc tccttacaat tcccccattt tacctgtcct aaaaccgcac 3180
   aagtcttaca ggttagttca ggatctgcgc cttatcaccc aaattgtttt gcctatccac 3240
   cctgcggtgc ccaacccgta cactcttttg tcctcaatac cttcctccac aactcactat 3300
   tccattcttg atcttaaaga tgcttttttc actattcccc ttcacccctc gtcccagcct 3360
   ctctttgctt tcacctggac tgaccctgac acccatcagt cccagcagct tacctgggct 3420
   gtgctgccgc aaggtttcag ggacagccct cattacttca gccaagctct ttctcatgat 3480
   ttactttctt tctacccctc cgcttctcac cttattcaat atattgatga ccttcttctt 3540
   tgtagcccct cctttgaatc ttctcaacaa gacacacttc tgctccttca gcatttattc 3600
   tccaaaggat atcgggtatc cctctccaaa gctcaaattt cttctccatc cgttacctac 3660
   cttggcataa ttcttcataa aaatacacgt gctctccctg ctgatcgtgt tggactaatc 3720
   tctcaaaccc caaccccttc tacaaaataa caactccttt ccttcctggg catggttgga 3780
   tattttcgcc tttagatacc tggttttgcc atcctaacaa aaccattata taaactcaca 3840
   aaaggaaacc tagctgaccc catagatcct aaatcctttc cccactcctc tttctgttcc 3900
   ttgaagacag ctttagagac tgcccccact ctagctctcc ctgactcatc ccaacccttt 3960
   tcattacaca cagccgaagt gaagggccgt gcagtcagaa ttcttacaca aggaccggga 4020
   tcacgtcctg tagccttttt gctcaaacaa cttgacttta ctgttttagg ctggccatca 4080
   tgtctccgtg cagcggctgc tgccgcccta atacttttag aggcccttaa aatcacaaac 4140
   tatgctcaac tcactctcta cagctctcat aatttccaaa atctattttc ttcctcacac 4200
   ctgacacata cactttctgc tccccagctc cttcagcagt actcactctt tgttgagtct 4260
   cccacaatta ccattgttcc tggcccggac ttcaatccag cctcccacag tattgctgat 4320
   accacacctg accctcatga ctgcatctct ctgatccacc tgacgttcac cccatttccc 4380
   cacatttcct tcttccctgt ttctcaccct gatcacagtt ggtttattga tggcagttcc 4440
   accaggccta atcgccactc accagcaaag gcaggctatg ctatagtaca agccactagc 4500
   ctgcctctta aaacctctca tttcctttcc attgtggaaa tctatcctca aggaaataac 4560
   ttctcaatgt tccatctgct attctactac tcctcaggga ttattcaggc cccctccctt 4620
   ccctacacat caagctcgag gatttgcccc cacccaggac tggcaaatta gctttactca 4680
   acatgccccg agtcagataa ctaaaatacc tcttagtcta ggtagatact ttcactggat 4740
   aggtacaggc ctttcctaca gggtctgaga aggccactgc agtcatttct tcccttctgt 4800
   cagacataat tcctcagttt agccttccca cctctataca gtctgacaac ggaccagcct 4860
   ttattagtca aatcagacaa gcagtttttc aggctcttag tattcagtga aacctttata 4920
   tcccttacgg tcctccgtct tcaagaaaag tagaacggac taaaggtctt ttaaaaacac 4980
   acctcaccaa gctcagccac caacttaaaa aggactggac aatactttta ccactttccc 5040
   ttctcagaag tcagacctgt cctcagaatg ccacaaggta cagtccattt gagctcctgt 5100
   atggacactc ctttttatta ggccccagtc tcattccaga caccagacca acttagactg 5160
   tgccccaaaa aaacttgtca tccctactat cttctgtcta gtcatactcc tattcactgt 5220
   tctcaactac tcatacatgc cctgctcttg tttacactgc cggtttacac tgtttctcca 5280
   agccatcaca gctgatatct cctggtactg tccccaaact gccactgtaa actcttgaag 5340
   taaataaata atctttgctg gcaggactat gctgaatctc cttaggcact ctctaatcag 5400
   atgtcctggg tcctcccaat tcttagacct tttatacctg tttttctcct tctcttattc 5460
   catttagttt ttcaattcat acaaaactgt atccaggcca tctccaataa ttctacacga 5520
   caaatgtttc ttctaacaac cccacgacat cacctcttac cacaaaatct tccttcagct 5580
   taatctctcc cactctaggt tcccacgctg cctctaatac cgcttgcagc agccctgaga 5640
   aacatcgccc attatctctc cacaccaccc ccaaaaattt tcaccatccc aacactttac 5700
   cactatttca ttttattttt cttattaaca taagaagaca ggaatgtcag gcctctgagc 5760
   ccaagctaag ccatcatatc ccctgtgacc tgcaggtaca cacccagatg gctggttcct 5820
   gccttaactg atgacattcc accacaaaag aagtgaaaat ggcctgttcc tgccttaact 5880
   gatgacattg tcttgtgaaa ttccttctcc tggctcatcc tggctcaaaa gctcccctac 5940
   tgagcacctt gtgaccccca ctttgcccgc cagagaacaa cccccctttg actgtaattt 6000
   tcctttatct acccaaatcc tataaaatgg ctataccctt atctcctttc tctgactctc 6060
   ttttcgaact cagcctgcct gcacccaggt gaaataaaca gccatgttgc tcacacaaag 6120
   cctgtttggt ggtctcttca cactga 6146
<210> 6
   <211> 3184
   <212> DNA
   <213> Homo sapiens
<400> 6
   gtcaggcctc tgagcccagg ccaggccatc gcatcccctg tgacttgcac gcatacatcc     60
   agatggcctg aagtaactga agatccacaa aagaagtaaa aacagcctta actgatgaca    120
   ttccaccatt gtgatttgtt cctgccccac cctagctgat caatgtactt tgtaatctcc    180
   cccaccctta agaaggttct ttgtaattct ccccaccctt gagaatgtac tttgtgagat    240
   ccacccctgc ccagcagaga acaaccccct ttgactgtaa ttttccatta ccttcccaaa    300
   tcctataaaa cggccccacc cctatctccc ttccctgact ctcttttcgg acgcagcccg    360
   cctgcgccca ggtgaaatac acagccatgt tgctcacaca aagcctgttt ggtgggctct    420
   tcacacggac acgtatgcaa tttggtgccg tgactcggat cgggggacct cccttgggag    480
   atcaatcccc tgtcctcctg ctctttgctc cgtgggaaag atccacctat gacctcaggt    540
   cctcagaccg accagcccaa gaaacatctc accaatttca aatccggtaa gcggcctctt    600
   tttactctct tctccaacct ccctcactat acccttagcg gcaagtcccg ctttcctggg    660
   gcaggggcag gtacccctca accccttctc cttcaccctc agcggcaagt cccgctttcc  720
   tggggcaggg gcaggtgccc ctcgacccct tctccttcac cctcagcggc aagtcccgct    780
   ttcctggggc aggggcaggt gcccctcaac cccttctcct tcaccctcag cggcaagtcc    840
   cgctttcctg gggcaggggc aggtgcccct cgaccccttc tccttcaccc tcagcggcaa    900
   gtcccgcttt cctggggcag gggcaggtac ccctcgaccc tttctccttc accctcagcg    960
   gcaagtcccg ctttcctggg gcaggacttg ccgctttcct ggggtaggga caagtacccc   1020
   tgaacccctt ctccacatta cctgcttttc aagggcctgt ttcccttgcc tccataactg   1080
   ttgtgggtat tgacagccag gcttctaaac ctcttaaaac tcccccactc tggtgccaac   1140
   ttggacgaca ctcttttatg cactcttttt tagttatccc cacctgccca gttcccttat   1200
   taggccgaga tattttaacc aaattatctg cttccctgac tattcctgga ctacagctgc   1260
   aactcattgc tgcccttctc gctaacccaa agcctccttc gcgtctttct ttcatatccc 1320
   cccaccttaa cccacaagta tgggacatct ctactccttc cctggcaact gatcacatgc 1380
   ccattaccat cccgttaaaa cctaatcacc cttaccccac tcaacgccag tatcccatcc 1440
   cacagcatgc tttaaaagga ttaaagcctg ttatcactcg cctgctacag catgggcttc 1500
   taaaacctat aaactctcct taccattccc ccattttacc tgtcctaaaa ccagacaggg 1560
   cttacaggtt agttcagaat ctgtgcctta tcaaccaaat tgttttgcct atccaccccg 1620
   tggtgccaaa cccatatact ctcctatcct caatacctgc ctctacaacc cattattctg 1680
   ttctggatct caaacatgct ttctttacta ttcctttgca cccttcatcc cagcctctct 1740
   ttgctttcac ttggactgac cctgacaccc attaggctca gcaaattacc taggctgtac 1800
   tgccgcaagg cttcacagac agcccccatt acttcaatca agcccaaatt tcttcctcat 1860
   ctgttaccta tctcggcata attctcataa aaacacacgt gctctccctg ccaatcgtgt 1920
   ccgactgatc tctcaaaccc cggcaccttt ctacaaaaca ataactcctt tccttcctag 1980
   gcatggttag cgtggtcgta attcttacac aagagccagg accacaccct gtagcctttc 2040
   tgtccaaaca acttgacctt actgttttaa cctagccctc atgtctgcgt gcagcggctg 2100
   ccgctgcttt aatactttta gaggccctca aaatcacaaa ctttatcagt cctccaggcc 2160
   caagttgact cttcagctgc agttgtcctc caaaaccgcc aaggccttga ctgacttact 2220
   gctgaaaaag gaggactctg catattctta aatgaggagt gttgttttta cctaaatgca 2280
   tctggcctgg tgtatgacaa cataaaaaaa ctcaaggata gagcccaaaa acttgccaac 2340
   caagcaagta attacgctga acccccttgg gcactctcta attggatgtc ctgggtcctc 2400
   ccaattctta gtcctttaat acccattttt ctcctccttt tattcggacc ttgtatcttc 2460
   cgtttagctt ctcaattcat ccaaaaccgt atccaggcca tcaccaatca ttctatacga 2520
   caaatgtttc ttctaacatc cccacaatat caccccttac cacaagacct cccttcagct 2580
   taatctctcc cactctaggt tcccacgccg cccctaatcc cgctcgaagc agccctgaga 2640
   aacatcaccc gttctctctc cataccaccc cccaaaaatt tccgccgctc caacacttca 2700
   acgctatttt gttttatttg tcttattaat ataagaaggc aggaatgtca ggcctctgag 2760
   cccaggccag gccatcacat cccgtgactt gcacgcatac atccagatgg cctgaagtaa 2820
   ctgaagatcc acaaaagaag taaaaacagc cttaactgat gacattccac cattgtgatt 2880
   tgttcctgcc ccaccctaac tgatcaatgt actttgtaat ctcccccacc cttaagaagg 2940
   ttctttgtaa ttctccccac ccttgagaat gtactttgtg agatccaccc ctgcccagca 3000
   gagaacaacc ccctttgact gtaattttcc attaccttcc caaatcctat aaaacggccc 3060
   aacccctatc tcccttccct gactctcttt tcggacgcag cccgcctgcg cccaggtgaa 3120
   ataaacagcc atgttgctca cacaaagcct gtttggtggg ctcttcacac ggacgcgcat 3180
   gaaa 3184
<210> 7
   <211> 1847
   <212> DNA
   <213> Homo sapiens
<400> 7
   actttcttgc tcagccccaa cctcttccca gacaccagcc ctctaggcga ctatcttcca      60
   gtcctccagc aggctagata ggaaattcgc taggttgcta atcttctctt gcctactcca     120
   gattcccagc catataaaga cacgctagtg ggaatcagtt cttgttaaga atccgatccc     180
   tcaaactcta caacctcatt ggaccagacc ctacttagtc atctataata ccccaactgc     240
   ctccacctgc aggaccctcc ccactgggtt taccattcca gaataaagct gtgtccatca     300
   gacagccagc ttgatctctc ctcttcctcc tagaagtcgc aagtacttat ccctacttcc     360
   cttaaagtca cccacatttc taaagaacag tagtgaccct tatgagccta atacatcctt     420
   tcattttgtt aggtctattc atccttaccc tactctttgc aatagtgctt tatgcagtca     480
   cccctactac ttaaactgca tcctaaaaac ttttcatcct tgctgtcttc tgtctagtca     540
   tactcttatt cttcgttctc acttatccat gagtgcccac ccttgtctac actactggct     600
   tatatttttt ctccaaacca tcatagctgg tcctggtctt atcccctaac caccactctt     660
   aactccctct tacagtggat aaatgacctt tgctgaaaaa acacactcca attctttccc     720
   ctactttaca tttctagttt tgccttacac aaggtctctt cttcctctgt ggctcctcta     780
   cctacatgtg tctacctgtt aattagacag gcacatgtac actagttttc cttaccccta      840
   aaaatcagtt tgcaaatatt accgaacagc ttcctgttcc cctcatgaca ccaatacttc     900
   accactatct tgttttgttt ttcttattat taatacaata agacgggaat aggtcttgac     960
   ttactgctga aaaaggagga ctctgtatat ttttaaatga aaagtgttgt ttttacctaa    1020
   atcaatctgg catggtacat ggcaacataa aaaaaaactc aaagataggg cccaaaaact    1080
   caccaaccaa gcaaataatc atgctgaacc tccttgggca ctctctaatt ggatgtcctg    1140
   ggtcctccca actcttagtc ctctaatacc tgtttttctc cttctcttat tcagtccttg    1200
   tgtcttccgt ttagtttctc aattcataca aaaccgtatc gaggccatca cctatcattc    1260
   tacacaacaa atgctctttc taacaacccc acaatatcac cccttaccac aaaatctttc    1320
   ttcagcttaa tctctcccac tctaggttcc cataccgccc ctaatcccac tcaaagcagc    1380
   cctgagaaac atcgcccatt atctcttcat accacctcca aaaattttca ccaccctaac    1440
   acttcaccac tatctggttt tgcttttctt attaatataa gacaggaatg tcaggccttt    1500
   gagcccaagc ctgcacgtat acatccagat ggcctgaagc aactgaagaa tcacaaaaga    1560
   agtgaaaatg gccgattcct gccttaactg atgatattac cttgtgaaat tccttctcct    1620
   ggctcagaag ctccccgact aagcaccttg tgacccccac ccctgcctac aggagaacaa    1680
   ctccctttga ctgtaatttt ccactaccta cccaaatcct ataaaactgc cccaccccta    1740
   actccctttg ctgactctcc tttcagactc acctgcaccc aggtgattaa aaagctttat    1800
   tactcacaca aagcctgttg gtggtctctt cacacggatg cgcaaga                  1847
<210> 8
   <211> 2668
   <212> DNA
   <213> Homo sapiens
<400> 8
   tgcggtcaga attcttacac aaggaccagg accgcaccct gtagcctttt tatccaaaca      60
   acttgacctt actgttttag cctagccctc aagtctgcgt atggcggcta ccactgccct     120
   aatactttta gaggccctta aaatcacaaa ctatgctcaa ctcactctct acagttctca     180
   taacttccaa aatctatttt cttcctcaca cctgacacat atactgtctg cttcccggct     240
   ccttcagctg tactcactct ttgttgagtc tcccacatta ttccggatac cacacctgac     300
   cctcatgact gcctctctct gatccacctg acgttcaccc catttcccca catttctttc     360
   tttcatgttc ctcaccctga acacacttag cttattgatg gcagttccac caagcctaat     420
   ctccactcac cagcaaaggc aggctatgct atagtatctt ccacatctat cattgaggct     480
   accgctcttt ccccctccac tacctctcag caagccgaac tcattgcctt aagtcaagcc     540
   ctcactcctg caaaaggact aaatgtcaat atttatactg actctaaata tgccttccat     600
   atcctgcacc tccatgcaag aggtttcctc actacacaaa tgtcctctat cattaatgcc     660
   tctttaataa aaatgcttct caaagctgct ttacttccaa aggaagctag agtcattcac     720
   tgcaaaggcc atcaaagggc atcagatccc atcgctcagg acaatgctta tgctgataag     780
   atagctaaaa aagcagctag cattccaact tatatccctc actttcactt tttctccttc     840
   ccctcagtca ctcccaccta ctcccctgct gaaacttcca cctatcaatc tcttcccaca     900
   caaggcaaat agttcttaga ccaaggaaaa tatctccttc cagcctcaca ggcccattct     960
   attctgtcgt catttcataa cctcttccat gtaggttaca agccgctagc ccgtctctta    1020
   gaacctctca tttcctttcc atcctggaaa tctatcctca aggagatcac ttctcagtgt    1080
   tccatctgct attctactac ccctcaggga ttgttcaggc cccctccctt ccctacacat    1140
   caagctcaag gatttgtccc tgcccaggac tggcaagttg actttactca catgccccga    1200
   gtcagaaaac gaaagtatct cttagtctag gtagacactt tcactggata tgtagaggcc    1260
   tttcctacaa ggtctgagaa ggccactgcg gtcatttctt cctttctgtc agacataatt    1320
   ccttggttta gccttcccac ctctatacag tccgatagca gaccggcctt tattagtcaa    1380
   atcagccaag cattttttca ggctcttagt attcagtgaa acctttatct cccttacagt 1440
   cctcagtctt caggaaaggt aaacggacta aaggtctttt aaaaacacac ctcaccaagc 1500
   tcagccacca acttaaaaaa gactagacca tacttttacc actttccctt ctcagaattc 1560
   aggcttgtcc tcggaatgct acaaagtaca gcccatttaa gctcctgttt agacgctcct 1620
   ttttattagg ccccagtctc attccagaca ccagaccaac ttagactgtg ccccaaaaaa 1680
   cttgtcatcc ctactatgtt ctgtctagtc atactcctat tcaccgttct caactactca 1740
   tacatgccct gctcttgttt acactggcag tttacactgt ttctccaagc catcacagct 1800
   gatatctcct ggtgctatcc ccaaactgcc actcttaact cttaaagtaa ataaataatc 1860
   tttgctggca ggactatgct gaatctcttt aggcactctc taattaaatg tcctaggtcc 1920
   tcccaattct tagaccttta atacctgttt ttctccttct cttattctgt ttagttttcc 1980
   aattcataca aaactgtatc caggccatca ccaataattc taaatgacaa atgtttcttc 2040
   taacagtccc acaatatcgc cccttaccac aaaatcttcc ttcagcttaa tctctcccac 2100
   tctaggtccc cacaccaccc ctaatcccgc tcgaagcagc ccattatctc tccataccat 2160
   cccccaaaat tttcgccatc ccaacacttt accactattt cgttttattt ttcttattaa 2220
   tataagaaga caggaatgtc aggcctctga gcccaagcta agccatcata tcccagatgg 2280
   ccagttcctg ccttaactga tgacattcca ccacaaaagt gaaaatggcc tgttcctgcc 2340
   ttaactgatg acattccacc gcaaaagtga aaacggcctg ttcctgcctt aactgatgac 2400
   attatcttga gaaattcctt ctcctggctc atcctggctc aaaagctccc ctactgagca 2460
   ccttgtgacc cccactcctg cccatcagag aacaaccccc ctttgactgt aattttcctt 2520
   taactaccca aatcttataa aacggcccca cccctatctc cctttgctga ctcttttcgg 2580
   actcagcccg cctgcactca ggtgattaaa agctttattg ctcacacaaa gcctgtttgg 2640
   tggtctcttc acacggacac tcatgaaa 2668
<210> 9
   <211> 1441
   <212> DNA
   <213> Homo sapiens
<400> 9
   acaatagtcc agcttttatt agtcaaatca cccaagccgt ctctcaggct ctcggtattc 60
   agtggaacct tcatacccct taccgtcttc aatcttcagg aaaggtagaa tggactaatg 120
   atcttttaaa gacacacctc accaagctca gcctccaact taaagaggac tggacagtac 180
   ttttacttct tgcccttctc agaattagcg cctgtccttg agatgctaca gggtacagcc 240
   cttttgaact tttatatgga tgtgctttct tgcttggccc caaccttgtt ccagacacca 300
   gccctctggg caactatctt ccagtcctcc agcaggctag acagaaaatt caccaggctg 360
   ctaatcttct cacaaccttc cgtagcttct ctaataactt ctctgctagc atcgcagaca 420
   tatcacaaac tttattaatc cttcaggccc aggttgactc tttagctgcg gttgtcctcc 480
   aaaaccgcca aggccttgac ttactcactg ctaaaaaagg aggactctgt atatttttaa 540
   atgaagagtg ttgtttttac ctaaatcaat ctggcctagt atatgacaac ataaaaaaac 600
   tcaaggatag agcccaaaaa cttgccatcc aagtaaataa ttatgctgaa cccccttggg 660
   cactctctaa ttggatgtcc tgggtcctcc caattcttag ccctttaata cctgtttttc 720
   tccttctctt atttgtacct tgtgtcttct gtttagtttc tcaattcata caaaaccaca 780
   cccaggccat caccaatcat tctatacaac aaatgctcct tccaacaacc ccacaatatc 840
   accccttacc ccaaaatctt tcttcagttt aatctctccc actctaatta cccatgccac 900
   cacaatcctg ctcaaagcag ccctgagaaa catcgcccat tatctctcca taccacagcc 960
   aaaatttttt gctgccccaa cacttctcca ctattttgtt ttgtttttcc actattttgt 1020
   tttgttttgt tttgttttgt caggcctctg agcccaggct aagccatcat atcccctgtg 1080
   acctgcaggt atacatctag atggcctgaa gcaactgacg aaccacaaaa gaagtgaaaa 1140
   taggcagttc ctgccttaac tgatgacttt ccaccattgt gatttgttcc tgccccgccc 1200
   caaccaatca atcgaccttg tgacattcct cccctggaca atgagtctca tgatctcccc 1260
   tctgagcacc ttgtgacccc tgcacctgcc tgcaagagaa aacccccttt aactgtaatt 1320
   ttccactgcc tacccaaatc ctataaaact gccccacccc atctcccttt gctgactcct 1380
   ttttcggact cagtctgcct cgcctgcacc caggtgatta aaaagcttta ttgctcacac 1440
   a 1441
<210> 10
   <211> 2376
   <212> DNA
   <213> Homo sapiens
<400> 10
   acaacttgac cttactgttt taggctggcc atcatgtccc cgtgcagcag ctgccgctgc      60
   cctaacactt ttagaggccc tcaaaatcac aaactatgct caactcactc tctatagttc     120
   tcataacttc caaaatctat tttcttcctc acacctgatg catatacttt ctgctccccg     180
   gctccttcag ctgtactcac tctttgttga gtctcccaca gttaccattg ttcctggccc     240
   ggacttcaat ccagcctccc acactattcc ggatacatct gactcccatg actgtatctc     300
   tctgatccac atgacattca ctccctttct ccatgtttcc ttctttcctg ttcctcaccc     360
   tgatcacact tggtttattg atggcagttc caccaggcct aatcgccact caccagcaaa     420
   ggcaggctat gctatagtat cttccacatc tatcactgag gctaccgctc tgcccccctc     480
   cattacctct cagcaagctg aactcactgc cttaacttga gccctcactc ttgcaaaggg     540
   actacccatc aatatttata ctgactctaa atatgccttc catatcctgc accaccatgc 600
   tgttatatag gcagaaagaa gtttcctcac tatgcaagag tcctccatca ataatgcctc 660
   tttaataaga actcttctca aggctgcttt acttccaaag aaagccggag tcattcactg 720
   caaaggccat caaaaggctt cagatcccat tgctctggac aacgcctatg ctgataagat 780
   agctaaaaaa gcagctagcg ttccaacttc tatccctcag ggcagttttc ctccttctca 840
   tctggccact cccacctact ccctcgctga aacttccacc catctcttcc cacacaaagc 900
   aaatggttct tggaccaaag aaaaatctcc ttccagtctc acaggcccat tctattcgtc 960
   atttcataac ctcttccatg taggttgcaa gccgctagcc cgcctcttag aacccgctag 1020
   cccacctctt agaacctctc atttcctttc catcgcaaaa atctatcctc aaggaaatca 1080
   cttttcagtg ttccatctgc tattctacta ctcctcaaga atttctcagg ccccctccct 1140
   tccccacaca tcaagctcgg ggatttgccc cgcccaggac tggcaaattg actttactca 1200
   catgcctcga gtcaggaaac taaaatacct cttggtctgg gtagacactt tcactggatg 1260
   ggtagaggcc tttcccacag ggtctaagaa ggccaccgtg gtcatttatt cccttctgtc 1320
   agacatagtt cctcggtttg gccttctcac ctctatacag tccgataacg gaccggcctt 1380
   tactagtcaa atcacccaag cagtttctca ggctgttggt attcagtggc acctggtttt 1440
   tcctcaaact gccaccctta agtctctctt taagtggata gaagatcttc agtggcaagg 1500
   taccctccaa tactttcacc ctgatgaagt cctattcttt acttttatac ttactcttat 1560
   tctcattccc gttcttatgc caccctctac ctctccccag ctatctctat cacactatca 1620
   atctcagtta ctctctccta gccgtttcta atccttcttt aacaaacaat tgctggcttt 1680
   gcatttctct ttcttccaaa atcacaaagg tctcgactta ctgctaaaaa aaaaaaaaag 1740
   gggactctat atttttaaat gaagagtgct atttttacct aaatcaatct ggcctggtat 1800
   atgacaacat taaaaaaaac tcaaagatag agcctaaaag cttgccaacc aagcaagtaa 1860
   ttacactaac cccccttgga cactctaatt agatgtcctg ggtcctccca attcttagtc 1920
   cttttatacc tgtttttctc cttctcttat tcagaccttg tgtcttccat ttagtttctc 1980
   aattcatcca aaaccatatc caggccatca ccaatcattc tatacgacaa atgtttcttc 2040
   taacaacccg acaattatca ccccttacca caaaatcttc cttcagcttc atctctccca 2100
   cactaggttt ccatgttgcc ccagtcctgc tcaaagcagc cctgagaaac attgcccatt 2160
   atctctccat accacccccc aaaatttccc ccaccccaac actttaccac tattttattt 2220
   ttcttattaa tataagaaga caggaatgtc aggcctctga gcccaagcta agccatcata 2280
   tccccagtga cctgcacgta tacatccaga tggcctgaag caactgaaga tacacaaaag 2340
   aagtgaaaat agccttaact gataacattc caccat 2376
<210> 11
   <211> 6005
   <212> DNA
   <213> Homo sapiens
<400> 11
   gtcaggcctc tgagcccaag ctaacccatc atatcccctg tgacctgcac atatagatcc 60
   agatggcctg aagcaagtga aggatcacaa aagaagtgaa aatggctggt tcctgcctta 120
   actgatgaca ttaccttgtg aaattccttc tcctggctca gcagctcccc cattgagcac 180
   cttgtaaccc ccgcccctgc caccagagaa caaccccttt gactgtaatt ttccactacc 240
   tacccaaatc ctataaaacg gccccacccc tatctccctt tgctgactct ctttttggac 300
   tcagcccgct ggcacccagg tgattaaaaa gctttattgc tcacacaaag cctgtttggt 360
   ggtctcttca cacggacgca catgacattt ggtgccaaag acctgggaca ggaggactcc 420
   ttccagagac ttgtcccctg tcctcgtcct cactccgtga ggagatccac ccacgacctc 480
   aggtcctcag accaaccagc ccaaggaaca tctcaccaat ttcaaatcgg ttaagcggtc 540
   ttttcactct cttctccagc ctctcttgct acccttcaat ctccctctct cgctatcctt 600
   caatctccct gtccttccaa ttccagtttt ttttcctctc tagtagagac aaaggagaca 660
   catttcatcc gtggacccaa aactctggtg ctggtcacgg acttgggaag acagccttcc 720
   cttggtgttt aatcactgtg gggacgcctg cctgattatt cacccacact ccattgttgc 780
   ctgatcacca tggggatgcc tgccttcatt cacccacatt cacttggtgg catgtcaatg 840
   gtgggaatgc ctgctttggc tgctcaccca cactgcagcc cagggctgct caccaccccc 900
   cttctccatg tctctaccct ctcttttctc tgggcttgcc tccttcacta tgggcaacct 960
   tccaccctct attccccctt cttctccctt agcttgtgtt ctcaaaaact taaaatctct 1020
   tcaactcaca cctgatctaa aacctaaaca ccttgttttc ttctgcaatg ccacttgacc 1080
   ccaatacaaa ctcgacaatt gttccaaata gccagaaaat ccactttcaa tttctccatc 1140
   ctacatgatc tagatagttc ttgtcataaa atgagcaaat ggtctgaggt gcctgacgtc 1200
   caggcattct tttacacatc agtccctccc tagtctctgt tcctaatgca actcatccca 1260
   aatcttcctt ctttccctct cgcccgtccc ctcagtccca accccaagcg tcactgagtc 1320
   ttttcaatct tccttttcta ccaaccaatc tgatctctcc cctcttcccc agagtgctcc 1380
   tcctcagttt gctccccgcc aggctgaatc aggctccaat tcttcctcgg cctccactcc 1440
   ccgacactat aatccttcta tcacctccct tccttacacc tgctctggct tacagtttca 1500
   ttctgagact agccctcccc cacctgccca acaatttcct cttaaagagg tggctggagc 1560
   taaaggcata gtcaaggtta atgctccttt tctttatctg acctctccca aagcagttag 1620
   tgtttaggct ctttttcatc aaatgtaaaa atccagccca gtttatggct tgtttagcag 1680
   caaacttgag accctttacc accctagacc ctaaaaggtc agaaagccgt cttattctca 1740
   ataagcattt tattacccaa tccgctcccg acattagaaa aagctccaaa aattagattc 1800
   cggccctcaa accccacaac aggacttaac taacttcacc ttcaaggcat acaataatag 1860
   agttagaggc ggccaagtag caacatattt ctgagttgca attccttgcc tccactgtga 1920
   gagaaacccc agccacatct ccagcacaca agaacttcaa aacgcctaag ccacagtggt 1980
   caggcattct ttcaggacct cctcccccag gatcttgctt caagtgccgg aaatctggcc 2040
   actgggccaa ggaatgcccg cagcccggga tacctcctaa gccatgtccc atctgtgcag 2100
   aaccccactg gaaatcggac agtccaactc acccatcagt cactcccaga gcccctggaa 2160
   ctctggccca aggctctcgg attgacgcct tcccagattt tctcagctta gcggctgaag 2220
   actgacactg cccgattgcc tcggaagtct cctggaagcc tcctggacca tcacatatgc 2280
   tttgggtaac tcttacattg agaggtacat ctgtcacctt cttaatcaat atggaggcta 2340
   cccactccac attaccttct tttcaagggc gtgtttccct tgcctccata actgttgtgg 2400
   gtactgacgt ctgggcttct aaatctctta aaactcccca actctgatgc caacttggac 2460
   aatattcttt tatgcactcc tttttagtta tccccacctt cccagctccc ttattagact 2520
   gacacatctt aagcaaatta tctgcttccc tgatgccacc cttcttccca acccaaagcc 2580
   tccttcacat cttcctctca tatcccccta cttctactac ctccctggca actgatcaca 2640
   tgtccattac tatcccatta aaccctaatc accattacct tgctcaacac cagtatccta 2700
   tcccacaaca ggctttaagg ggattaaagc ctgttatcac tcacctgcta cagcatgggc 2760
   ttctaaaact tataaactct ccttacaatt cccccatttt acctattcaa aaaccagaca 2820
   agtcttacag gttagttcaa gatctgcgcc ttatcaacca aactgttttg cctatccacc 2880
   ctctggtgcc caacccgtac actcttttgc cctcaatacc ttcctccaca actcactatt 2940
   ccattcttga tcttaacgat ggttttttca ctattccact gcacccctca tcccagcctc 3000
   tctttgcctt taccctgaca cccatcagtc cctgcaaatt acctgggctg tactgctgca 3060
   aggcttcagg gacagccctc attacttcag ccaagctctt tctcatgatt tactttcttt 3120
   ccactcctcc acttctcacc ttattcaata tgttgatgac ctactttgta gccctgcctt 3180
   tgaatcttct caaaaagaca ccctcctgct ccttcaacat ttattctcca tgggatatca 3240
   agtatctcct tccaaagctc aaatttcttc tccatccatt acctaacttg gcatagttct 3300
   tcatgaaaac ccacgtgctc tccctgctga tcatgaccaa ctgatctctc agaccccaac 3360
   cccttctaca aaacaacagc tcctttcctt cctgggcatg attggatact ttcacctttg 3420
   gatacctgct tttgccatcc taacaaaacc atcacataaa ctcacaaaag gaaatttagc 3480
   tgaccccata gatcctaaat cctttgccca ctcctctttc cgttccttga agacagcttt 3540
   agagactgct cccacactag ctctccctga ctcatcccaa cccttttcat tacacacagc 3600
   caaagtgcag ggctgtgcag ttggaattct tacacaagga ccaggaccac accttgtagc 3660
   ctttttgtcc aaacaacttg accttactgt tttaggctgg ccatcatgcc tccgtgcggc 3720
   agctgctact gccataacac ttttagaggc cctcaaaatc acaaactatt ctcaacacac 3780
   tctctacaat tctcataact tccaaaatct attttcttcc tcacacctgg tgcgtatgct 3840
   ttctgctacc cggctccttc agctatactc accctttgtt gagtctccca caattactat 3900
   tgttcctggc ctggacttca atccagcctc ccacattatt ctgaatacca cacttgaccc 3960
   ccatgactgt atctctctga tccacttgac attcattcca tttccccata tttccttctt 4020
   tcctgttcct caccctgatc acacttggtt tatcgatggc agttccacca ggccaaatca 4080
   ccactcacca gcaaaggcag gctatgctat agtatcttcc acatctatca ttgaggctac 4140
   cgctctgccc ccctccactc cctctccgca agccgaactc attgccttaa ctagagccct 4200
   cactcttgca aagggactac acatcagtat ttatactgac tctgaatatt ccgtccatat 4260
   cctgcactac catgctgtta tatgggcaga aagaggttgc ctcactatgc aaaggtcctc 4320
   catcattaat gcctctttaa tgaaaactct tctcaaggct gctttacttc caaaggaagc 4380
   tggagtcata tactgcaagg gccatcaaaa ggcatcagat cccatcgctc aggacagtgc 4440
   ttaggctgat aaggtagcta aagaagcagc tagtgttcca acttctgtcc ttcagtgcca 4500
   gtttttctct ttctcactgg tcactcccac ctattccccc actgaaacgt tcacctatca 4560
   atctcttccc acacaaggca aatggttctt ggaccaagga aaatatctcc ttccagcctc 4620
   acaggcccat tctattctgt cgtcatttca aaacctcttc catttaggtt acaagccact 4680
   agcccgcctc ttagaacctc tcatttgctt tccatcatgg aaatctatcc tcaaggaaat 4740
   cacttctcag tgttccatct gctcttctac tactcctcag ggattactca ggccccctcc 4800
   cttccctaca catcaggctc agggatttgc ccctgcccag gactggcaaa ttgactttac 4860
   tcacatgcca tgagtcagga aactgaaata cctcttggtc tgggtagaca ctttcactgg 4920
   atgggtaaag gcctttccct cagggtctga gaagtccact gcggtcattt cttcccttct 4980
   gtcagacata attcttcagt ttgggcttcc cacctctata cagtccaata acggaccggc 5040
   ctttattagt caaatcaccc aagcagtttc tcagtctctt ggtattcagt agaaccttca 5100
   taccccttac caacctcaat ctttaggaaa gatagaacgg actaatgatc ttttaaaagc 5160
   acacctcacc aagctcagcc tccaacttaa aaaggaggac tctgtcaagg atagagccca 5220
   aaaactcacc aaccaagcaa gtaattacac tgaacccctt ggacactctc taattggatg 5280
   tcctgggtcc tcccaattct tagtccttta atacctgttt tcctctttct ccttgtgtct 5340
   tctgtctagt ttctcaattc atacaaaact gtatccaggc catcaccaac cattctatac 5400
   aaccaatgtc acttctaaca accccacaat atcacccctt accacaaaat cttccttcag 5460
   ctaaatctct cccactctag gctcccacat tgcccctaat cccactcgaa gctgccctga 5520
   gaaacattga ccattatctc tccataccac ccccccaaaa tttttgctgc tctaacactt 5580
   caacattagt ttatgttatt tttcttatta atataagaag acaggaatgt caggcctctg 5640
   agcccaagct aagccatcat atcccctgtg acctgcacat atagaatcag atggcctgaa 5700
   gcaagtgaag aatcaccaaa gaagtgaaaa tggcctgttc ctgccttaac tgatgacttt 5760
   accttgtgaa attacttccc ctggctcaga agctccccca ttgagcacct tgtgacccct 5820
   gcccctgtcc gccagagaac aacccttttg actgtaattt tccactacct acccaaatcc 5880
   tataaaatgg ccccacacct atcttccttc actgactctc tttttgaact cagcctgcct 5940
   gcacctaggt gattaaaaag ctttattgct cacacaaagc ctgtttggta gtcacttcac 6000
   acaga 6005
<210> 12
   <211> 7330
   <212> DNA
   <213> Homo sapiens
<400> 12
   gtagggaaaa gaaagagaga tcagactgtt actgttgtct atgtagaaaa ggaagacata     60
   agaaactcca ttttgacctg taccctgaac gattgttttg ccccgagatg ctgttaatct    120
   gtaactttgc cccaaccttg agctcacaga aacatgtgtt gtatggaatc aaggtttaag    180
   ggatctaggg ctgtgcagta tgtgccttgt taacaaaatg tttacaggca gtatgcttcg    240
   taaaagtcat caccattctc cattctcgat aagccagggg cacaatgcac tgcggaaagc    300
   cgcagggacc tctgccctgg aaagccgggt attgtccaag gtttctcccc atgtgatagc    360
   ctgagatatg gcctcgtggg gcgggaaaga cctgaccgtc ccccagccca acacccgtga    420
   agggtctgtg ctgaggagga aggcctcttg cagttgagat aagaggaagg cctctgtctc    480
   ctgcctgccc ctgggaacta aatgtctcag tataaaactc gattgtacat ttgttctctt    540
   ctgagataag agaaaacccg ccgtgtggcg ggaggcgaga catgttggtg gcagcaatgc    600
   tgctctgtta ctctttactc cactgagatg tttgggtgga gaaaagcata aatctggcct    660
   atgtgcacat ccaggcatag taccttccct tgaacttatt tgtgacacag attcctttgc    720
   tcacatgttt tcttgttgac cttctccaca ctatcaccct gttctcctgc cacattcccc    780
   ttactgagat agtaaaaata gtaatcaata aatactgagg gaactcagag accggtgcca    840
   gtgcgggtcc tccgtatgct gagcaccagt ctcctgggcc cactgttctt tctctatact    900
   ttgtctctgt gtcttatttc ttttctcagt ctctcgtccc acctgacgag aaatacccac    960
   aggtgtggat ggggctggcc ctcttcattt ggcgcccaac gtggggcctt tctctagggt   1020
   gaaggtgcgc taagaccgtg agcattgagg acagtcgatg agagattccc gagtacgtcc   1080
   acggtgagcc ttgcggtaag cttgtgcaca cggaggaacc cagggtaaca atgggacaaa   1140
   ctgaaagtaa atatgcctct tatctcagct ttattaaaat tcttttaaga agaaggggag 1200
   ttagagcttc tacagaaaat ctaattatgc tatttcaaac aatagaacaa ttctgcccat 1260
   agtttccaga acagggaact ttagatctaa aagactggga aaaaattggc aaagaattaa 1320
   aacaagcaag tagggaaggc aaaatcatcc cgcttacagt atgcaatgat tgggccatta 1380
   ttaaagcagc tttagaaccg tttcaaacag aagaagatag cgttttggtt tctgatgccc 1440
   ctgaaagctg tgtaatagat tgtgaagaag aggcggggac agagttcaag aaaggaacgg 1500
   aaagttcaca ttgtgaaaat gtagcagagt ctgtaatggc tcggtcaaca caaagtgttg 1560
   actacaatca attacaggag gtaatatatc ctgaatcacc aaaactgggg gaaggaggtc 1620
   cagaaccatc ggggccgtca gggctaaaac cacgatggcc acctcctcct cagtcgagtg 1680
   agtgctgggg gagggagcct gaaaccaggc tggctgcaac tcggctcgcg gtgcccatta 1740
   ttgcccaacc ggcagttcac tgcggtgaag gagcaattca gactcgccct gtagcatcct 1800
   gtctgggtca aacagtggcc gctccctaag gaaaagttag gggcgctaca taaaatagtt 1860
   aaaaaaacta tttaaaaaag gacatgtttc acccactgtc tctccttaga attcgccagt 1920
   gtttgtaatt cagaaaaaat ccggcagatg gcgcatgcta accgacttaa gagccgctaa 1980
   tgccgtaatt caacccatgg gggctctcca acgcaggctg ccctctccgg ccgtgatccc 2040
   caaaggttgg cctttaatta taattgatct gaaggattgc tttttttttt tttttttttt 2100
   ttaccattcc tctggcaaaa caggattttg aaaaatttgc ttttgctata ccagccataa 2160
   ataataaaga accagccacc aggtttcagt ggaaagtgtt gcctcaggga atgcttaata 2220
   gtccaactat ttgtcagact tttgtagctc aagctcttca accagttaga gacatgtttt 2280
   cagactgtta tatcattcat tatgttgatg atattttgtg tgctgcagaa atgagagaca 2340
   aattaattga ctgttacaca tttctgcaga cagaggttgc caacgcagga ctgacaatag 2400
   catctgataa aattcaaaca acagctcctt ttcattattt agaaatgcag gtagaggaaa 2460
   ggaaggttaa tcctcaaaag atagatagaa atgagaaaag acacattaaa atatgaaatg 2520
   actttcaaaa attgctggga gatattaatt ggattcggtc aaccctaggc atccctactt 2580
   atgccatgtc aaatttgttc tctatcttaa gaggggatcc agaatcaaat agtaaaagaa 2640
   cattaactcc agaggcaact aaagaaattg aattagttga agacaaaatt cggtcagcac 2700
   aagtaaatag aatagatcac ttagccccac tccaactttt gatttttgct actgcacatt 2760
   ctccaacagg catcattgtt caaaatacag atcttgtgga gtggtccttc cttcctcaca 2820
   gtacgattaa gacttttaca ttgtacttgg atcaaatggc tacattaatt ggtcaggcaa 2880
   gactacgaat agtaaaattg tgtggaagtg acccagataa aatcattgtt cctttaaaca 2940
   aggaacaggt tacacaagcc tttatcaatt ctggtgcatt gcagattggt cttgctgatt 3000
   ttgtgggaat tattgacaat cattacccaa aaacaaaaac cttccagttt ttaaaattga 3060
   ctacttggat tttacctaaa attaccagac atacaccttt agaaaatgct ctgacagtgt 3120
   ttactgatgg ttccagcaat ggaaaggtgg cttacaccag gccaaaaaaa cgagtcactg 3180
   aaactcaata tcactcagct caaagagcag agttggttgc tgtcatttca gtgttacaag 3240
   attttaatca gcttattaac cttgtatcag attctgcata tgtagtacag gctacaaagg 3300
   atgttgagac agccctagtc aaatacagta tggatgatcg gttacaccag ctgtttaatt 3360
   tgttacaaca aactgtaaga aaaagaaatt tcccatttta tattactcat gttcaagcac 3420
   atactaattt accagggcct ttaactaagg caaatgaaca agctgacttg ctagtatcat 3480
   ctgcattcat agaagcacaa gaacttcatg ccttgactca tgtaaatgca acaggactaa 3540
   aaaataaatt tgatatcaca tggaaacagg caaaaaatat tgtacagcat tgcacctagt 3600
   gtcaagtctt acactggccc actcaggagg caggagttaa tcccagaggt ttatgtccta 3660
   atgcattatg gcaaatggat gtcacacatg taccttcatt tggaaaaatg tcatttgtcc 3720
   atgaagacag ttgatactta ttcacatttc atatgggcaa cctgccagac aggagaaagt 3780
   acttcccatg ttaaaagaca tttattatct tgttttgctg tcatgggagt tccagaaaaa 3840
   attaaaacag ataatgggcc aggatactgt agtaaagcat ttcaaaaatt cctaaatcag 3900
   tggaaaatta cacatacaac aggaatcccc tataattccc aaggacaggc cataattgaa 3960
   agaactaata aagctcaatt ggttaaacaa aaaaaggaaa aagatagtaa ggagtataac 4020
   actcctcaga tgcaactcaa tctagcactc tatactttaa aatttttaaa cgtttataga 4080
   aatcagacca ctacttctgc agaacaacat tttactggta aaaagaacag cccacatgaa 4140
   ggaaaactga tttggtggaa agacatcaaa aataagacat gggaaatagg gaaggtgata 4200
   acctggggga aaggctttgc ttgcgtttca ccaggaaaaa agtcagcttc ctgtttggat 4260
   acccactaga catttaaagt tctacaatga acccatcgga aatgcaaaga aaagcgcctc 4320
   cgcggagaca gaaaacccgc aatcgagcat catcgactcg ccaggtgaac aaaatggtgg 4380
   tatcagaaga acagatgaag ttgacatcca ccaaggaagt ggagccgccg acctgggccc 4440
   aactaaagaa gttgacacag ttagctgaaa aaagcctgaa gaaaacaagg gtaacacaaa 4500
   ctccagagaa tatgctgctt gcagttttga tgattgtatc aatggtggta agtgtcccca 4560
   tgtctgcagg agcagctgca gctaattata cttactggac ctatgtgcct ttcccgccct 4620
   taattcgggc agtcacatgg atagataatc ctattaaagt atgtgttaat aatagtgcat 4680
   gagtaccagg ccccacagat gattgttgcc ctgcccaacc tgaaaaagaa ggaatgatga 4740
   taaatatttc cattgggtat cattatcctc ctatttgcct agggaaggca ccaggatatt 4800
   taattcctac aacccaaaat tggttggtag aagtacctac tgtcattgcc agcaatagat 4860
   ttacttatca catggtaagt gaaatgtcac tcgggccaca gataaataat ttacaggatc 4920
   cttcttatca aagatcatta aaatttaggc ctaagaggaa gccttgcccc aaggaaattc 4980
   ccaaagaatc aaaaggccca gaagtctcag tttgggaaga atgtgtggct gatactgctg 5040
   tggtattaca aaacaatgaa tttggaacta ttatagactg ggcccctcaa ggccaattat 5100
   attatgattg tacaggccag actcactgat gttcacaggc cccatccatc tggcccacta 5160
   atccggccta tgatagtgat ttaactgaaa ggctggacca ggtttacaga aggttagaat 5220
   caccctatcc atggaaatgg ggtgaaaagg gaatttcatc acttcgaaca aagttagtta 5280
   gtcctgttgt tggtcctgaa cacccagaat tatggaagct tactgtggcc tcgcaccaca 5340
   ttagaatttg gtctggaaat gaagctatag gaacaagaga tcgtaagcca tattatacta 5400
   ttaacctaaa ttccaatctg acaattcctt tgcaaagttg tgtaaaaccc ccttatatgt 5460
   tagttgtagg aaaaatagtt attaaaccag attcccaaac tataacctgt gaaaattgta 5520
   gattgtttac ttgcattgat tcgacttctg attggcagca ccatattctg ctggtgaggg 5580
   caagagaggg cgtgtggatc cctgtgtcca tggaccgacc gtgggaggct tccccatccg 5640
   tccatatttt aatggaagta ttaaaaggag ttctaactag atccaaaaga ttcattttta 5700
   ctttaattgc agtcattatg ggtcttgttg cagtcacagc tactgctgtg gctgctggaa 5760
   ttgctttaca ctcctctgtt caaatggtaa aatatgtaaa taattggcaa aagaattcct 5820
   caaaattgtg gaattctcag acccaaatag atcaaaaatt ggcaaaccaa attaatgacc 5880
   ttagacaaac tgtcatttgg atgcgagata ggctcatgag cttgaaatat ctttttcagt 5940
   tacagtgtga ctggaatacg tcagattttt gtattacacc ccgagcctac aatgagtctg 6000
   agcatcactg ggccatggtt agatgccatc tacaaggaag agaagataat cttactttag 6060
   atatttcaaa attaaaagaa caaatttttg aggcatcaaa agcccattta aatctggtgc 6120
   cagaaactga ggcaatcgtg aaagctgctg atggcctcac aaatcttaat gccgtcactt 6180
   gggttaaaac tatcagaagt tccactattg taaatttcat attaatcctt gtatgtctgt 6240
   tctgtctgtt gttagtctac aggtgtatcc aacagctccg aagagacagt gaccagcgaa 6300
   aacgggccgt gatgatgatg gtggttttgt cagaaagaaa agggggatat gcaggcaaga 6360
   gaaagagaga tcagactgtt actgttgtct gtgtagaaaa ggaagacata agaaactcca 6420
   ttttgacctg taccctgaac gattgttttg ccccgagatg ctgttaatct gtaactttgc 6480
   cccaaccttg agctcacaga aacatgtgtt gtacagaatc aaggtttaag ggacctaggg 6540
   ctgtgcagga cgtgccttgt taacaaaatg cttacaggca gtatgcttgg taaaagtcat 6600
   cgccattctc cattctcgat aaaccagggg cacaatgcac tgcggaaagc cgcagggacc 6660
   tctgccctgg aaagcgggat attgtccaag gtttctcccc atgtgatagc ctgagatatg 6720
   gcctcgtggg atgagaaaga cctgaccgtc ccccagcctg acacccgtga agggtctgtg 6780
   ctgaggagga ttagtaaaag aggaaagcct cttgcagttg agataagagg aaggcctctg    6840
   tctcctgcct gcccctggga actaaatgtc tcggtataaa actctattgt acatttgttc    6900
   tcttctgaga taggagaaaa cccaccctgt ggcgggaggc gagacatgtt ggtggcagca    6960
   atgctgttct gttactcttt actccactga gatgtttggg tggagaaaag cataaatctg    7020
   gcctatgtgc acatccaggc atagtacctt cccttgaact tatttgtgac acagattcct    7080
   ttgctcacat gttttcttgc tgaccgtctc cgcactatca ccctgttctc ctactacatt    7140
   ccccttactg agatagtaaa ataataatca ataaatactg agggaactca cagaccggtg    7200
   ctggtgcagg tcctccggat gctgagtgcc gtctcctggg cccactgttc tttctccata    7260
   ctttgtgtct tatttctttt ctcagtctct cgtcccacct gacgagaaat acccacaggt    7320
   gtggaggggc                                                           7330
<210> 13
   <211> 961
   <212> DNA
   <213> Homo sapiens
<400> 13
   gtggggaaaa gaaagagaga tcagattgtt actgtgtcta tgtagaaaac agaagacata      60
   agaaactcca ttttgttctg ctttgagatg ctgttaatct gtaacttttg tcccaacctt     120
   gtgctcacaa aaacatgtgc tgtattgaat caaggtttaa tggatctagg gctgtgcagg     180
   atgtgccttg gtaaaaatgt gtttgcaggc agtatgcttt gtaaaagtca tcgccattct     240
   ccattctcta ttaactagag acacaatgca ctgcggaagg ccgcaggaac ccctgcccaa     300
   gaaagcctgg gtattgtcca ggtttccccc cactgagaca acctgagata cggcctcgtg     360
   ggaagggaaa gaccttacag ccccccagcc cgacatccgt aaagggtctg tgctgaggag     420
   gattagtgaa agaagaaggc ctcaatgcgg ttgagataag aggaaggcgt ctgtctcccg     480
   cacgtccctg ggaatggaat gacaaatgta aaaccaacca tacattctac tctaagagaa     540
   aatcgcctta tggatacagg tgagacatca tggcagcaat actactcttt actgcactga     600
   gatgtttatg taaagttaaa tataaatcta gcctacgtgt acatttaggc ccagcacttt     660
   tccttaaact tatttatgac acagattcct ttactcacat gtttttctgc tgaccctctc     720
   cccaccttca ccctatagcc ccaccacatt cccctcgcca agataggaaa aatagtgatc     780
   aataaatact gagagaactc agagactcag agtaagtgag caccggtgtt ggtcctcact     840
   tactaagcgc cggtccccca gggcccactt ttcttcctct gtactttgtc tctgtgtctt     900
   atttcttttc tcagtctctc atctccacct tgcaagaaat acccacagga gtggagaggc     960
   a                                                                     961
<210> 14
   <211> 437
   <212> DNA
   <213> Homo sapiens
<400> 14
   gtcaggcctc tgagcccaag ctaagccatc atataccctg tgacctgcac gtatacatcc      60
   agatggcctg aagccactga agaaccacaa aagtgaaaat agccagttcc taccttaact     120
   gatgacattc cacgattgcg atttgttcct gcccttccct aactgatcaa tggaccttgt     180
   gacactcctt ctcctggaca atgagtctca ggagctcccc actgagcacc ttgtgacccc     240
   cacccctgcc cgcaagagaa aaaccccctt taactgtaat tttccactac ctacccaaat     300
   cctataaaga ctgcctcacc cctatctccc tttgctgact cctttttcga actaagtcgg     360
   cctacaccca catgattaaa agctttattg ctcacccaaa gcctgtttgg tggtctcttc     420
   acactgacgc gcgttaa                                                    437
<210> 15
   <211> 966
   <212> DNA
   <213> Homo sapiens
<400> 15
   gtggggaaaa gagagagaga tcacattgtt actgtgtctg tgtagaaaga agtagacata      60
   ggagactcca ttttgttctg tactaagaaa aattcttctg ccttgagatg ctgttaatct     120
   aaccctagcc ccaaccctgt gctccctgag acatatgctg tgtcaactca gggttaaatg     180
   gattaagggc tgtgcaagat gtgctttgtt aaagaaatgc ttgaaggcag catgctcgtt     240
   aagagtcatc tccactccct aatctcaagt actcagggac acaaaacact gaggaaggcc     300
   acagggacct ctgcctagga aagccaggta ttgtccaagg tttctcccca tgtgatagtc     360
   tgaaatgtgg cctcgtggga agggaaagac ctgaccgtcc cccagcccga cacccgtaaa     420
   gggtctgtgc tgaggaggat tagtaaaaga ggaaggaacg cctctttgca gttgagacaa     480
   gaggaaggca tctgtctcct gctcgtccct gggcaatgga atgtctcagt gtaaaacccg     540
   attgtatatt ccatctactg agatagggga aaactgcctt agggctggag gtgggacatg     600
   ctggcagcaa tactgctctt caagtcattg agatgtttat gtgtatgcat atctaaagca     660
   cagcacttaa ttctttacct tgtttatgat gcagagacct ttgttcacgt gtttacctgc     720
   tgaccttctc tccactatta tcctttgacc ctgccacatc cccctctccg agaaacaccc     780
   aataatgatc aataaatact aagggaactc agaggccggt gggatcctcc gtatgctgaa     840
   caccggtccc ctggacccct ttttttcttt ctctatactt tgtctctgtg tctctttctt     900
   ttccaagtct ctcattccac ctaacgagaa acaaccacag gtgtggaggg gcagcccaac     960
   ccttca                                                                966
<210> 16
   <211> 5924
   <212> DNA
   <213> Homo sapiens
<400> 16
   tggggcaaac taaaagtaaa attaaaagta aatatgcctc ttatctcagc tttattaaaa      60
   ttcttttaaa aagaggggga gttaaagtat ctacaaaaaa atctaatcaa gctatttcaa     120
   ataatagaac aattttgccc atggtttcca gaacaaggaa ctttagatct aaaagactgg     180
   aaaagaattg gtaaggaact aaaacaagca ggtaggaagg gtaatatcat tccacttata     240
   gtatggaatg attgggccat tattaaagca gctttagaac catttcaaac agaagaagat     300
   agcgtttcag ttcctgacgc ccctagaagc tgtatagtag attgtaatga aaagacaagg     360
   aaaaaatccc agaaagaaat ggaaagttta cattgcgaat atgtagcaga gccggtaatg     420
   gctcagtcaa cgcaaaatgt tgactataat caattacagg aggtgatata tcctgaaacg     480
   ttaaaattag aaggaaaagg tccagaatta gtggggccat cagagtctaa gccacgaggg     540
   ccaagtcctc ttccagcagg tcaggtgccc gtaacactac aacctcaaac gcaggttaaa     600
   gaaaataaga cccaaccgcc agtagcttat caatactggc cgccagccga acttcagtat     660
   cggccacccc cagaaagtca gtatggatat ccaggaatgc ccccagcacc acagggcagg     720
   gcgccatacc ctcagccgcc cactaggaga cttaatccta cggcaccacc tagtagacag     780
   ggtagtgaat tacatgaaat tattgataaa tcaagaaagg aaggagatac tgaggcgtgg     840
   caattcccag taacgttaga accgatgcca cctggagaag gagcccaaga gggagagcct     900
   ctcacagttg aggccagata caagtctttt tcgataaaaa tgctaaaaga tatgaaagag     960
   ggagtaaaac agtatggacc caaatcccct tatatgagga cattattaga ttccattgct    1020
   catggacata gactcattcc ttatgattgg gagattctgg caaaatcgtc tctctcaccc    1080
   tctcaatttt tacaatttaa gacttggtgg attgatgggg tacaagaaca ggtccgaaga    1140
   aatagggctg ccaatcctcc agttaacata gatgcagatc aactattagg aataagtcaa    1200
   aattggagta ctattagtca acaagcatta atgcaaaatg aggccattga gcaagttaga    1260
   gctatctgcc ttagagcctg ggaaaaaatc caagacccag gaagcgcctg cccctcattt    1320
   aatacagtaa gacaaggttc gaaagagccc taccctgatt ttgtggcaag gctccaagat    1380
   gttgctcaaa agtcaattgc caatgaaaaa gcccgtaagg tcatagtgga gttgatggca    1440
   tatgaaaacg ccaatcctga gtgtcaatca gccattaagc cattaaaagg aaaggttccc    1500
   gcaggatcag atgtaatctc agaatatgta aaagcctgtg atggaatcag aggagctatg    1560
   cataaagcta tgcttatggc tcaagcaata acaggagttg ttttaggagg acaagttaga    1620
   acatttggag gaaaatgtta caattgtggt caaattggtc acttaaaaaa gaattgccca    1680
   gtctcaaata aacagaatat aactattcaa gcaactacaa caggtagaga gccacctgac    1740
   ttatgtccaa gatgtaaaaa aggaaaacat tgggctagtc aatgtcgttc taaatttgat 1800
   aaaaatgggc aaccattgtc gggaaacgac caaaggggcc agcctcaggc cccacaacaa 1860
   actggggcat tcccaattca gccatttgtt cctcagggtt ttcagggaca acaaacccac 1920
   tgtcccaagt gtttcaggga ataagccagt taccacgata caacaattgt cccccgccac 1980
   gagcggcagt gcagcagtag atttatgtac tatacaagca gtctctctgc ttccagggga 2040
   gcccccacaa aaaatcccca cagaggtata tggcccactg cctgagagga ctgtaggact 2100
   aatcttggga agatcaagtc taaatctaaa aggagttcaa attcatactg gtgtggttga 2160
   ttcagactat aaaggcgaaa ttcagttggt tattagctct tcaattcctt ggagtgccag 2220
   tccaggagac aggattgctg aattattact cctgccatat attaagggtg gaaatagtga 2280
   aataaaaaga acaggagggt ttggaagcac tgatccgaca ggaaaggctg catattgggc 2340
   aagtcaggtc tcagagaaca gacctgtgtg taaggccatt attcaaggaa aacagtttga 2400
   agggttggta gacactggag cagatgtctc tatcattgct ttaaatcggt ggccaaaaaa 2460
   ttggcctaaa caaaaggctg ttacaggatt tgtcggcata ggcacagcct cagaagtgta 2520
   tcaaagtact gagattttac attgcttagg gccagataat caagaaagta ctgttcagcc 2580
   aatgattact tcaattcctc ttaatctgtg gggtcgagat ttattacaac aatggggtgc 2640
   ggaaatcatg cccgctccat tatatagccc cacgagtcaa aaaatcatga ccaagatggg 2700
   atatatacca ggaaagggac taggaaaaaa tgaagatggc attaaagttc cagttgaggc 2760
   taaaataaat caagaaagag aaggaatagg gtatccttgt taggggcagc cactgtagag 2820
   cctcctaaac ccataccatt aacttggaaa acagaaaaac cggtgtgggt aaatcagtgg 2880
   ccgctaccaa aacaaaaact ggaggcttta catttattag caaatgaaca gttagaaaag 2940
   ggtcacattg agccttcgtt ctcgccttgg aattctcctg tgtttgtaat tcagaagaaa 3000
   tcaggcaaat ggcgtatgtt aactgactta agggccgtaa acgccgtaat tcaacccatg 3060
   gggcctctcc aacccgggtt gccctctctg gccatgatcc caaaagactg gcctttaatt 3120
   ataattgatc taaaggattg cttttttacc atccctctgg cggagcagga ttgcgaaaaa 3180
   tttgccttta ctataccagc cataaataat aaagaaccag ccaccaggtt tcagtggaaa 3240
   gtgttacctc agggaatgct taatagtcca actatttgtc agacttttgt aggtcgagct 3300
   cttcaaccag ttagagacaa gttttcagac tgttatatta ttcattatat tgatgatatt 3360
   ttatgtgctg cagaaacaaa agataaatta attgactgtt atacatttct gcaagcagag 3420
   gttgccaatg caggactggc aatagcatct gataagatcc aaacctctac tccttttcat 3480
   tatttaggga tgcagataga aaatagaaaa attaagccac aaaaaataaa aataagaaaa 3540
   gacacattaa aaacactaaa ttattttcaa aaattgctgg gagatattaa ttggattcgg 3600
   ccaactctag gcattcctac ttatgccatg tcaaatttgt tctctatctt aagaggagac    3660
   tcagacgtaa atagtaaaag aatgttaacc ccagaggcaa caaaagaaat taaattagtg    3720
   gaagaaaaaa ttcagtcagc gcaaataaat agaatagatc ccttagcccc actccaactt    3780
   ttgatttttg ccactgcaca ttctccaaca ggcatcatta ttcaaaatac tgatcttgtg    3840
   gagtggtcat tccttcctca cagtacagtt aagactttta cattgtactt ggatcaaata    3900
   gctacattaa ttggtcagac aagattacga ataataaaat tatgtggaaa tgacccagac    3960
   aaaatagttg tccctttaac caaggaacaa gttagacaag cctttatcaa ttctggtgca    4020
   tggcagattg gtcttgctaa ttttgtggga attattgata atcattaccc aaaaacaaag    4080
   atcttccagt tcttaaaatt gactacttgg attctaccta aaattaccag acgtgaacct    4140
   ttagaaaatg ctctaacagt atttactgat ggttccagca atggaaaagc agcttacaca    4200
   gggccgaagg aacgagtaat caaaactcca catcaatcgg ctcaaagagc agagttggtt    4260
   gcagtcatta cagtgttaca agattttgac caacctatca atattatatc agattctgca    4320
   tatgtagtac aggctacaag ggatgttgag acagctctaa ttaaatatag catggatgat    4380
   cagttaaacc agctattcaa tttattacaa taaactgtaa gaaaaagaaa tttcccattt    4440
   tatattactc atattcgagc acacagtaat ttaccagggc ctttgactaa agcaaatgaa    4500
   caagctgact tactggtatc atctgcattc ataaaagcac aagaacttca tgctttgact    4560
   catgtaaatg cagcaggatt aaaaaacaaa tttgatgtca catggaaaca ggcaaaagat    4620
   atcgtacaac attgcaccca gtgtcaagtc ttacacctgc ccactcaaga ggcaggagtt    4680
   aatcccagag gtctgtgtcc taatgcatta tggcaaatgg atgtcacgca tgtaccttca    4740
   tttggaaaat tatcatatgt tcatgtaata gttgatactt attcacattt catatgggca    4800
   acttgccaaa caggagaaag tacttcccat gttaaaaaaa catttattgt cttgttttgc    4860
   tgtaatggga gttccggaaa aaatcaaaac tgacaatgga ccaggatatt gtagtaaagc    4920
   tttccaaaaa ttcttaagtc agtggaaaat ttcacataca acaggaatgc cttataattc    4980
   ccaaggacag gccatagttg aaagaactaa tagaacactc aaaactcaat tagttaaaca    5040
   aaaagaagca ggagacagta aggagtgtac cactcctcag atgcaactta atctagcact    5100
   ctatacttta aatttttaaa acatttatag aaatcagact actacttctg cagaacaaca    5160
   tcttactggt aaaaagaaca gcccacatga aggaaaacta atttggtgga aagataataa    5220
   aaataagaca tgggaaatag ggaaggtgat aacgtggggg agaggttttg cttgtgtttc    5280
   accaggagaa aatcagcttc ctgtttggat acgcactaga catttgaagt tctacaatga    5340
   acccatcgga gatgcaaaga aaagcacctc cgcggagacg gagacaccac agtcgagcac    5400
   cgttgactca caagatgaac aaaatggtga cgtcagaaga acagatgaag ttgccatcca    5460
   ccaagaaggc agagccgcca acttgggcac aactaaagaa gctgacgcag ttagctacaa    5520
   aatatctaga gaacacaaag gtgacacaaa ccccagagag tatgctgctt gcagccttga 5580
   tgattgtatc aacggtggta agtctcccta tgcctgcagg agcagctgca gctaactata 5640
   cctactgggc ctatgtgcct ttcccgccct taattcgggc agtcacatgg atggataatc 5700
   ctatagaagt atatgttaat gatagcgtat gggtacctgg ccccacagat gatcgctgcc 5760
   ctgccaaacc tgaggaagaa gggatgatga taaatatttc cattgggtat cgttatcctc 5820
   ctatttgcct agggagagca ccaggatgtt taatgcctgc agtccaaaat tggttggtag 5880
   aagtacctac tgtcagtccc atcagtagat tcacttatca catg 5924
<210> 17
   <211> 351
   <212> DNA
   <213> Homo sapiens
<400> 17
   caggcctctg agcccaagcc tgcaggtata catccagatg gcctgaacca actgaagaat 60
   cacaaaagaa gtgaaaatgg ccaattcctg ccttaactga tgacattacc ttgtgaaatt 120
   ccttctcctg gctcagaagc tcccccactg aacactttgt gactcctgcc cctgccctcc 180
   agagaacaac cccctttgac tataattttt cgctacctac ccaaatccta taaaactgcc 240
   ccacccctaa ctccctttgc tgactcattt tttggactca gcccccctgc acccaggtga 300
   aataaacagc tttattgctc acacaaagcc tctttggtgg tctcttcacg c 351
<210> 18
   <211> 56
   <212> DNA
   <213> Homo sapiens
<400> 18
   cctccactac gccccctcag caggaagaag ccagagtgat cgacggcctt ttccca 56
<210> 19
   <211> 314
   <212> DNA
   <213> Homo sapiens
<400> 19
   taataacatc aacccctgac ctaaactact tgtgctatcc gtaaattcca gacattgtat 60
   gaaaaagcat tgcaaagctt tctgttctgt tagctgatac atgtagcccc cagtcacgtt 120
   ccccgcttgc tcgatttatc acgacctttt cacgtggacc ccttagagtt gtaagccttt 180
   aaaaaggcca agaatttctt tttcagggag ctcggctctt aagacgcaag tctgccgaca 240
   ctcctggctg aataaacctc ttccttcttt aatccagtgt ctgaggagtt ttgtctgtgg 300
   ctcgtcctgc taca 314
<210> 20
   <211> 7190
   <212> DNA
   <213> Homo sapiens
<400> 20
   gccaggcctc tgagcccaag ctaagccatc atatcccctg tgacctgcac gtatacatcc 60
   agatggcctg aagtaactga agaatcacaa aagaactgaa aatgccctgt tcctgcctta 120
   actgatgaca ttaccttgtg aaattccttc tcctggctca tcctggctca aaagctcccc 180
   cactgagcac cttatgaccc ccgcccctgc ccgccagcga acaacaccct ttgactgtaa 240
   ttatccacta cccacccaaa tcttataaag ctgccccacc cttatctccc ttcgctgact 300
   cttttcggac tcagcccgcc tgcacccagg ttaaataaac agcctcattg ctcacacaaa 360
   gcctgtttgg tggtctcttc acacggatgc gtgtgacatt tggtgccatg actcggatca 420
   gggatccttg ggagatcaat cccctgtcct cctgctcttt gcacctacga cctctggtcc 480
   tcagaccaac cagcccaagg aacatctcac caattttaaa ttggataagc ggcctctttt 540
   tacgcttttc tccaacctct ctcactatcc ctcaacctct ttctcctttg aatcttggtg 600
   ccatctttca gtctctccct tctcttaatt tcagtgcctt tccttttctg gtagagacag 660
   gagacgcgtt ttatccgtga acccaaaact ccatcgctag ttacggactc gggaaaacag 720
   tcttcccttg gtgtttaatc acgcagggat gcctgcttga ttactcaccc acgtttcaga 780
   ggtgtctgat cacacaggga cgcctgcctt ggtccttcac ccttagcagc aagcactgct 840
   tttcttgggg gcaagcaccc ctcacccctt ctctccgtgt ctctacccct tttccactgt 900
   cctggggggc aagcaccccc catcccttct ctctgtgtgt ctaccccttt tccactgtcc 960
   tggggggcaa gcatcccccc accttctctc cgtgtctcta ccctctcttt tctctggact 1020
   tgcctccttc actataggca aacttccacc ctccattcct ccttcttctc ctttagcctg 1080
   tgttctcaag aactcaaaac ctcttcaact cacacctgac ctaaaaccta aatgccttat 1140
   tttcttctgc aatgctgctt gaccccaatg caaacttgac aatggttcca aatagccaga 1200
   aaacggcact ttcgatttct ccatcctaca aggtctaggt aattcttgtc atgaaatggg 1260
   caaatggtct gaggtgcctg acgtccaggc attcttttac acatcagtcc ctccctagtc 1320
   tctgttccca atgcaattag tcccaaatct tgcttctttc cctcccacct gtcccctcag 1380
   tcccaacccc aagcattgct gagtctttcc aatcttcctt ttctacagac ccatctgacc 1440
   tctcctttcc tccccaggtt gctcctcacc aggcctagcc aggtcccaat tcttcctcag 1500
   cctccgctcc cctaccctat aatcctttta tcacctcctc tcctcacacc cgacgcggct 1560
   tacagtttcg ttctgcgact agccctcccc gacctgccca gcaatttctt cttaaaaggt 1620
   ggctggagcc aaaggcatgg tcaaggtttt tctttatccg acctctccca aatcagttag 1680
   cgtttaggct ctttttcatc aaatagaaaa acccagccca gttcttggct cgtttggcag 1740
   caacctgaga cgctttacag ccctagaccc tgaaaggtca gaaagaaggc catcttattc 1800
   ttaatacgca ttttattacc cagtccgctc ctgacattaa ataaagctcc aaaaattaga 1860
   ttccagccct caaaccccac aacaagactt aattaacctc gccttcaagg tgtacaataa 1920
   tagagcagag gcagccagac agcaacgcat ttctgagtta caattacttg cctctgccat 1980
   gagacaaaac ccagccgcac ctccagcata caagaacttc aaaatgccta agccgcgcac 2040
   acctaagcca cagcggccag gcgttcctac aggacttcct ccaccaggat cttgcttcaa 2100
   gtgccagaaa tctggccact gggccaagga atgcccgcag cccaggattc ctcctaagcc 2160
   gtgtcccatg tgtatgggac cccactggaa atcagactgt ccaagtcacc cagcagccaa 2220
   ccccagagtc cctggaactc cggcccaagg ctctctgact gactccttcc cagatcttct 2280
   tgacttagcg gctgacgacc gacgctgccc aattgcctca gaagcttcct ggaccatcac 2340
   agatgctttg ggtaactctt atagagtgaa gggtaagtcc atccccttct taatcaatac 2400
   tgaggctacc cactccacat taccttcttt tcaagggcct gtttgttatg cctccataac 2460
   tgttgtgggt gttgacggcc aggcttctag acctcttaaa actccccaac tctgatgcta 2520
   gcttggacaa tattctttta tatactcctt ctagttatcc ccacctgccc agctcccttg 2580
   ttaggtcaag acattttaac taaattatct gcttccctga ctgttcctgg gctacagcca 2640
   catctcattg ctgccttttt ccccagttca aagcctcctt tgcattctcc ccttgtatct 2700
   ccccacctta atccacaagg gtaggacacc tctactccct ccttggcgat tgatcatgca 2760
   ccccttacca tcccattaaa acctaatcac ccataccccg ctaaacgcca atatcccatc 2820
   acacagcacg ctttagaagc ctgttattac tcgcctgtta cagcatggcc ttttaaaacc 2880
   tataaactct ccttacaatt cccccatttt acctgtccta aaaccagaca agtattacag 2940
   gttagttcag gatctgcgcc ttatcaacca aattgttttg cctatccacc ccgtggtgcc 3000
   aaacccatat actctcctat cctcaatacc tccctccaca acccattatt ctattctaga 3060
   taaacctagc tgaccccata aatcctaaat cctttcccca ctcccctttc cattccttaa 3120
   aaaacagctc taaaagctgc tcccacacta gctctcccta actcatccca acctttttca 3180
   ttatacacag ccaaagtgca ggactctgtg gtcggaattc ttacacaaga gccaggaccg 3240
   cgccctgtag tctttctgtc caaacaattt gaccttactc ttttagccta gccttcatgt 3300
   ctgtgtgtgg cagctgccgc tgctttaata ttttcagagg ccctcaaaat cacaaactat 3360
   gctcaactca ctctctacag ttctcataac ttccaaaatc tattttcttc ctcacacctg 3420
   acacatatac tttctgccct ctggctcctt cagctatact cgctctttgt tgagtctccc 3480
   acaattacca ttgttactgg cccggacttc aatccggcct cccacattat tcctgatacc 3540
   acacctgaca ttcactccaa ttccccatat ttccttcttt cctgttcctc accctgaaca 3600
   cacttggttt attgatagca gttccaccag gcctaactgc cactcaccag caaaggcagg 3660
   ctatgctata gtatcttcca cacctatcag tgaggctacc actctgcccc cgtccactac 3720
   ctctcaacta gccaaactca ttgccttcac tcgagccctt gctcttgcaa aaggactacg 3780
   catcgatatt tatattgact ttaaatatgc cttccatatc ctgcaccacc atgctgttat 3840
   atgggcagaa agaaatttcc tcactatgca agggtcctcc atcattaatg cctctttaat 3900
   aaaaactctt ctcaaagccg ctttacttcc aaaggaggct agagtcaccg ggcgcggtgg 3960
   ctcacgcctg taattccagc actttgggag gccgaggtgg gtggatcaca aggtcaggag 4020
   atcgagacca tcctggctaa cacggtgaaa ccccgtctct actaaaaata caaaaaatta 4080
   gccaggcatg gtggcgggtg cctgtagtcc cagctactcc agaggctgag gcaggagaat 4140
   ggcgtgaacc tgggaggcgg agcttgcggt gagccgagat ggcaccactg cactccagcc 4200
   tgggcgagag tgtgagactc tgtctcaaaa aaaaaaaaaa agaaagaaag aaaggaagga 4260
   agctagagtc attcactgca agcagccatc aaaaggcatc agacctctca ttgctcaggg 4320
   caatgcatat gctgataagg tagctaaaaa agcacctagc attccaactt ctatccctca 4380
   tggcagtttt tctccttctc atctggccac tcccacctac tcccccactc aaacttgcac 4440
   ctatcaatct cttcccacac aaggcaaatg gttcttggac caaggaaaat atctccttcc 4500
   agcctcacag gcccattcta ttctgtcatc atttcatagc ctcttccatg taggttacaa 4560
   gccgctggtc tgcctcttag aacctctcat ttcctttcca tcgtaaaaat ctatcctcaa 4620
   aaaaatctca gtgttccatc tgctatctac tactcctcag ggattattca ggccccctcc 4680
   cttccctaca catcaagctc ggggatttgc cccctgccca ggactggcaa attgacttta 4740
   ctcacatgcc ccgggtcaga aaactaaaat acctcttggt ctgggtaaac actttcactg 4800
   ggtgggtaga agcctttccc acagggtctg agaaggccac cgcggtcatt tcttctcttc 4860
   tgtcagacat aattcctcgg tttggccttc ccacctctat acagtctgat aatggaccgg 4920
   cctttattag tcaaatcacc caagcagttt ctcaggctct tgttatttag tggctcctgg 4980
   ttttacctca aatccccacc cttaagtctc tctttaagtg gatagaagat cttcagtgac 5040
   aaagtacact ccaatacttt caccctgatg aagtcctatt ctttactttt atactcactc 5100
   ttactcttgt tcccgttctt atgccacgct ctacctctcc ccagctatct ccaccacact 5160
   atcaatctca gtcactctct cctagccgtt tctaatcctt ctttaacaat tgctagcttt 5220
   gcatttctct ttcctccaaa atcgccgagg cctcgactta ctcactgcta aaaataaaat 5280
   aaaaaaaata aaaaaagggg ggggtactct gtatattttt aaatgaacag tgctgttttt 5340
   acctaaatca atctggcctg gtatatgaca acataaaaaa aaaaaaaaag ctcaaagata 5400
   gagcctaaaa acttgccaac caagcaagta attaggctga acccccttag gcactctcta 5460
   attggatgtc ctgggccctc ccaattctta gtcctttaat ctctgttttt ctccttctct 5520
   tatttggacc ttgtgtcttc tgtttagtct ctcaactcat acaaaactgc atccaggcca 5580
   tcaccaaaca ttctatggga caaatactcc ttttaacaac cccacaatat cgccccttac 5640
   cacaaaatct tccttcagct taatctctcc cactctaggt tcccatgtcg cccctaatcc 5700
   cgctcgaagc agccctgaga aacatcgccc attatctctc catatcaccc cccaaaattt 5760
   tcgccgcccc aacactttac cactatttca ttttattttt cttactaata taagaagaca 5820
   agaatgtcag gcctctgagc ccaagctaag ccatcatatc ccctgtgacc tgcacatata 5880
   tatacatcca ggtggcctga agtgaagaac cacaaaagaa gtgaaaatgg cctattcctt 5940
   gtggggaaaa gaaagacaga tcagattgtt actgtgtctg tgtagaaaga agtagacata 6000
   ggagactcca ttttgttctg tactaaaaaa aattcttctg ccttggtatg ctgttaatct 6060
   atgaccttac ccccaacgcc gtgctctctg aaacatgtgc tgtgtccact cagggttaaa 6120
   tggattaagg gcggtgcaag atgtgctttg ttaaacagat gcttgaaggc agcatgctcg 6180
   ttaagagtca tcaccactcc ctaatctcaa gtacccaggg acacaaacac tgcggaaggc 6240
   cgcaggttcc tctgcctagg aaagccaggt attgtccaag gtttctcccc atgggatagt 6300
   ctgaaatatg gcctcatggg aagggaaaga cctgaccgtc ccccagcccg acacccagta 6360
   aagggtctgt gctgaggagc attagtataa gaggaaggaa tgcctctttg cagttgagac 6420
   aagaggaagg catctgtctc ctgctcgtca ctgggcaatg gaatgtctcg gtataaaacc 6480
   tgattgtatg ttccatctac tgagataggg gaaaaccgcc ttagggctgg aggtgggaca 6540
   tgcgggcagc aacactgctc tttaaggtat tgagatgttt atgtgtgtgc atatctaaag 6600
   cacagcactt aatcctttac cttgtctatg aggtagagac ctttgttcac gtgtttatct 6660
   gctgaccttc tctccactat tatcctatga ccctgccaca tccccctctc cgagaaacac 6720
   ccaagaatga tcaataaata ctaagggaac tcagaggctg gcgggatcct ctatatgctg 6780
   aacgctggtc ccctgggccc ccttatttct ttctctatac tttgtctctg tgtctttttc 6840
   ttttccaagt ctctcgttcc acctaacaag aaagacccac aggtgtggag gggcaaccca 6900
   ccccttcaat tcctgcctta actgatgaca ttactttggg aaattccttt tcctggctca 6960
   taagctcccc aactgagcac cttgtgaccc ttgcccctgc ctgccagaga acaaccctct 7020
   ttgactgtaa ttttccacta cccacccaaa tcctataaaa tggccccacc cttatctccc 7080
   ttcgctgact cttttttcag actcagcccg cctgcaccca ggtgaaataa acagccttgt 7140
   tgctcacaca aagcctgttt ggtggtctct tcacacggac gtgagtgaaa 7190
<210> 21
   <211> 424
   <212> DNA
   <213> Homo sapiens
<400> 21
   aggagatccg tcagggtagt gggagaaatt gtaggaaaag atacaaacct tcctggaagg 60
   ctgggaggtt ttgcaaaagc ttcgaaaggc tgccttcagc caaactctct tatccggggc 120
   ctgagagcaa aggttagata acaaggggat gtaaagaaat tcatctagat aaattagttt 180
   acataggcct tggaacctgg cctttaatca ttagcatgca cggctgctct ctcagggcca 240
   gggggcaacc gtgttaatta tccacaagtt gtgttgactc aataaatgac ttgtcagggc 300
   cacagctgct acaactcttt ctgtgagtgg cccggtcccc cagcccgctg tttcactgga 360
   tacctgtgtc tgagtacatt ttttcatccg ttgctcctcc agggtctgct ggtcagacct 420
   ggca 424
<210> 22
   <211> 867
   <212> DNA
   <213> Homo sapiens
<400> 22
   tggtaacctg taaccttagc cccatccctg tgcccacaga aacatgtgct gtattgactc 60
   aaggtttagg ggatttaggg ctgtgcagga tgtgctttgt taacaatgtg tttgcaggca 120
   gtatgcttgg taaaagtcat cgccatcctc cattctccat taaccaggga cacagtgcac 180
   tgcggaaagc cgcagggacc tctgcccaag aaagcctggg tattgtccag gtttcccccc 240
   agtgagacgg cctgagatat ggcctcgtgg gaagggaaag acctgatcgt cccccagccc 300
   gacacccata aagggtctgt gctgaggagg attagtgaaa gagggaggcc tctttgcagt 360
   tgagataaga ggaaggcttc tgtctccgac atgcccctgg gaacggaatg tctccgtgta 420
   aaacccgatc gtacattagt tctattctga gacaggagaa aaccgccctg tggctggagg 480
   cgagatatgc tggcggcaat gctgctctgt tactctgcta cactgagatg tttgggtgga 540
   gagaagcatg aatctggcct acgtgcacat ccgggcacag caccttccct tcaagctatt 600
   tgtgacacag atgcctttgc tcacattttc ttgctgacct ctgctctgct gccgcattcc 660
   tcatgcaaag atagtgaaaa tggtaatgaa taaatactga gggaactcag agaccggggc 720
   cggtgcgggt cctccgtata ctgagcgccg tctcctgggc ccactgcctt ctctatactt 780
   tgtctctgtg tcttatttct tttctcagtc tctcatccca cctgacgaga aacacccaca 840
   ggtgtggagg ggctggccac cccttca 867
<210> 23
   <211> 107
   <212> DNA
   <213> Homo sapiens
<400> 23
   ttaggcctct gagcccaagc taagccatca tatcccctgt gacctgcacg tatacatcca     60
   gatagcctga agcaactgta aaaatatcct taactgatga cattcca                  107
<210> 24
   <211> 5888
   <212> DNA
   <213> Homo sapiens
<400> 24
   gtcaggcctc tgagcccaag ccaagtcatc gcatcccctg tgacttgcac gtatacaccc 60
   agatggcctg aagtaactga agaatcacca aagaagtgaa tatgccctgc cccaccttaa 120
   ctgatgacat tccaccacaa aagaagtgaa aatggccagt ccttgcctta agtgatgaca 180
   ttcccttgtg aaagtccttt tcctggctca tcctggctca aaaagctccc ccactgagca 240
   ccttgcgacc cccactccag cccgccagag aacaaacccc ctttgactgt aattttcctt 300
   tacctaccca aatcctataa aacggcccca cccttatctc ccttcgctga ctatcttttc 360
   ggactcagcc tgcctgcaac caggtgaaat aaacagccat gttgctcaca caaagcctgt 420
   ttggtggtct cttcacatgg acgtgcatga aatttggtgc tgtgactcgg atcgggggac 480
   ctcccttggg agatcaatcc cctgtcctcc tgttctttgc tccgtgagaa agatccacct 540
   acgacctcag gtcctccgac cgaccagccc aagaaacatc tcaccaattt caaatccggt 600
   aagtggcctc ttcttactct cttctccaac ctctctcact gtccctcaac cactttcttc 660
   tttccattct tcaatctctc ccttctctta atttcaattc ctttcatttt cagggagaga 720
   caaaggagac acgttttatc cgtggaccca aaactccggc gccggtcacg gactgggaag 780
   gcagccttcc cttggtgttt aatcattgca gggacgcctc tctgattata cacccacgtt 840
   tcaagggtgt cagaccacgc agggatgcct gccttggtcc ttcaccctta gcggcaagtc 900
   ccacttttct ggggaagggg caagtacctc aacccctttt ctccttgtct ctaccccttc 960
   tctgcttttc tgggagaggg gcaagtaccc ctcaacccct tctctccttg tctctacccc 1020
   ttctctgctt tcctggggca ggggcaagta cccctcaacc ccttctcctt cacccttagc 1080
   ggcaagtcct gctttcctgg ggcaggggca agtacccctc aaacccttct ctttcaccct 1140
   tagtggcaag tcccgctttt ctagggggca agaaccccca atcccttatt tccgcacccc    1200
   aacctcgtat ctctgtgccc caatacctta tttctgtgcc ccgacctctt atttccatgc    1260
   cccaacccct tatttctgtg ccccatccct tatttccatg ccctgacctc ttatctctgc 1320
   gccccaaccc cttttcccac ttttctggaa ggtaagaacc cccgaacccc ttccctccgt 1380
   ttctctactc tctcttttct ctaggcttgc ttccttcact ataggcaact ttccaccctc 1440
   cattcctctt tctactccct tggcctgtgt tctcaaaaac ttaaaacctc ttcaactcac 1500
   acctgaccta aaacctaaat gccttatttt cttctgcaat gccacttgac cccaatacaa 1560
   actcaacagt agttccaaat agccagaaaa cggcactttg aatttttcca tcctacaaga 1620
   tctaaataat tcttgtcgta aaataggcaa acggtctgag gtgcctgacg tccaggcatc 1680
   ctttacacat cagtcccttc ctagtctctg tgcccagtgc aacccgtccc aaatcttcct 1740
   tctttccctc ctgcctgtcc cctcagtacc aacaccaagc gtcgctgagt ctttctaatc 1800
   ttccttttct acagacccat ctgacctctc ccctcctcgc caggccgagc taggtcccaa 1860
   ttcttcctca gcctccgctc ctccacccta taatcttttt atcacctccc ctcctcacac 1920
   ctggtccggc ttacagtttc gttccgtgac tagccctccc ccacctgccc agcaatttac 1980
   tcttaaaaag gtggctggag ccaaaggcat agtcaaggtt aatgctcctt tttctttatc 2040
   ccaaatcaga tagcgtttag gctctttttc atcaaatata aaaatgcagc ccagttcatg 2100
   atttgtttgg cagcaaccct gagacgcttt acagccctag accctaaaaa gccaaaaggc 2160
   cgtcttattc tcaaaataca ttgtattacc cattctgctc cgaaataaaa ctccaaaaat 2220
   taaattccag ccctcaaacc ccacaacagt atttaattaa cctcgccttt aaggtgtaca 2280
   ataatagaaa aaagttgcaa ttccttgcct ccactgtgag acaaacccca gccacatctc 2340
   cagcacaaga gaacttccaa acgcctgaac cgcagcagcc aggcgttcct ccagaacctc 2400
   ctcccccagg agcttgctac acatgccgga aatctggcca ctgggccaag gaatgcccgc 2460
   agcctgggat tcctcctaag ccgcgtccca tctgtgtggg accccactga aaatcggact 2520
   gttcaactca cctggcagcc actcccagag cccctggaac tctggcccaa ggctctctga 2580
   ctccttccca gatcttctcg gcttagcggc tgaagactga cactgcccga ttgcctcgga 2640
   agccccctag accatcaagg acgctgagct tcaggtaact ctcacagtgg aaggtaggcc 2700
   cgtccccttc ttaatcaata cggaggctac ccactccaca ttaccttctt ttcaagggct 2760
   tgtttccctt gcctccataa ctgttgtggg tattgacggc caggcttcta aacctcttaa 2820
   aactccccaa ctctggtgcc aacttagaca atactctttt aagcactcct tttcagttat 2880
   ccccacctgc ccagttccct tattaggctg agacacttta actaaattat ctgcttccct 2940
   gactatccct ggactacagc tatatctcat tgccaccctt cttcccaatc caaagcctcc 3000
   tttgcgtcct cctcttgtat ccccccacct taacccacaa atatgagata cctctactcc 3060
   ctccttggcg accgatcatg caccccttac catctcatta aaacctaatc accattaccc 3120
   cactcagcgc caatatccaa tcccgcagca cgcttgaaaa agattaaagc ctgttatcac 3180
   tcgcctgcta cagcatggcc ttttaaaacc tataaactct ccttacaatt tccccatttt 3240
   acctgtccta aaaccagaag agccttacaa cttagttcag aatctgtgct ttatcaacca 3300
   aattgttttg cctatccacc ccgtggtgcc aaacccatat actctcctat cctcaatacc 3360
   tgcctctaca acccattatt ctgttctaga tctcaaacat gctttcttta ctattccttt 3420
   gcacccttaa tcccagcctc tcttcgcttt cacttggact gaccctgaca cccatcaagc 3480
   tcagcaaatt acctaggctg tactgccgca aagcttcaca gacagccccc attacttcaa 3540
   tcaagcccaa atttcctcct catctgttac ctatcttggc ataattctca taaaaacaca 3600
   cgtgctctcc ctgccaatcg tgtctgactg atcactcaaa ccccagcacc ttctacaaaa 3660
   caacaactcc tttccttcct aggcatggtt agcgcggtca gaattcttac acaagagcca 3720
   ggaccgcacc ctgtagcttt tctgtccaaa taacttgaca ttactgtttt agcctagccc 3780
   tcatgtctgc gtgcagcggc tgccgctgca ttaatacttt tagaggccct caaaatcgca 3840
   aactgtgctc aactcactct ctatagttct cataacttcc aaaatctatt ttcttcctca 3900
   tacctgacgc atatactttc tgcttcctgg ctccttcagc tatactcact ctttgttgag 3960
   tctcccacaa ttaccgttgt tcctggcccg gacttcaatc tggcctccca cattattcct 4020
   gataccacac ctgaccccca tgactgtatc tctctgatcc acctgacatt caccccattt 4080
   ccccaaattt ccttctttcc tgttcctcac cctgctcaca cttgatttat tgatggcagt 4140
   tccaccaggc ctaatcgcca cacaccagca aaggcaggtt atgctatagt acaagccact 4200
   agcccgcctc ttagaacctc tcatttcctt tccatcgtgg aaatctatcc tcaaggaaat 4260
   aacttctcag tgttccatct gctattctac tactcctcag ggattattca ggccccctcc 4320
   cttccctaca catcaagctc gaggatttgc ccccgcccag gactggcaaa ttagctttac 4380
   tcaacatgcc ctgagtcaga taactaaaat acctcttagt ctaggtagat actttcactg 4440
   gataggtaga ggcctttcct acagggtctg agaaggccac cgcagtcatt tcttcccttc 4500
   tgtcggacat aattcctcag tttaggcttc ccacctctat acagtctgat aacagacgag 4560
   cctttattag tcaaatcagc caagcagttt ttcaggctct tagtattcag tgaaaccttt 4620
   atatccctta cggtcctccg tcttcaagaa aagtagaatg gactaaaggt cttttaaaaa 4680
   cacacctcac caagccagcc accaacttaa aaaggaccgg acaatacttt taccactttc 4740
   ccttctcaga attcaggcct gtcctcggaa tgctacaggg tacagcccat ttgagctcct 4800
   gtatagatgc tctttttatt aggccccagt ctcattccag acaccagacc aacttagact 4860
   gtgcccccaa aaaaacttgg catccctact atcttctgtc tagtcataca tactcctatt 4920
   caccgttctc aactactcat acatgccctg ctcttgatta cactgccagt ttacactgtt 4980
   tttccaagcc atcacagctg atatctcctg atgctatccc caaactgcca cacttaactc 5040
   ttgaagtaaa taaataatct ttgctggcag gactatgctg aatctcctta ggcactctct 5100
   aatcagatat cctgagtcat cccaattctt agacgtttta tacctgtttt tctccttccg 5160
   ttattccatt tagtttctca attcatccaa aaccgtatcc aggccatcac caaatgtttc 5220
   ttctaacaac cccacaatat caccccttac cacaagacct cccttcagct taatctctcc 5280
   cactctaggt tcccacaccg cccctaatcc cgcttgaagc agccctgaga aacatcgccc 5340
   attctctctc cataccaccc cccaaaaatt tttgccgcgc caacacttca acatcgtttt 5400
   gttttatttt tcttattaat ataagaaggc aggaatgtca ggcctctgag cccaagccaa 5460
   gccatcgcat cccctgtgac ttgcacgtat acacccagat ggcctgaagt aactgaagaa 5520
   tcacaaaaga agtgaatatg ccctgcccca ccttaactga tgacattcca ccacaaaaga 5580
   agtgaaaatg gccagtcctt gccttaagtg atgacattcc cttgtgaaag tccttttcct 5640
   ggctcatcct ggctcaaaaa gcacccccac tgagcacctt gtgaccccca ctcctgcccg 5700
   ccagagaaca aacccccttt gactgtaact ttcctttacc tacccaaatc ctataaaacg 5760
   gccccaccct tatctccctt cgctgactct cttttcggac tcagcctgcc tgcacccagg 5820
   tgaaataaac agccatgttg ctcacacaaa gcctgtttgg tggtctcttc acagggacgt 5880
   gcatgaaa 5888
<210> 25
   <211> 354
   <212> DNA
   <213> Homo sapiens
<400> 25
   ccacaaaaaa agtgaaaata gccagttcct gccttaactg atgacattcc accattgtga 60
   tttgttcctg ctccacccta actgatcaat tgaccttgtg acattccttc tcctggacaa 120
   tgagtctcag aatctcccca ctgagcactt tgtgaccctt gcccctgccc acaagaaaaa 180
   gaaacccttt aactgtaatt ttccattacc tacccaaatc ctataaaact gccccacccc 240
   atctcccttt gctgacctct ttttcagact cagtccgcct gcacccaggt tattaaaaag 300
   ctttattgct cacgcaaagc ctgtttggtg gtctcttcac atagacacat gtga 354
<210> 26
   <211> 90
   <212> DNA
   <213> Homo sapiens
<400> 26
   tcaccaccat cttggaagca gcacaccgcc atcttggaag tggcctgcca ccatcttggg 60
   agctctggga gcaaggatcc cccggtaaca 90
<210> 27
   <211> 4724
   <212> DNA
   <213> Homo sapiens
<400> 27
   gtcaggcctc cgagccaaag ctaagccatc atatcccctg tgaactgcat gtacacatcc     60
   agatggcggg ttcctgcctt aactgatgac attccaccac aaaagaagtg gaaatggcct    120
   gttcctgcct taactgatga cattaccttg tgaaattcct tctcctggct catcctggct    180
   caaaagctcc ctcactgagc accttgtgac tcccacccct gcccaccaga aaagaacccc    240
   ctttgactgt aattttcctt tacctacgca aatcctataa aacggcccca ccccatgtcc 300
   cttcgctgac tcttttcgga ctcagcctac ctgcacccag gtgattcaaa agctttattg 360
   ctcacacaaa gcctgtttgg tggtctcttc acacggacgc gagtgaaatt tggtgccgtg 420
   actcagatcg ggggacctcc cttgggagat caatcccctg tcctcctgct ctttgctccg 480
   tgagaaagat ccacctacga cctcgggtcc tcagaccaac cagcccaagg aacatctcac 540
   caattttaaa tccagtaagc ggcctctctt tactctcttc tccaacctcc ctcactatcc 600
   ctcaacctcg ttctcctttc agtcttggtg ccacacttca atctctccct tctcttaatt 660
   tcagttcctt tccttttctg gtagagacaa aggagatgcg ttttatctgt ggacccaaaa 720
   ctctggcacc ggtcacggac ttgggaagac cgtcttccct tggtgtttaa tcattgcggg 780
   gactcctgcc tgattataca cccacaatcc attggtatct gatctccgtg gggacgcctg 840
   ccttggtcat tcacccacat tcccttggtg gcaagtcaat tgcggggatg cctgctttgg 900
   ctgctcaccc acattgcagc cagggctgct cacccaaccc attctctctg tgtctctacc 960
   ctctcttctc tccactttcc tggggggaca agcatccccc accccttctc cactttcctg 1020
   gggggcaagc atcccccacc ccttctctcc gtatctctac ccttcttttt aaacttgtct 1080
   ccttcactat gggcaacctt ccaccctcta ttcctccttc ttctccctta gcctgtgttc 1140
   tcaagaactt aaaacctctt taactctcgc ctgacctaaa atctaagtgt cttattttct 1200
   tctgcaacac cgcttgaccc caatacaaac tcgacagtgg ttccaaatag ccagaacacg 1260
   gcactttcga tttttccatc ctacaaaatc tagataattc ttgtcgtaaa atgggcaacc 1320
   ggtctgaggt gcctgacatc caggcattct tttacacatc ggtccctccc tagtctctgt 1380
   tcccaatgca acttgtccca aatcttcctt ctttccttcc cgcctgtccc ctcagtccca 1440
   accccaagcg tcactgagtc ttttgaatct tccttttcta cagacccatc tgacctctcc 1500
   cctcctcccc aggctgctct gcgccaggct gagctaggtc cgaattcttc ctcagcctcc 1560
   atttccccac cctataatcc ttttatcacc ttccctcctc acacccggtc tggcttacag 1620
   tttaattctg cgactagccc tcccccacct gcccagcaat ttcctcttaa aaaggtggct 1680
   ggagctaaag gcatagtcaa ggttaatgct cccttttctt tatccgacct ctcccaaatc 1740
   agttagtgtt taggctcttt ttcatcaaat atgaaaaagc cagcccagtt catggctgtt 1800
   tggcagcaac tgtgagatgc tttacagccc tagaccctaa aaggtcaaaa ggccatctta 1860
   ttctcaatat acattttatt acccaatccg ctcccgacat taaataaacc ccccaaatta 1920
   aattccggcc ctcaaacccc acaacaggac ttaattaacc ttgccttcaa ggtgtacaat 1980
   aatagagtag aggcagccaa gtagcagtgt atttccgagt tgcaattcct tgcctccact 2040
   gtgagataaa ccccaaccac atctccagga cacaagaact tcaaacgcct gaaccgcagc 2100
   tgccaggcat tcctccagaa cctcttcccc caggagcttg ctacgagtgt tggaaatctg 2160
   gccactgggc cgaggaatgc ccccagcccg ggattcctcc taagccatgt cccatctgtg 2220
   cgggacccca ctgaaaattg gactgttcaa ctcacctggc agccactccc agagcccctg 2280
   gaactctggc ccaaggctgt ctcactgact ccttcccaga tcttcttggc ttagtggctg 2340
   aagactcacg ctgcccgatc gcctcagaag ccccctagac catcacggat gctgagcttc 2400
   gggtaactct cacagtggag ggtaagtccg tccccttctt aatcaatacg gaggctaccc 2460
   actccacatt accttcaagg gcctgtttcc tttgcctcca taactgttgt aggtattgac 2520
   agccaggctt ctaaacctct taaaactccc caactctgcc aacttggaca acattctttt 2580
   atgcactctt ttttagttat ccccacatgc ccagttccct tattaggccg agacatttta 2640
   accaaattat ctgcatttca actaaattat ctgcttccct gactattcct ggactacagc 2700
   cacatctcat tgccgccctt cttcccaacc caaagcctcc tttgtgtctc cctcttgtat 2760
   ctccccacct taatccacaa gtatatcatg caccccttac catcctatta aaacttaatc 2820
   acccttaccc tgctcaatgc caatatccca tcccacagca cgctttaaaa ggattaaagc 2880
   ctgttatcac tcgcctgcta cagcatgggc ttctaaagcc tacaaactcc ccttacaatt 2940
   cccccatttt acctgtccga aaaccaggca agcctcacag gctagttcag gatctgcgcc 3000
   ttatcatcca aattgttttg tctatccacc ccgtgatgcc aaacccatat actctcctat 3060
   cctcaatacc tccctccaca acccattatt ctgttctgga tctcaaacat gctttcttta 3120
   ctattccttt gcacccttca tcccagcctc tctttgcttt cacttggact gaccctgaca 3180
   cccatcaggc tcagcaaatt acgtgcgctg tactgccaca aggcttcaca gacagccccc 3240
   attacttcag tcaagcccaa atttcttcct catctgttac ctatctcggc ataattctca 3300
   tgaaaacaca cgtgctctct ccctgctgat cgtgtgcagc taatctccca aaccccaatc 3360
   ccttctataa aacaacaact cctttccttc ctaggcatgg tcagtgcagt cagaattctt 3420
   atgcaagagt cgggactgcg ccccgtagcc tttctgtcca aacaacttga ccttactgtt 3480
   ttagcttatc cctcatgtct gcgtgcagca gctgccgctg ctttaatact tttagaggcc 3540
   ctcagaataa caaactatgc tcaactccct ctctaatctt ttctggcagg gctatgctga 3600
   acctccttgg gcactcaatt ctgtcctggg tcctcccaat tcttagtcat ttaatacctg 3660
   ttttttttct tctcttattc ggaccttgtg tcttccgttt agtttttctt tttcttttct 3720
   ttttcttttt tttttttttt ttttttgaga cagagtctca ctgtgtttcc caggctggag 3780
   tgcagtggcg cgatctcggc tcactgcaag ctctgcctcc cgggttcacg ccattcttct 3840
   gcctcagcct cccgagtagc tgggactaca ggcacccgcc accatgctcc gctaattttt 3900
   tgtattttta gtagagacgg ggttacaccg tgttagccag gatggtctca atctcctgac 3960
   ctcatgatcc acccgcttca gcctcccaaa atgctgggat tacgggtgtg agccaccgcg 4020
   cccgacctgc gtttagtttt tcaattcata caaaaccgca tccaggccat caccaatcat 4080
   tctatacgac aaatgctcct tctaacaacc ccacaatatc acctcttacc acaaaatctt 4140
   ccttcagctt aatctctccc actctaggtt cccacgccgc ccctaatctc gcttgaagca 4200
   gccctgagaa acatcgccca ttatctctcc ataccacccc caaaaaattt tcgctgcccc 4260
   aacacttcaa cattattttg ttttattttt ctaattaata taagaagaca ggaatgtcag 4320
   gcctctgagc caaagctaag ccatcatatc ccctgtgacc tgcatgtaca catccaggtg 4380
   gccggttcct gccttaactg atgacattcc accacaaaag aagtgaaaat ggcctgttcc 4440
   tgccttaact gatgacatta ccttgtgaaa ttccttctcc tggctcatcc tggctcaaaa 4500
   gctcccccac tgagcacctt gtgaccccca ctcctgccca ccagagaaca accccctttg 4560
   actgtaattt tcctttacct acgcaaatcc tataagacgg cccaccccat ctcccttcgc 4620
   tgactctctt tttggactca gcctgcctgc acccaggtga ttcaaaagct ttattgctca 4680
   cacaaagcct gtttggtggt ctcttcacac ggacacgagt gaaa 4724
<210> 28
   <211> 5270
   <212> DNA
   <213> Homo sapiens
<400> 28
   ctgagcccaa gctaagccat catatcccct gtgacctgca cgtatacatc caaatggcct     60
   gaagcaactg aagaatcaca aaagaagtga aaatggctgg tttctgcctt aactgatgac    120
   attaccttgt gaaattcctt ctcctggctc agaagctccc ccactgagca ccttgtgacc    180
   cctgcctctg ctcaccagag aacaaccctt tgactgtaat tttccattac ctacccaaat    240
   cctataaaac tgcccaaccc ctatctccct tcactgactc cttttttgga ctcagctcac    300
   ctacacccag gtgattaaaa tctttattgc tcatacaaag cctgtttgat ggtctcttca    360
   cacggacgtg catgacattt ggtgttgaag acctgggaca ggaggactcc tttgggagac    420
   cagtcctctg tccttgtcct cactctgtga ggagatccac ctacgatctc gggtcctcag    480
   accaaacagc ccaaggaaca tcttaccaat ttcaaatcag ataagcagtc ttttcactct    540
   cttctccagc ctctcttgca cccttctatc tccctctgtc gctacccttc aatccccctg    600
   tccttccaat tccagttctt tttcctctct agtagcgaca aaggagacac attttatcca    660
   tggacccaaa actccagtgc cagtcacgga cttgggaaga cggtcttccc ttggtgtcta    720
   atcactacgg ggatgcctgc ctgattattc acccacactg cattggtgtc tgatcaccac    780
   agggatgcct gtcttggtca ttcacccaca ttcccttggt ggcaagtcaa ttgcggggat    840
   gcctgctttg gctgctcacc atcccccttc tccatgtctc taccctctct tttctctggg    900
   cttgcctcct tcactatggg caaccttcca ccctccatta ccccttctcc tttagcctgt    960
   gttctcaaaa acttaaaacc tctttgactc ttacttgatc taaaatctaa gcgtcttatt 1020
   ttcttctgca acaccgcttg gccccagtac aaactcgata attgttctaa atagccagaa 1080
   aatggcactt tggatttctc cattttacaa gatctggatg atttttgttg aaaaatgggc 1140
   aaatggttct gagatgcctg atgtccaggc atccttttac acattggtcc ctccctagcc 1200
   tctgctccca atgtgacttg tcccaaatct ttcttctttc tctcctgtct gttccttcag 1260
   tctccactcc aagctctgag tcctttgaat cctccttttc tacggaccca tccgacctct 1320
   ccactcctcc ccaggctgct cctcaccagg ccgagcctgg ccccaattct tcctcagccc 1380
   tcagcctcca ctcccccacc ctatagtcct tttatcacct tccctcctca cacccagtct 1440
   ggcttacagt ttcattcgtc aactagccct cccccacctg cccaacaatt tcctcttgaa 1500
   gaggtggctg aagctgaagg catagccaaa gttaatgctc cttttccttt atctgacatc 1560
   tcccaaatca gttagaattt aggctctttt tcatcaaaca taaaaactca acccagttca 1620
   tggcccattt ggcaacaccc cttagacgct ttaccgccct agactcagag gggccagaag 1680
   gctctcttat tctcaatatt cattttatta cccaaccagc tcccgacatt agaaaaagct 1740
   ctaaaaatta gattctgacc ttcaaaccct gcaacaggac ttaactaacc tcgccatcaa 1800
   ggtgtacaat aaaagaggca gccaagtagc aacgtatttc tgagttgcaa ttacttgcct 1860
   ccactgtgag agaaacccca gccacatcta cagcacacaa gaacttcaaa atgcctgaac 1920
   cacagtggcc aggcattcct ccaggactgc ctcccccagg atcttgcttc aagtgctgga 1980
   aatctggcca ctgggccaag gaatgcccac agccaaggtt ttctcctaag ccatgtccca 2040
   tctgtgtggg accccactgg aaatcggact gtccaactca cctggcagcc actcccagag 2100
   cccctggaac tctggcccaa ggctccctga ctgactcctt cccggatctt ctcagcttag 2160
   cggctgaaga ttgatgctgc ccaattgcct tggaagcctc ctggaccatc acagacactt 2220
   tgggtaactc ttacaatgaa gagtaggtct gtcctcttct taatcaatat ggaggctacc 2280
   cactccacat taccttcttt tcaagggcct gtttcccttg cctccataac tgctgtggta 2340
   ttgacggcca ggcttctaaa cctcttaaaa ctccccaact ctggtgccaa tttagacaat 2400
   attcttttat acactccttt ttagttatcc ccacctgccc agctccctta ttaactcaag 2460
   atattttaac taaattatct gcttccctga ctattcctgt acgacagcca caccttattg 2520
   ccaccctttt ccccagttca tagcctcctt tgcatcctcc ccttgtgtct ccctacctta 2580
   atccacaagt atgggacacc tctacttcct ccttggtgac ccatcaggca tgccttacga 2640
   tcccattaaa acctaatcac tcttacctgg ctcaatgcca gtaccacatc ccacaacaga 2700
   ctttacgagg actaaagcct gctatcactt gcctgttaca acacggcctt ttaaagccta 2760
   cacattctcc ttacaactcc cctaccccac ctgtccagaa actggacaaa tcttatgggc 2820
   tagttcagga tattcacctt atctgcagca cctcttacaa atcttcccaa caggacacac 2880
   tcctgctcct ccaatatcta ttctcaaagg gatatcacat atccccctcc aaagcctaag 2940
   tttcttcctc atctattacc tatctctgca taattcttca taaaaacaca tgtgctctcc 3000
   ctactgattg tgtgtggcta atctccaaac ctcaacccct tctacaaaac aacagctctt 3060
   ttcctcgtag gcatggctag gtacttttgc ctttggatac ctagttttac catcctgact 3120
   aaaccattat ataaactcac agaaggaaac atagctgacc ccatacatcc taaatccttt 3180
   ctccactcct ttctccattc cttaaaaaca gccctagaag ctgcttccac actagctctc 3240
   cctaactcat cccaactctt ttcattacac acagccaaag tacagggcta tgcagttgga 3300
   attccttaca caaaagccag gaccgagccc tgtagccttt ctgtccaaac aacttgacct 3360
   cacagttttg ggcaagccct catatctctg tgtggcaaag ggactatgtg tcaatatcta 3420
   cactgattcc aagtatgtct tccacatcct tcaccaccat gctattacat gggcagaaag 3480
   aagtttcctc actacacaag ggtcctccat cattaatacc tctttaataa aaactcttct 3540
   caaggctgct ttacttccaa aaaaaaaaaa aagctggagt cattcactgc aaaggccatt 3600
   aaagggcctc agaccccatt gctcaaggca acaattatgc tgataagata gctaaaaaag 3660
   cagccaatat tcctacttct gtccctcatg gccagttttt ctcctcatca gtcactccta 3720
   tttactctcc cactgaagtt tccacctatc gatccctcac cactcaaggc agatggttct 3780
   tagaccaaaa aagaatctcc ttccagcctc acaagcccgt tctattctgt cgtcatttca 3840
   ttacctcttc catgtaggtt acaagccgct agcccacctc ttagaaccaa cctctcattt 3900
   cctttccatc ataggactct atcctcaatg aaatcacttc tcagtgtttc atctgctatt 3960
   ctactactcc ttggggattg ttcaggcccc ctctcttccc tacacatcaa gctcagggat 4020
   ttgcccctgc ccaggactgg caaattgact ttattcacat gcccctagtc aggaaactaa 4080
   aatacctctt tgtctgggta catactttca ctagatgggt agaggccttt cccacatggt 4140
   ctgaaaaggc cacagtggtc atttcttccc ttctgtaaga cataatacct cggtttggcc 4200
   ttcccacctc tatacagtcc aataatggac cggactttat tagtcaaatc agctaagcag 4260
   tttctcaggc tcttggtatt caatggaaat ttcatacccc ttaccgtcct caatcttcag 4320
   gaaaggtaga acgggactaa tggtctttta aaaacacacc tcaccaagct cagcctccaa 4380
   cttaaaaagg aggactctgt caaggataga gccccaaaat tcaccaacca agcaagatat 4440
   tatgctgaac ccccttgggc actctctaat tggatgtcct gggtcctccc aattcttagt 4500
   cctttgatac ctgtttttct ccttctctta tttggacctt gtgccttctg tttagtttct 4560
   caattcatac aaaaccgcat ccaggcgatc accaatcatt ctgtatgaca aatgctcctt 4620
   ctaacaaccc cacaatatca ccccttacca caaaatcttc cttcagctta atttctccca 4680
   ctctaggttc ccatgccacc ccaatcctgc tcgaaacagc catgaaaaaa atcgcccatt 4740
   atctctccat atcacccccc aaaattttca ctgccccaat gcttcaacac tattttgttt 4800
   tatttttctt atttttctta ttaatataag aagacaggaa tgtcaggcct ctgagcccaa 4860
   gctaagccat catatcccct gtgacctgca cgtatacatc caaatggcct gaagcaactg 4920
   aagaatcaca aaagaagtga aaatggctga ttcctgcctt aattgatgac attccaccat 4980
   tgtgatttgt tcctacccca cctaaactga gcaattaacc ttgtgaaatt ccttctcctg 5040
   gctcagaagc tcccccactg agcaccttgt gactcctgcc ccttcccacc agagaacaac 5100
   ccctttgact gtaattttct gctactaccc aaatcctata aaactgccca acccctatct 5160
   cccttcgctg actccttttt cggacttagc ccacctgcac ccaggtgatc aaaaagcttt 5220
   attgctcaca caaagcctgt ttggtgacct ctgcacatgg atgtgcatga 5270
<210> 29
   <211> 5517
   <212> DNA
   <213> Homo sapiens
<400> 29
   gtcaggcctc tgagcccagg ctaagccatc atgtcccctg tgacctgcat gtacacatcc     60
   agacggctgg ttcctgcctt aactgctgac attccaccac aaaagaagtg aaaatgtcct    120
   gttcctgcct caactgatga cattgtcttg tgaaattcct tctcctggct catcctggct    180
   caaaagctcc cccactgagt aacttgtaac ccccactctg cccgccagag aacaaccccc    240
   cctttgactg gattgggtag gatttaccta cccaaatcct ataaaacggc cccaccccta   300
   tctcccttcc ctgactctct ttttggaccc agcacacctg cacccagggg aaataaacag    360
   ctttattgct cacagaaagc ctgtttggtg gtctcttcac acagacacga gtgaaatttg    420
   gtgctgtgac ttggatcggg ggacctccct tgggacatca atcccctgtc ctcctgttct    480
   ttgctccgtg agaaagatcc acctacaacc tcaggtcctc agactgacca gcccaagaaa    540
   cgtctcacca atttcaagta tggtaagtgg cctcttttta ctctctcctc caacctcact    600
   tactatccct caacctcttt ctcctttcaa tcttggcacc acacttcaat ctctcccttc    660
   ttttaatttc aattcctttc attttctggt agagacaaag gagacgcgtt ttatccgtgg    720
   acccaaaact ccagtgctgg tcacggactg ggaaggcagc cttcccttgg tgtttaatca    780
   tttcagggac gcctctctga ttattcaccc atgtttcaga ggtgtcagac cacgcaggga    840
   cgcctgtctt ggtccttcac ccttagaggc aagtcccgct tttctagggg aggggaaagt    900
   accccaacct catatctctg tgccctgatc ccttatttcc acaccccaac ctcttatatc    960
   tctgtgcccc gatcccttat ttccgtgccc ctacctctcc cgcttttctg gagggtaaga   1020
   acccccaaac cccttccctc cgtttctcta ctctctcttt tctctgggct tgcctccttc   1080
   actgtgggca accttccacc ctccattgct ccttctccct tagcctgtgt tcttaagaac   1140
   ttaaaacctc ttcagctctc acctgaccta aactctcagc atcttatttt cttccgcaat 1200
   gccacctgac cgcaatacaa actcgacagt agttccaaat agccagaaaa cggcactttc 1260
   aatttttcca tcctgcaaga tctaaataat tcttgttgta agatgggtaa atggtctgag 1320
   gtgcctgacg tccaggcatt cttttacacg tcggtccctc tctagtctct gttcccaatg 1380
   caactcatcc caaatcttcc ttctttccct cccacccgtc ccctcagtcc caaccccaag 1440
   ggtcgctgag tctttctaat cttccatttc tacagaccca tctgacctct cccctcctcc 1500
   ccaggctgct cctcgccagg ccgagctacg tcccaattct tcctcagcct ctgctcctcc 1560
   accctataat ccttttatca cctcccctcc tcacacctgg tccggcttac agtttcgttc 1620
   cgtgaccagc cctcccccac ctgcccagca atttactctt aaaaaggtgg ctggagctaa 1680
   aggcatagtc aaggttaatg ctcctttttc tttatcccaa atcagatagc gtttaggctc 1740
   tttttcatca aatataaaaa tccagcccag ttcatgactt gtttggcagc aaccctgaga 1800
   cactttacag ccctagaccc taaaaggtca aaaggccgtc ttattctcaa aatacatttt 1860
   attacccaat ctgctcccga cattaaataa aactccaaaa attaaattcc ggcccttaaa 1920
   cccctcaaca ggatttaatt aacctcacct tcaaggtgta caataataga aaaaagttgc 1980
   aattccttgc ctccactgtg agacaaaccc cagccacatc tccagcacac aagaacttcc 2040
   aaacgcctga accacagcgg ccaggccttc ctccagaaca tcctccccca ggagcttgct 2100
   acaagtgcca gaaatctggc caccaggcca aggaatgcct gcagcccggg attcctccta 2160
   agccacgtcc catctgtgcg ggaccccact ggaaatcgga ctgtccaact cacctggcag 2220
   ccactcccag agcccctgga actctggccc aaggctctct gacggcttcc cagatcttct 2280
   tggcttagcg gctgaagact gactctgccc gatcacctcg gaagccccct aggccatcac 2340
   ggacgccgag cttcgggtaa ctctcacggt ggaagctaag cccgtcccct tcttaatcaa 2400
   tacggagcct acccactcca cattaccttc ttttcaaggg cctgtttccc ttgcctccat 2460
   aactgttgtg ggtattgacg gccaggcttc taaacctctt aaaactcccc aactctggtt 2520
   ccaacttaga caatactctt ctaagcactc cttttagtta tccccacctg cccagttccc 2580
   ttattaggcc gagacacttt aactaaatta tctgcttccc tgactattcc tgtattacag 2640
   ctacatctca ttgctgccct tcttcccaat ccaaagcctc ctttgcgtcc tcctcttgta 2700
   ttctcccacc ttaacccaca agaataagat acctctactc cctccttggc gaccgatcat 2760
   gcacccctta ctatctcatt aaaacctaat cacccttacc ccgctcaatg ccaatatccc 2820
   atcccacacc atgctttgaa aggattaaag cctgttatca ctcacctgct acagcatggc 2880
   cttttaaagc ctataaactc tccttacaat tcccccatta tacctgtcct aaaaccagac 2940
   aagccttcca agttagttca ggatctatgc cttatcaacc aaattgtttt gcctatccac 3000
   cccatggtgc caaacccata tactctccta tcctcaatac ctccctccac aatccattat 3060
   tctgtgctgg atctcaaacc tgctttcttt actattcctt tgcacccatc atcccagcct 3120
   ctcttcgctt tcacctggac tgaccctgac acccatcagg ctcaggaaat tacctgggct 3180
   gtactgccgc aaagtttcac agacagcccc cattacttca gtcaagccca aatttattcc 3240
   ttatctgtta cctatctcag cataattctc ataaaaacac acgtgctctc cctgctgatg 3300
   tccaattaat ctcccaaacc tcaatccctt acaaaacaac aactcctttc cttcctaggc 3360
   atggtttgtg cggtcagaat tcttacacaa gagccaggac cgcaccctgt agcctttctg 3420
   tccaaacaac ttgaccttac tgttttagcc tagccctcat gtctgcgtgc gtggctgctg 3480
   ccaccctaat acttttagag gccctaaaaa tcacaaacta tgctcaactt actctctaca 3540
   tttctcataa cttccaaaat ctattttctt cctcatacct gacgcatata ctttcggctt 3600
   cctggctcct tcagctatac tcactctttg ttaactccca caattaccat tgttcctggc 3660
   ccggacttca atctggcctc ccacattatt cctgatacca cacctgacct ccatgactgt 3720
   atctctctga tccacctgac attcaccaca tttccccata tttccttctt tcctgttcct 3780
   caccctgatc acgcttgatt tattgatggc agttccacca ggcctaatcg ccacacacca 3840
   gcaaaggcag gctatgctat agtacaagcc actagccccc ctcttagaac ctctcatttt 3900
   ctttccatcg tggaaatctg tcctcaagga aataacttct cagtgttcca tctgctattc 3960
   tactactcct cagggattat tcaggccccc tcccttcctt acacatcaag ctcgaggatt 4020
   tgcccctacc caggactggc aaattagctt tactcaacat gccccgagtc agataactaa 4080
   aatacctctt agtctaggta gacactttca ctggataggt acaggccttt cctacagggt 4140
   ctgagaaggc caccgcagtc atttcttccc ttctgtcaga cacaattcct cagtttagcc 4200
   ttcccacctc aatacagtct gataacagac gagcctttat tagtcaaatc agccaagcag 4260
   tttttcaggc tcttagtatt cagtgaaacc tttatatccc ttatggtcct ccgtcttcag 4320
   gaaaagtaga atggactaaa ggtcttttaa aaacacacct caccaagctc agtcaccaac 4380
   ttaaaaagga ctggacaata cttttaccac tttcacttct cagaactcag gcctgtcctc 4440
   ggaatgctac agggtacagc ccatttgagc tccttttttt attaggcccc agtctcattc 4500
   cagacaccag accaacttgg actgtgcccc aaaaaacttg tcatccctac tatcttctgt 4560
   ctagtcatac tcctattcac cgttctcaac tactcataca tgccctgctc ttgtttacac 4620
   tgccagttta cactgtttct ccaagccagc acagctgata tctcctggtg ctatccccaa 4680
   accgccactc ttaactctta aagtaaataa ataatcttta ctggcaaggc tatgctgaac 4740
   caccttaggc actctctaat tagatgtcgt aggtcctccc aattctgagt cctttaatac 4800
   ctgtttttct ccttctctta ttctgtttag tttttcaatt catacaaaac tgtatccagg 4860
   ccaacaccaa taattctaaa taacaaatgt ttcttctaac agccccacaa tatcacccct 4920
   tgccacaaaa ttttccttca gcttaatctc tcccactcta ggttcccacg ccgcccctaa 4980
   tcccgctcga agcagccctg agaaacatca cccattatct ctccatacca ccaaaaaatt 5040
   ttcactgtcc caacacttta ccactatttc attttattgt tcttattaat ataagaagac 5100
   aggaatgtca ggcctctgag cccaagctaa gccatcacat accctgtgac ctgcaggtac 5160
   acatccagat ggctggttcc tgccttaact gctgacattc caccacaaaa gaagtgaaaa 5220
   tgtcctgttc ctgccttaac tgatgacatt gtcttgtgaa attccttctc ctggctcatc 5280
   ccggctcaaa agctccccca ctgagtacct tgtgaccccc actctgccgg ccagagaaca 5340
   cccccctttg actgtaattt tcctttatct acccaaatcc tataaaacgg ccccacccct 5400
   atttcccttc gctgactctc tttttggact cagcccacct gcacccaggt gaaataaaca 5460
   gctttattgc tcacacaaag cctgtttgat ggtctcttca catggatgag agtgaaa 5517
<210> 30
   <211> 5742
   <212> DNA
   <213> Homo sapiens
<400> 30
   gtcaggcctc tgagcccaag ctaagccatc atatcccctg tgacctgcac gtacacatcc      60
   agatggccag ttcctgcctt aactgatgac attccaccac aaaagaagtg aaaatggtct     120
   gttcctgcct taactgatga cattatcttg tgaaattcct tctcctggtt catcctggct     180
   caaaagctcc cctactgagc gccttttgac ccccacacct gctccccctt tgactgtaat     240
   tttcctttac ctacccaaat cttataagac ggccctatcc ctgtctccct tggctgactc     300
   tcttttcgga ctcagcccgc ctgcacccag gtgaaataaa cagccttgtt gctcacacaa     360
   agcctgtttg gtggtctctt cacacaaacg cgcatgaaat ttggtgccat gactcggatc     420
   ggggtacctc ccttgggaga tcaatcccca gtcctcctgc tctttgctcc gtgagaaaga     480
   tctacctagg acctcaggtc ctcagactga ccagcccaag gaacatctca ccaatttcaa     540
   atctggtaag tggcctcttt ttactctctt ctccaacctc cctcactatc cctcaacctc     600
   tttctccttt ctatcttggc gccacacttc aatctctccc ttctcttaat ttcaattcct     660
   ttcattttct ggtagagaca aaggagacac attttatccg tggacccaaa actccggcgc     720
   cagtcacgga ctcggggagg cagccttccc ttggtgttta ataattgcgg gggtgcctct     780
   ctgattattc acccacgttc cattggtgtc tgatctctgc ggggacgcct gcctttgatc     840
   attcacccac gttcccttgg tggcaagtca attgcaggga ctcctgcttt ggctgctcac     900
   ctacgttgca gcccagggct gctccccacc ccccttctcc gtgtccctac ccttctcttt     960
   aaacttgcct ccttcactat gggcaacctt ccaccctcca ttcctccttc ttctccctta    1020
   gcctctgttc ttaagaactt aaaacctctt caactctcgc ctgacctaaa atctaagtgt    1080
   cttattttct gcaatgccat ttgaccccaa tacaaacttg acagtagttc caaacagcca 1140
   gaaaatggca ctttgaattt ttccattcta caaaatctaa ataattcttg ttgtaaaatg 1200
   ggcaaacggt ctgaggtgcc tgacgtccag gcattctttt acacatcagt ccctctctag 1260
   tctctgttcc cagtgcaact catctcaaat cttccttctt tccctccccc ctgtcccctc 1320
   agtaccaacc ccaagcgtca ctgagtcttt ctaatcttcc ttctctacag acccatctga 1380
   cctctcccct cctcaccagg ccaagctagg tcccaattct tcctcagcct ccgttcctcc 1440
   accctataat ccttttatca cctctcctcc tcacacctgg tcaggcttac actttccttc 1500
   tgtgactagc cctcccccac ctgcccagca atttcctctt aaaaaggtgg ctggagctaa 1560
   aggcatagtc aaggttaatg ctcctttttc tttatcccaa atcacatagc gtttaggctc 1620
   tttttcatta aatataaaaa cccagcccag ttcatggctt gtttggcagc accctgagat 1680
   gctttacagc cctagaccct aaaaggtcaa aaggctgtct tattctcaat attcatttta 1740
   ttacccaatc cattcccgac attaaataaa actccaaaaa ttaagttcca tccctcaaac 1800
   cccacaacag gacttaatta accttgcctt caaggtgtac aataatagag tagagacagc 1860
   caatagcaac atatttctga gttgcacttc cttgcctcca ctgtgagaca aaccccaccc 1920
   acatctccag cacacaagaa cttccaaacg cctaaatcgc agtggccagg cattcctcca 1980
   ggcctgcctc ccccaggggc ttgctacaag tgccagaaat ctggccatga ggccaaggaa 2040
   tgcccgcagc ccgggattcg tcctaagctg cgtcccatct gtgcaggacc ccactgaaaa 2100
   tcggactgtt caactcatct ggcagccact cccagagccc ctggaactct ggcccaagcc 2160
   tctctgactg actccttccc agatcttctc ggcttagcag ctgaagactg acactgccca 2220
   atcgccttgg aagcccccta gaccatcacg gatgccgagc ttcgagtaac tctcacagtg 2280
   gagggtaagt cctgtcccct tcttaatcaa tacggaggct acccactaca cattactttc 2340
   ttttcaaggg cctgtttccc ttgcctccat aactgttgtg ggtattgatg gccaggcttc 2400
   taaacctctt aaaactcccc aactctggtg ccaacttaga caatactttt ttaagccctc 2460
   ctttttagtc atccccacct gcccagttcc cttattaggc cgagacactt taactaaatt 2520
   atctgctgcc ctgactattc ctgggctaca gctatatctc attgccgccc ttcttcccaa 2580
   tccaaagcct cctttgcgtc atcctcttgt atccccccac cttaacccac aagtatagga 2640
   tacctctact ccctccttgg caaacgatca tgcacccctt accatctcat taaaacctaa 2700
   tcacccttac cccgctcaag gccaatatcc catcccacag ctcgctttaa aaagattaaa 2760
   gcctgttatc actcgcctgc tacagcatgg ccttttagag cctataagct ctccttacaa 2820
   ttcccccatt ttacctgtcc taaaaccaga caagccttac aagttagttc aggatctgcg 2880
   ccttgtcaac caaattgttt tgcctatcca ccccatggtg ccaaacccat atacactcct 2940
   atcctcaata cctccctcca caacccatta ttctgttctg gatctcaaac attctttcgt 3000
   tactattcct ttgcaccctt catcccagcc tctcttcact ttcacttgga ctgaccctga 3060
   cacccatcag gctcagcaaa tcacctgggc tgtactacca caaggcttca cagatagccc 3120
   ccactacttc agtcaagccc aaatttcatc ctcatctgtt acctatctcg ggataattct 3180
   cataaaaaca cacgtgctct ccctgctgat cgtgttcgac tgatctccca aacctcaatc 3240
   ccttctacaa aacaacaact cctttccttc ctaggcatgg ttagtgcggt cagaattgtt 3300
   acataagagc caggacaaca ccctgtagcc tttctgtcca aacaacttga ccttactgtt 3360
   ttagcctagc cctcatgtct gtgtgcagtg gctgctgctg ctttaatact tttagagtcc 3420
   ctaaaaatca caaactatgc tcaagtcact ctctacagtt cttataactt ccaaaatcta 3480
   ttttcttcct catacctgat gcatatactt tctgcttccc ggctccttca gctatactca 3540
   ctctctgtcg agtctcccac aattaccatt gttcctggca cgggcttcaa tccggcctcc 3600
   cacattattc tggataccac atctgaccct catgactgta tctctctgat ccacctgaca 3660
   ttcaccccat ttccccatat ttccttattt cctgttcctc actctgatca catttggttt 3720
   attgatggca gttacaccag gcctaatcgc cactcaccag caaaggcagg ctatgctata 3780
   gtatcttcca catctatcat tgaggctacc gctctgcccc tctccactac ctctcagcaa 3840
   gccgaactag ttgacttaac ccgggccctc actcttgcaa aacgactacg tgtcaatatt 3900
   tatactgact ctaaatatgc cttccatatt ctgcaccacc atgctgttac ataggcagaa 3960
   agaagtttcc tcactatgca agggtccttc atcattaatg cctcttaata aaaactctgc 4020
   tcaaggctgc tttacttcca aaggaagctg gagtcattca ctgcaaaggc catcaaaagg 4080
   catcagatcc cattgctcta ggcaacgctt atgctgataa ggtggctaga caagcagcta 4140
   gctttccaac ttctgtccct caacaagcag ctagctttcc aacttctgtc cctcatggcc 4200
   aatttttctc cttcacattg gtcactccca cctgctcggg gatttgcccc tgcccaggac 4260
   tggcaaattg actttattca catgccccga gtcagaaaac taaaatacct cttagtctag 4320
   gtagacactt tcactggata ggtagaggcc tttcctatag ggtctgagaa ggccactgct 4380
   gtcatttctt cccttctgtc agacataatt cctcggttta gacttcccac ctctatacag 4440
   tccgatagca gaccagcctt tattagtcaa atcagccaag tattttttca ggctcttagt 4500
   attcagtgaa acctttatat cccttacggt cctcagtctt caggaaaggt agaacagact 4560
   aatggtcttt tacaaacaca cctcaccaag ctcagccacc aacttaaaaa agactggaca 4620
   atacttttac cactttccct tctcagaatt caggcctgtc ctcggaaagc tacagggtac 4680
   agcccatttg agctcctgta tagacactac tttttattag gccccagtct cattccagac 4740
   tccagaccaa cttggactgt gccccaaaaa acttgtcatc cctactatct tctgtctagt 4800
   catactccta ttcattgttc tcaactactt acacatgccc tgctcttgtt tacactgcca 4860
   gtttacactg tttctccaag ccatcacagc tgatatctcc tggtgctatc cccaaactgc    4920
   cactcttaac tcttaaagta aataaataat ctttgctggc aggactgtgc tgaatctcct    4980
   taggcactct ctaaatagat gtcctaggtc ttcccaattc ttagaccttt aatacctgtt    5040
   tttgtccttc ttttattccg tttagttttt caattcatac aaaaccatat ccaggccatc    5100
   accaataatt ctaaatgaca aatgtttctt ctaacaaccc cacaatatca ccccttacca    5160
   caaaatcttc cttcagctta atctctccca ctctaagttt ccacactgcc cataatcccg    5220
   cttgaagcag ccctgagaaa catggcccat tatctctcca taccaccccc aaaaattttt    5280
   gccgtcacaa cattttacca ctatttcgtt ttatttttct tattaatata agaagacagg    5340
   aatgtcaggc ctctgagccc aagctaagcc atcatatccc ctgtgacctg cacgtacaca    5400
   tccagatggc cagttcctgc cttaactgat gacattccac cacaaaagaa gtgaaaatgg    5460
   cctgttcctg ccttaactga tgacattatc ttgtgaaatt ccttctcctg gttcatcctg    5520
   gctcaaaagc tcccctactg agcgccttgt gacccccaca cctgcccctc ctttgactgt    5580
   aattttcctt tacctaccca aatcttataa aatggcccca cccctatctc ccttggctga    5640
   ctctcttttc ggactcagcc cgcctgcacc caggtgaaat aaacagcctt gttgctcaca    5700
   caaaccctgt ttgctggtct cttcacagga acgcgcatga aa                       5742
<210> 31
   <211> 5642
   <212> DNA
   <213> Homo sapiens
<400> 31
   gtcaggcttc tgagcccaag ctaagccatc gtatcccctg tgacctgcac atacacatcc 60
   agatggccgg ttactgcctt aactgatgac attcctccaa aaaagaagtg aaaatggcct 120
   gttcctgcct taactgatga cattatcttg tgaaattcct tctcctgggt caaaagctcc 180
   cttactgagc accttgtgac ccccacccct gcccgccaga gaacaacccc tttgactgta 240
   attttccttt acctacccaa atcctataaa acggccccac ccctatctcc cttcactgac 300
   tctcttttcg gactcagccc gcctgcaccc aggtgaaata aacagccttg ttgctcacac 360
   aaagcctgtt tggtggtctc ttcacatggt cgcgcatgaa atttggtgcc gtcactcaga 420
   tcaggggacc tcccttggga gatcaatccc ctgtcctcct gctctttgct ccatgagaaa 480
   gatccaccta tgacctcagg tcctcagacc gaccagccca aggaacatct caccaatttc 540
   aaatccagta agcggcctct ttttactctc ttctccaccc tctctcacta tccctcaacc 600
   tctttctcct ttcaatcttg gcaccacact tccatctctc ccatctctta ttttcaattc 660
   ctttcatttt ctggtagaga caaaagagac acattttttc cgtggaccca aaactccggc 720
   gccagtcaca gactcaggaa ggcagccttc ccttggtgtt taatcattgt ggggacacct 780
   ctctgattat tcacccacgt tccactggtg tctgatctcc acggagacgc ctgccttgat     840
   cattcaccca cgttcccttg gtggcaagtc aattgcgggg acgcctgctt tggctgctca     900
   accacgttgc agcccagggc tgctccccac caccttctct gtgtctctat ccttctcttt     960
   aaacttgcct cctttactat gggcaacctt ccaccctcca ttcccccttc ttctccctta    1020
   gcctgtgttc ttaaaaacct aaaacctctt cacctcacac ctgacctgaa acctaaatgc    1080
   cttatttttt tctgcaatgc tgcttgaccc caatacaaac tcaacagtgg ttccaaatag    1140
   ccagaaaatg gcactttcaa ttattccatc ctacaaggtc taaataattc ttgtcgtaaa    1200
   atgggcaaac ggtctgaaat gcctgacgtc caggcattct tttacacatc ggtccctccc    1260
   tagtctctgt tcccaataca actcgtccca aatcttcctt ctttccctcc tgcctgtccc    1320
   ctcagtccca accccaagcg tcgctgagtc tttctaatct tccttttcta cagacccatc    1380
   tgacctctcc cctcctcgcc aggctgagct aggtcccact tcttcctcag cctccgctcc    1440
   tccaccctat aatcatttta tcacctccct tcctcacacc cggtccagct tacagttttg    1500
   ttctgtgact agccctcccc cacctgccca gcaatttact cttaaaaagg tggctggagc    1560
   taaaggcgta gtcaaagtta atgctccttt ttctttatcc caaatcagat agcatttaga    1620
   ctctttctca tcaaatataa aaacccagcc cagttcatgg cagcaaccct gagatgcttt    1680
   acagccctag atcctaaaag gtcaaaaggc cgtcttattc tcaatataca ttttattacc    1740
   caatctgctc ccgacattta ataaaactcc aaaaattaaa ttccggccct caaaccccac    1800
   aacaggactt aattaacctt gccttcaagg tgtacaacaa tagagtagag gcagccaagt    1860
   agcaacatat ttctgagttg caattccttg cctccaccgt gagacaaacc ccagccacat    1920
   ctccagcaca caagaacttc caaatgcctc aaccacagtg gccaggcatt cctccaggcc    1980
   tgcctcccct aggagcttgc tacaagtgcc agaaatctgg ccaccaggcc aaggaatgtc    2040
   cacagcccag gattcctcct aagccgcgtc ccatctgtgt gggaccccac tgaaaatcgg    2100
   actgttcaac tcacctggca gccactccca gagcccctgg aactctggcc caaggctctc    2160
   tgactgactc cttcccagat cttcttggct tagcggctga agactgatgc tgcccgatcg    2220
   cctcagaagc cccgtagacc atgatggaca ccgagcttta ggtaactctc acagtggaag    2280
   ataagtccgt ccccttctta atcaatacgg aggctaccca ctccacgtta ccttcttttc    2340
   aagggcctgt ttcccttgcc tccataactg ttgtgggtat tgacggccag gcttctaaac    2400
   ctcttaaaac tccccaactc tggtgccaac ttagacaata ctcttttaag cactcctttt    2460
   tagttatccc cacctgccca gttcccttat taggccgaga cactttaact aaatgatctg    2520
   cttccctgac tattcctgga ctatagctac atctcattgc tgcccttctt cccaatccaa    2580
   agcctccttt gtgtcctcct cttgtatccc cccaccttaa cccacaagta taggatacct    2640
   ctactccctc cttggtgacc gatcatgcac cccttaccat ctcattaaaa cctaatcacc    2700
   cttaccccgc tcaatgccaa tatcccatcc cacagcacgc tttaaaagga ttaaagcctg 2760
   ttatcactcg cctgctacag catggccttt taaagcctat aaactctcct tacaattccc 2820
   ccattttacc tgtcctagaa ccagacaagc cttacaggtt agttcaggat ctgcgcctta 2880
   tcaaccaaat tgttttgcct atccaccccg tggtgccgaa cccatacact ctcctatcct 2940
   caatacctcc ctccacaacc cattattctg ttctggatct caaacatgct ttctttacta 3000
   ttcctttgca cccttcatcc cagcctgtct tcgctttcac ttggactgac cctgacaccc 3060
   atcaggctca gcaaattacc tgggctgtac tgcctcaagt cttcacagac agcccccatt 3120
   acttcagtca agcccaaatt tcttcctcat ctgttaccta tctcagcgta attctcataa 3180
   aaacacacgt gctctccctg ctgatcatgt tcagctgatc tctcaaaccc cgagaactac 3240
   aaaacaacaa ctcctttcct tcctaggcat ggttggatac tttcgacttt agatagctgg 3300
   ttttgccatc ctaacaaaac cattatataa actcacaaaa agaaacctag ctgaccccat 3360
   agatcctaaa tcctttcccc actcctcttt ccattccttg aagacagctt tagaggctgc 3420
   ccccacccta gctctccctg atttatccca acccttttca ttacacacag ctgaagtgca 3480
   gggctgtgca gtcaggattc ttacataagg accgggatca tgtcctgtag cctttttatc 3540
   caaacaactt gaccttactg ttttgcccag ccctcaagtc tgcgtgcagc ggccgccgct 3600
   gccctaatac ttttagaggc ccttaaaatc acaaactatg ctcaactcac tctctgcagt 3660
   tctcataact tccaaaatct attttcttcc tcacacctga cacatacttt ctgctccccg 3720
   gctccttcag ctgtactcac tctttgttaa gtctcccaca attaccatcg ttcctggccc 3780
   ggacttcaat ccagcctccc acattattcc tgataccaca cctgaccccc atgactctat 3840
   ctctctgatc cacctgacat tcaccccatt tccccatatt tccttcttcc ctgtttctca 3900
   ccctgatcac acttggttta ttgatggcag ttccaccagg cctaatcgcc acacaccagc 3960
   aaaggcaggc tatgctatag tacaagccac tagcccacct cttagaacct ctcatttcct 4020
   ttccatggtg gaaatctatc ctcaaggaaa taacttctca gtgttccatc tgctattcta 4080
   ctactcctca gggattattc aggccccctt ccttccctac acatcaagct cggagatttg 4140
   cccccatcca ggactggcag attggcttta ctcaacatgc cccgagtcag ataactaaaa 4200
   tacctcttag tctaagtaga cattttcact ggatgggtag aggcctttcc tacagggtct 4260
   gaggaggcca ccacagtcat ttcttccctt ctgtcagaca taattcctct gttcagcctt 4320
   cccacctcta tccagtctga taacagacca gcctttatta gtcaaatcag ccaagcagtt 4380
   tttcaggctc ttggtattca gtgaaacgtt tatatccctt acagtcctca gttttcagga 4440
   aaagtagaac agactaatgg tcttttaaaa acacacctca ccaagctcag ccaccaactt 4500
   aaaaaggaat ggacgatact tttaccactt tcccttctca gaattcaggc ctgacctcag 4560
   aatgctagaa ggtacagccc atttgagctc ccgtatagac gctccttttt attaggcccc    4620
   agtctcatcc cagacaccag accaacttag actgtgcccc caaaatcttg tcatctctac    4680
   tattttctgt ctagtcatac tcctattcac cagtttcaac tactcataaa tgccctgctc    4740
   ttgtttacac tgccagttta cactgtttct ccaatctatc acagctgata tctcctggtg    4800
   ctatccccaa accgccactc ttaaagtgaa taaataatct ttgctggcaa ggctatgctg    4860
   aacctcctta ggcactctct aattagatgt cctaggtcct cccaattctt agtcctttaa    4920
   tacctgtttt tctccttctc ttgttctgtt tagtttttca attcatacaa aactgtatcc    4980
   aggccatcac caataattct acacaacaaa tgtttcttct aacaacccca caatatcacc    5040
   ccttaccaca aaatcttcct tcagcttaat ctctcccact ctaggttccc acgcctcccc    5100
   taatcccgct tgaagcagcc ttgagaaaca tcgcccattc tctctccata ccacccccaa    5160
   aaattttcgc cgccccaaca ctttaccact atttcttttt atttttctca ttaatataag    5220
   aagacaggaa tgtcaggcct ctgagcccaa gctaagccat catatcccct gtgacctgca    5280
   catacacatc cagatggctg gttcctgcct taactgatga cattccacca caaaagaagt    5340
   gaaaatggcc tgttcctgcc ttaactgatg acattctctt gtgaaattcc ttctcctggc    5400
   tcatcctgga tcaaaagctc ccctactgag caccttgtga cccccactcc tgcctgccag    5460
   ataacaaccc ctttgactgt aattttcctt tatctaccca aatcctgtaa aacagcccca    5520
   cccctatctc ccttcactga ctctcttttc ggactcagcc cacctgcacc caggtgaaat    5580
   aaacagcctt gttgctcaca caaagcctgt ttggtggtct cttcacacgg acgtgcatga    5640
   aa                                                                   5642